# EUROPEAN PATENT SPECIFICATION

(11) **EP 2 237 774 B1**
(45) Date of publication and mention of the grant of the patent: **22.06.2016**
(21) Application number: 08864594.0
(22) Date of filing: 11.12.2008
(51) Int. Cl.: A61K 31/00, A61K 31/519, A61P 33/00

(54) **THE USE OF 6-HALOGENO-(1,2,4)-TRIAZOLO-(1,5-a)-PYRIMIDINE COMPOUNDS FOR COMBATING PESTS IN AND ON ANIMALS**
VERWENDUNG VON 6-HALOGEN-(1,2,4)-TRIAZOLO-(1,5-A)-PYRIMIDIN-VERBINDUNGEN ZUR BEKÄMPFUNG VON SCHÄDLINGEN IN UND AUF TIEREN
UTILISATION DE COMPOSÉS 6-HALOGÉNO-(1,2,4)-TRIAZOLO-(1,5-A)-PYRIMIDINE DANS LA LUTTE CONTRE LES NUISIBLES CHEZ L'ANIMAL

(30) Priority: 21.12.2007 US 16091 P
(43) Date of publication of application: 13.10.2010
(73) Proprietor: Merial Limited, Duluth, GA 30096 (US)
(72) Inventor: BAUMANN, Ernst, 67373 Dudenhofen (DE); POHLMAN, Matthias, 67251 Freinsheim (DE); CLARK, Jeffrey Norman, Pittsboro North Carolina 27312 (US); POWELL, Kerrie Maria, Durham North Carolina 27713 (US); BOECKH, Albert, Mattawan MI 49071 (US); SOLL, Mark David, Alpharetta Georgia 30005 (US)
(74) Representative: D Young & Co LLP
(86) International application number: PCT/EP2008/067326
(87) International publication number: WO 2009/080546

(56) References cited:
- WO-A-03/039259
- WO-A-2005/025315
- WO-A-2007/104557
- WO-A-2007/149211
- ALI B H: "AGENTS AMELIORATING OR AUGMENTING EXPERIMENTAL GENTAMICIN NEPHROTOXICITY: SOME RECENT RESEARCH" FOOD AND CHEMICAL TOXICOLOGY, PERGAMON, GB, vol. 41, no. 11, 1 November 2003 (2003-11-01), pages 1447-1452, XP008035785 ISSN: 0278-6915
- SRIVASTAVA, RAVI P. ET AL: "Studies in antiparasitic agents. Part 24. Synthesis of 5-(2-furyl)-2-substituted-amino-1,3,4-tria zoles and substituted 1,3,4-triazolo[1,5-a]pyrimidines as potential antifilarial and leishmanicidal agents" INDIAN JOURNAL OF CHEMISTRY, SECTION B: ORGANIC CHEMISTRY INCLUDING MEDICINAL CHEMISTRY , 34B(3), 209-14 CODEN: IJSBDB; ISSN: 0376-4699, 1995, XP002512388

## Description

### FIELD OF THE INVENTION

The present invention relates to 6-halogeno-[1,2,4]-triazolo[1,5-a]-pyrimidine compounds of the general formula I 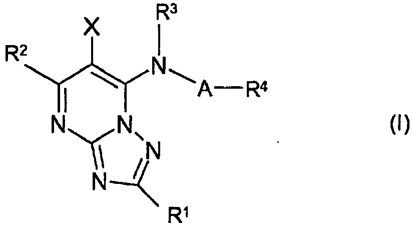 wherein
- X: is halogen;
- R¹: is selected from hydrogen, halogen, OH, CN, C₁-C₁₀-alkyl, C₃-C₁₀-cycloalkyl C₁-C₁₀-haloalkyl, C₁-C₁₀-alkoxy, C₁-C₆-alkoxy-C₁-C₆-alkyl, C₁-C₄-alkoxy-C₁-C₄-alkoxy, C₁-C₄-alkoxy-C₁-C₄-alkoxy-C₁-C₄-alkoxy, C₁-C₁₀-alkylthio, C₁-C₁₀-alkylsulfinyl, C₁-C₁₀-alkylsulfonyl, C₁-C₁₀-alkylamino, di(C₁-C₁₀-alkyl)amino, C₂-C₁₀-alkenyl, phenyl, phenoxy, benzyloxy, C₂-C₁₀-alkenyloxy, C₂-C₁₀-alkynyloxy or C₂-C₁₀-alkynyl, wherein C₁-C₁₀-alkylthio, C₁-C₁₀-alkylsulfinyl and C₁-C₁₀-alkylsulfonyl may be unsubstituted or partially or completely substituted with halogen and/or may carry a group selected from C₁-C₄-alkoxy, C₁-C₄-alkoxycarbonyl or COOH;
- R²: is selected from hydrogen, C₁-C₁₀-alkyl, C₁-C₁₀-haloalkyl, C₁-C₆-alkoxy-C₁-C₈-alkyl, C₃-C₁₀-cycloalkyl, C₂-C₁₀-alkenyl or C₂-C₁₀-alkynyl;
- R³: is selected from hydrogen, C₁-C₁₀-alkyl, C₁-C₁₀-haloalkyl, C₁-C₆-alkoxy-C₁-C₆-alkyl, C₁-C₁₀-alkylcarbonyl, C₁-C₁₀-alkoxycarbonyl or arylcarbonyl;
- A: is a single bond or a C₁-C₆-alkylene chain which may comprise one bridging heteroatom selected from oxygen or sulfur;
- R⁴: is selected from C₃-C₁₀-cycloalkyl, phenyl, naphthyl, 3- to 7-membered heterocyclyl, being unsubstituted or carrying 1, 2 or 3 radicals which are selected, independently from each other, from the group consisting of halogen, cyano, nitro, hydroxy, C₁-C₁₀-alkyl, C₁-C₁₀-alkoxy, C₁-C₁₀-haloalkyl, C₁-C₁₀-haloalkoxy, amino, C₁-C₁₀-alkylamino, di(C₁-C₁₀-alkyl)amino, C₁-C₁₀-alkylthio, C₁-C₁₀-alkylsulfinyl, C₁-C₁₀-alkylsulfonyl, C₃-C₈-cycloalkyl, C₃-C₈-cycloalkyl-C₁-C₄-alkyl, phenyl, phenyl-C₁-C₄-alkoxy and phenyloxy, wherein the five last-mentioned radicals for their part may be unsubstituted or may carry one, two or three substituents which are selected, independently from each other, from the group consisting of C₁-C₄-alkyl, C₁-C₄-alkoxy and halogen, and wherein the 3 to 7 membered heterocyclyl contains 1, 2 or 3 heteroatoms selected, Independently from each other, from the group consisting of oxygen, sulfur, nitrogen and a group NR^{a}, it being also possible for C₃-C₁₀-cycloalkyl, phenyl and 3- to 7-membered heterocyclyl to be fused-to a 5- to 7-membered saturated, unsaturated or aromatic carbocyclic ring or to a 5- to 7-membered heterocydic ring and said fused ring may be unsubstituted or may itself carry one, two, three, four, five or six substituents which are selected, independently from each other from the group consisting of halogen and C₁-C₄-alkyl; wherein R^{a} is hydrogen, C₁C₆-alkyl, C₃-C₆-alkenyl or C₃-C₆-alkynyl;
and/or an enantiomer, diastereomer, tautomer, solvate, crystalline form or veterinarily acceptable salt thereof alone or in combination with an additional parasiticidal agent for use in treating, controlling, preventing or protecting against infestation or infection by parasites wherein the compound is for oral, topical, or parenteral administration to an animal.

### BACKGROUND OF THE INVENTION

It Is generally a goal of agronomists and veterinarians to possess sufficient means to control and combat parasites when they attempt to invade or attack animals.

DD 55 956, DD 99 794 and FR 1567021 describe [1,2,4]-triazolo[1,5-a]pyrimidines of the general formula (X) having a pharmaceutical activity, 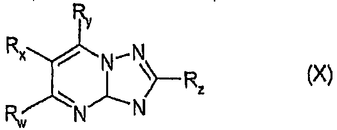 wherein R_{w}, R_{4z}may be hydrogen, lower alkyl, alkoxyalkyl, halogen, aryl or arylalkyl, Rₓ may be hydrogen, halogen, lower alkyl, lower alkenyl, arylalkyl or aryl and Ry may be an optionally substituted amino group, wherein the substituents are selected - Inter alia- from alkyl, cycloalkyl, alkenyl, hydroxylalkyl, alkylaminoalkyl, alkoxyalkyl, aryl, arylalkyl, heteroaryl or heteroaralkyl. WO 03/039259 describes substituted 1,2,4-triazolo[1,5-a]pyrimidine compounds having fungicidal activity. The insecticidal and acaricidal activity in crop protection of some of the compounds of formula I has been described in WO 2005/025315 (U.S. Patent Publication 2006/264446).

Activity of compounds against agricultural pests does not suggest their suitability for control of endo- and ectoparasites in and on animals which requires, for example, low, non-emetic dosages in the case of oral application, metabolic compatibility with the animal, low toxicity, and a safe handling.

So far, no 6-halogeno-[1,2,4]-triazolo-[1,5-a]pyrimidines have been described for their use in or on animals.

Surprisingly, it has now been found that compounds of formula I are suitable for combating endo- and ectoparasites In and on animals.

### SUMMARY OF THE INVENTION

It is an object of the present invention to provide compounds for use in treating, controlling, preventing or protecting against infestation or infection by parasites wherein the compound is for oral, topical, or parenteral administration to an animal.

Another object of the invention is to provide safer pesticides for animals.

Another object of the Invention is to provide pesticides for animals that may be used in lower doses than existing pesticides.

Another object of the invention is to provide pesticides for animals which provide a long residual control of the parasites.

These objects are met in whole or in part by the present invention.

The Invention also provides a compound of formula I alone or in combination with an additional parasiticial agent or a composition comprising a compound of formula I alone or in combination with an additional parasiticidal agent for use in treating, controlling, preventing and protecting animals against infection by parasites wherein the compound is for oral, topical or parenteral administration or application to an animal.

The description also provides a process for the preparation of a compostion for treating, controlling, preventing or protecting animals against infestation or Infection by parasites which comprises a parasiticidally effective amount of a compound of formula I alone or in combination with an additional parasiticidal agent or a composition comprising a compound of formula I alone or in combination with an additional parasiticidal agent.

The description also provides methods for removing parasites of vertebrates including endo- and ecto-parasites. Depending on the host-parasite pairs, a method according to the description can take on a therapeutic aspect, when it is intended to prevent or treat parasitoses which are pathogenic in their nature or their induced effects, or a method may be without any therapeutic purpose, when the treatment consists in combatting parasite which cause non-pathological effects in their hosts, such as signs of discomfort or an unsightly appearance.

The description also provides compositions which make possible the implementation of these methods
The invention also provides compounds for use in new methods for combatting parasites which make it possible to remove many ectoparasites of vertebrates, in particular of mammals.

The invention also provides compounds for use in a method which, by a single administration, makes it possible to remove endo- or ecto-parasites with an extremely high effectiveness for a long period of time.

### DETAILED DESCRIPTION OF THE INVENTION

### Parasiticidal compounds

In the substituents, the parasiticidal compounds used in the invention may have for a given constitution different spatial arrangement of the atoms, e.g. they may carry one or more centers of chirality, in which case they are present as mixtures of stereoisomers, such as enantiomers or diastereomers. The present invention provides both the pure stereoisomers, e.g. the pure enantiomers or diastereomers, and mixtures thereof.

Also encompassed by the invention are the tautomers, solvates and crystalline structures of the parasiticidal compounds. The crystalline structures also include polymorphic forms of the parasiticidal compounds.

Salts of the parasiticidal compounds which are suitable for the use according to the invention are especially veterinarily acceptable salts. They can be formed in a customary method, e.g. by treating the compound with an acid of the anion in question.

Suitable veterinarily useful salts are the salts of those cations and/or anions, respectively, which do not have any adverse effect on the action of the compounds according to the present invention, which are useful for combating harmful parasites. Thus, suitable cations include the ions of the alkali metals, such as lithium, sodium and potassium, of the alkaline earth metals, such as calcium, magnesium and barium, and of the transition metals, such as manganese, copper, zinc and iron, and also the ammonium ion which may, if desired, carry one to four C₁-C₄-alkyl substituents and/or one phenyl or benzyl substituent (examples of ammonium ions include but are not limited to diisopropylammonium, tetramethylammonium, tetrabutylammonium, trimethylbenzylammonium), phosphonium ions, sulfonium ions (which include but are not limited to tri(C₁-C₄-alkyl)sulfonium), and sulfoxonium ions (which include but are not limited to tri(C₁-C₄-alkyl)sulfoxonium).

Suitable anions include chloride, bromide, fluoride, hydrogen sulfate, sulfate, dihydrogen phosphate, hydrogen phosphate, phosphate, nitrate, hydrogen carbonate, carbonate, hexafluorosilicate, hexafluorophosphate, benzoate, and the anions of C₁-C₄-alkanoic acids, such as formate, acetate, propionate and butyrate. They can be formed by treating the compounds of formula I with an acid of the corresponding anion, such as hydrochloric acid, hydrobromic acid, sulfuric acid, phosphoric acid or nitric acid.

In the definition of formula I shown above, the substituents have the following meanings:
The organic moieties mentioned in the above definitions of the variables are - like the term halogen - collective terms for individual listings of the individual group members. The prefix Cₙ-Cₘ indicates in each case the possible number of carbon atoms in the group.

The term halogen denotes in each case fluorine, bromine, chlorine or iodine. In one embodiment of the invention, the halogen is fluorine or chlorine.

Examples of other meanings are:
The term "C₁-C₁₀-alkyl" as used herein and the alkyl moieties of alkylamino and dialkylamino refer to a saturated straight-chain or branched hydrocarbon group selected from the ranges consisting of having 1 to 10 carbon atoms, 1 to 6 carbon atoms and 1 to 4 carbon groups, respectively. Examples of C₁-C₁₀ alkyl include methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, heptyl, octyl, 2-ethylhexyl, nonyl and decyl and their isomers. C₁-C₄-alkyl means for example methyl, ethyl, propyl, 1-methylethyl, butyl, 1-methylpropyl, 2-methylpropyl or 1,1-dimethylethyl.

The term "C₁-C₆-alkylene chain which may comprise one heteroatom selected from the group consisting of oxygen and sulfur" as used herein refers to, for example, methanediyl, ethane-1,1-diyl, ethane-1,2-diyl, propane-1,1-diyl, propane-1,2-diyl, propane-1,3-diyl, propane-2,2-diyl, butane-1,1-diyl, butane-1,2-diyl, butane-1,3-diyl, butane-1,4-diyl, 2-methylpropane-1,3-diyl, 2-methylpropane-1,2-diyl, 2-methylpropane-1,1-diyl, 1-methylpropane-1,2-diyl, 1-methylpropane-2,2-diyl, 1-methylpropane-1,1-diyl, pentane-1,1-diyl, pentane-1,2-diyl, pentane-1,3-diyl, pentane-1,5-diyl, pentane-2,3-diyl, pentane-2,2-diyl, 1-methylbutane-1,1-diyl, 1-methylbutane-1,2-diyl, 1-methylbutane-1,3-diyl, 1-methylbutane-1,4-diyl, 2-methylbutane-1,1-diyl, 2-methylbutane-1,2-diyl, 2-methylbutane-1,3-diyl, 2-methylbutane-1,4-diyl, 2,2-dimethylpropane-1,1-diyl, 2,2-dimethylpropane-1,3-diyl, 1,1-dimethylpropane-1,3-diyl, 1,1-dimethylpropane-1,2-diyl, 2,3-dimethylpropane-1,3-diyl, 2,3-dimethylpropane-1,2-diyl, 1,3-dimethylpropane-1,3-diyl, hexane-1,1-diyl, hexane-1,2-diyl, hexane-1,3-diyl, hexane-1,4-diyl, hexane-1,5-diyl, hexane-1,6-diyl, hexane-2,5-diyl, 2-methylpentane-1,1-diyl, 1-methylpentane-1,2-diyl, 1-methylpentane-1,3-diyl, 1-methylpentane-1,4-diyl, 1-methylpentane-1,5-diyl, 2-methylpentane-1,1-diyl, 2-methylpentane-1,2-diyl, 2-methylpentane-1,3-diyl, 2-methylpentane-1,4-diyl, 2-methylpentane-1,5-diyl, 3-methylpentane-1,1-diyl, 3-methylpentane-1,2-diyl, 3-methylpentane-1,3-diyl, 3-methylpentane-1,4-diyl, 3-methylpentane-1,5-diyl, 1,1-dimethylbutane-1,2-diyl, 1,1-dimethylbutane-1,3-diyl, 1,1-dimethylbutane-1,4-diyl, 1,2-dimethylbutane-1,1-diyl, 1,2-dimethylbutane-1,2-diyl, 1,2-dimethylbutane-1,3-diyl, 1,2-dimethylbutane-1,4-diyl, 1,3-dimethylbutane-1,1-diyl, 1,3-dimethylbutane-1,2-diyl, 1,3-dimethylbutane-1,3-diyl, 1,3-dimethylbutane-1,4-diyl, 1-ethylbutane-1,1-diyl, 1-ethylbutane-1,2-diyl, 1-ethylbutane-1,3-diyl, 1-ethylbutane-1,4-diyl, 2-ethylbutane-1,1-diyl, 2-ethylbutane-1,2-diyl, 2-ethylbutane-1,3-diyl, 2-ethylbutane-1,4-diyl, 2-ethylbutane-2,3-diyl, 2,2-dimethylbutane-1,1-diyl, 2,2-dimethylbutane-1,3-diyl, 2,2-dimethylbutane-1,4-diyl, 1-isopropylpropane-1,1-diyl, 1-isopropylpropane-1,2-diyl, 1-isopropylpropane-1,3-diyl, 2-isopropylpropane-1,1-diyl, 2-isopropylpropane-1,2-diyl, 2-isopropylpropane-1,3-diyl, 1,2,3-trimethylpropane-1,1-diyl, 1,2,3-trimethylpropane-1,2-diyl or 1,2,3-trimethylpropane-1,3-diyl.

When the C₁-C₆-alkylene group comprises a heteroatom, the heteroatom can be arranged in the alkylene chain at any position or at the end of the chain so that it connects the alkylene chain to the radical R⁴. In one embodiment of the invention, the heteroatom is not arranged at the end of the alkylene chain. In another embodiment of the invention, the heteroatom is oxygen.

The term "C₁-C₁₀-haloalkyl" as used herein refers to a straight-chain or branched saturated alkyl group having 1 to 10 carbon atoms (as mentioned above), where some or all of the hydrogen atoms in these groups may be replaced by halogen atoms as mentioned above, for example C₁-C₄-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl and the like.

The term "C₁-C₂-fluoroalkyl" as used herein refers to a C₁-C₂-alkyl which carries 1, 2, 3, 4 or 5 fluorine atoms, for example difluoromethyl, trifluoromethyl, 1-fluoroethyl, 2-fluoroethyl, 2,2-difluoroethyl, 2,2,2-trifluoroethyl, 1,1,2,2-tetrafluoroethyl or pentafluoroethyl.

The term, "C₁-C₁₀-alkoxy" as used herein refers to a straight-chain or branched saturated alkyl group having 1 to 10 carbon atoms (as mentioned above) which is attached via an oxygen atom. Examples include C₁-C₆-alkoxy such as methoxy, ethoxy, OCH₂-C₂H₅, OCH(CH₃)₂, n-butoxy, OCH(CH₃)-C₂H₅, OCH₂-CH(CH₃)₂, OC(CH₃)₃, n-pentoxy, 1-methylbutoxy, 2-methylbutoxy, 3-methylbutoxy, 1,1-dimethylpropoxy, 1,2-dimethylpropoxy, 2,2-dimethyl-propoxy, 1-ethylpropoxy, n-hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy, 1-ethyl-2-methylpropoxy and the like.

The term "C₁-C₁₀-haloalkoxy" as used herein refers to a C₁-C₁₀-alkoxy group as mentioned above which is partially or fully substituted by fluorine, chlorine, bromine and/or iodine, i.e., for example, C₁-C₆-haloalkoxy such as chloromethoxy, dichloromethoxy, trichloromethoxy, fluoromethoxy, difluoromethoxy, trifluoromethoxy, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, pentafluoroethoxy, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, 2,2,3,3,3-pentafluoropropoxy, heptafluoropropoxy, 1-(fluoromethyl)-2-fluoroethoxy, 1-(chloromethyl)-2-chloroethoxy, 1-(bromomethyl)-2-bromoethoxy, 4-fluorobutoxy, 4-chlorobutoxy, 4-bromobutoxy, nonafluorobutoxy, 5-fluoro-1-pentoxy, 5-chloro-1-pentoxy, 5-bromo-1-pentoxy, 5-iodo-1-pentoxy, 5,5,5-trichloro-1-pentoxy, undecafluoropentoxy, 6-fluoro-1-hexoxy, 6-chloro-1-hexoxy, 6-bromo-1-hexoxy, 6-iodo-1-hexoxy, 6,6,6-trichloro-1-hexoxy or dodecafluorohexoxy. In one embodiment of the invention, "C₁-C₁₀-haloalkoxy" is selected from the group consisting of chloromethoxy, fluoromethoxy, difluoromethoxy, trifluoromethoxy, 2-fluoroethoxy, 2-chloroethoxy and 2,2,2-trifluoroethoxy.

The term "C₁-C₆-alkoxy-C₁-C₆-alkyl" as used herein refers to C₁-C₆-alkyl which is substituted by C₁-C₆-alkoxy as mentioned above, i.e., for example, CH₂-OCH₃, CH₂-OC₂H₅, n-propoxymethyl, CH₂-OCH(CH₃)₂, n-butoxymethyl, (1-methylpropoxy)methyl, (2-methylpropoxy)methyl, CH₂-OC(CH₃)₃, 2-(methoxy)ethyl, 2-(ethoxy)ethyl, 2-(n-propoxy)ethyl, 2-(1-methylethoxy)ethyl, 2-(n-butoxy)ethyl, 2-(1-methylpropoxy)ethyl, 2-(2-methylpropoxy)ethyl, 2-(1,1-dimethylethoxy)ethyl, 2-(methoxy)propyl, 2-(ethoxy)propyl, 2-(n-propoxy)propyl, 2-(1-methylethoxy)propyl, 2-(n-butoxy)propyl, 2-(1-methylpropoxy)propyl, 2-(2-methylpropoxy)propyl, 2-(1,1-dimethylethoxy)propyl, 3-(methoxy)propyl, 3-(ethoxy)propyl, 3-(n-propoxy)propyl, 3-(1-methylethoxy)propyl, 3-(n-butoxy)propyl, 3-(1-methylpropoxy)propyl, 3-(2-methylpropoxy)propyl, 3-(1,1-dimethylethoxy)propyl, 2-(methoxy)butyl, 2-(ethoxy)butyl, 2-(n-propoxy)butyl, 2-(1-methylethoxy)butyl, 2-(n-butoxy)butyl, 2-(1-methylpropoxy)butyl, 2-(2-methylpropoxy)butyl, 2-(1,1-dimethylethoxy)butyl, 3-(methoxy)butyl, 3-(ethoxy)butyl, 3-(n-propoxy)butyl, 3-(1-methylethoxy)butyl, 3-(n-butoxy)butyl, 3-(1-methylpropoxy)butyl, 3-(2-methylpropoxy)butyl, 3-(1,1-dimethylethoxy)butyl, 4-(methoxy)butyl, 4-(ethoxy)butyl, 4-(n-propoxy)butyl, 4-(1-methylethoxy)butyl, 4-(n-butoxy)butyl, 4-(1-methylpropoxy)butyl, 4-(2-methylpropoxy)butyl, 4-(1,1-dimethylethoxy)butyl and the like.

The term "C₁-C₆-alkoxy-C₁-C₆-alkoxy" as used herein refers to C₁-C₆-alkoxy which is substituted by C₁-C₆-alkoxy as mentioned above, i.e., for example, OCH₂-OCH₃, OCH₂-OC₂H₅, n-propoxymethoxy, OCH₂-OCH(CH₃)₂, n-butoxymethoxy, (1-methylpropoxy)methoxy, (2-methylpropoxy)methoxy, OCH₂-OC(CH₃)₃, 2-(methoxy)ethoxy, 2-(ethoxy)ethoxy, 2-(n-propoxy)ethoxy, 2-(1-methylethoxy)ethoxy, 2-(n-butoxy)ethoxy, 2-(1-methylpropoxy)ethoxy, 2-(2-methylpropoxy)ethoxy, 2-(1,1-dimethylethoxy)ethoxy, 2-(methoxy)propoxy, 2-(ethoxy)propoxy, 2-(n-propoxy)propoxy, 2-(1-methylethoxy)propoxy, 2-(n-butoxy)propoxy, 2-(1-methylpropoxy)propoxy, 2-(2-methylpropoxy)propoxy, 2-(1,1-dimethylethoxy)propoxy, 3-(methoxy)propoxy, 3-(ethoxy)propoxy, 3-(n-propoxy)propoxy, 3-(1-methylethoxy)propv, 3-(n-butoxy)propoxy, 3-(1-methylpropoxy)propoxy, 3-(2-methylpropoxy)propoxy, 3-(1,1-dimethylethoxy)propoxy, 2-(methoxy)butoxy, 2-(ethoxy)butoxy, 2-(n-propoxy)butoxy, 2-(1-methylethoxy)butoxy, 2-(n-butoxy)butoxy, 2-(1-methylpropoxy)butoxy, 2-(2-methylpropoxy)butoxy, 2-(1,1-dimethylethoxy)butoxy, 3-(methoxy)butoxy, 3-(ethoxy)butoxy, 3-(n-propoxy)butoxy, 3-(1-methylethoxy)butoxy, 3-(n-butoxy)butoxy, 3-(1-methylpropoxy)butoxy, 3-(2-methylpropoxy)butoxy, 3-(1,1-dimethylethoxy)butoxy, 4-(methoxy)butoxy, 4-(ethoxy)butoxy, 4-(n-propoxy)butoxy, 4-(1-methylethoxy)butoxy, 4-(n-butoxy)butoxy, 4-(1-methylpropoxy)butoxy, 4-(2-methylpropoxy)butoxy, 4-(1,1-dimethylethoxy)butoxy and the like.

The term "C₁-C₄-alkoxy-C₁-C₄-alkoxy-C₁-C₄-alkoxy" as used herein refers to C₁-C₄-alkoxy which is substituted by C₁-C₄-alkoxy-C₁-C₄-alkoxy as mentioned above, i.e., for example, 2-(2-methoxyethyloxy)ethyloxy, 2-(2-ethoxyethyloxy)ethyloxy.

The term "C₁-C₁₀-alkylcarbonyl" as used herein refers to a straight-chain or branched saturated alkyl group having 1 to 10 carbon atoms (as mentioned above) bonded via the carbon atom of the carbonyl group at any bond in the alkyl group. Examples include C₁-C₆-alkylcarbonyl such as CO-CH₃, CO-C₂H₅, n-propylcarbonyl, 1-methylethylcarbonyl, n-butylcarbonyl, 1-methylpropylcarbonyl, 2-methylpropylcarbonyl, 1,1-dimethylethylcarbonyl, n-pentylcarbonyl, 1-methylbutylcarbonyl, 2-methylbutylcarbonyl, 3-methylbutylcarbonyl, 1,1-dimethylpropylcarbonyl, 1,2-dimethylpropylcarbonyl, 2,2-dimethylpropylcarbonyl, 1-ethylpropylcarbonyl, n-hexylcarbonyl, 1-methylpentylcarbonyl, 2-methylpentylcarbonyl, 3-methylpentylcarbonyl, 4-methylpentylcarbonyl, 1,1-dimethylbutylcarbonyl, 1,2-dimethylbutylcarbonyl, 1,3-dimethylbutylcarbonyl, 2,2-dimethylbutylcarbonyl, 2,3-dimethylbutylcarbonyl, 3,3-dimethylbutylcarbonyl, 1-ethylbutylcarbonyl, 2-ethylbutylcarbonyl, 1,1,2-trimethylpropylcarbonyl, 1,2,2-trimethylpropylcarbonyl, 1-ethyl-1-methylpropylcarbonyl or 1-ethyl-2-methylpropylcarbonyl and the like.

The term "C₁-C₁₀-alkoxycarbonyl" as used herein refers to a straight-chain or branched alkoxy group (as mentioned above) having 1 to 10 carbon atoms attached via the carbon atom of the carbonyl group. Examples include (C₁-C₆-alkoxy)carbonyl, for example CO-OCH₃, CO-OC₂H₅, COO-CH₂-C₂H₅, CO-OCH(CH₃)₂, n-butoxycarbonyl, CO-OCH(CH₃)-C₂H₅, CO-OCH₂-CH(CH₃)₂, CO-OC(CH₃)₃, n-pentoxycarbonyl, 1-methylbutoxycarbonyl, 2-methylbutoxycarbonyl, 3-methylbutoxycarbonyl, 2,2-dimethylpropoxycarbonyl, 1-ethylpropoxycarbonyl, n-hexoxycarbonyl, 1,1-dimethylpropoxycarbonyl, 1,2-dimethylpropoxycarbonyl, 1-methylpentoxycarbonyl, 2-methylpentoxycarbonyl, 3-methylpentoxycarbonyl, 4-methylpentoxycarbonyl, 1,1-dimethylbutoxycarbonyl, 1,2-dimethylbutoxycarbonyl, 1,3-dimethylbutoxycarbonyl, 2,2-dimethylbutoxycarbonyl, 2,3-dimethylbutoxycarbonyl, 3,3-dimethylbutoxycarbonyl, 1-ethylbutoxycarbonyl, 2-ethylbutoxycarbonyl, 1,1,2-trimethylpropoxycarbonyl, 1,2,2-trimethylpropoxycarbonyl, 1-ethyl-1-methylpropoxycarbonyl or 1-ethyl-2-methylpropoxycarbonyl.

The term "C₁-C₁₀-alkylthio (C₁-C₁₀-alkylsulfanyl: C₁-C₁₀-alkyl-S-)" as used herein refers to a straight-chain or branched saturated alkyl group having 1 to 10 carbon atoms (as mentioned above) which is attached via a sulfur atom, for example C₁-C₄-alkylthio such as methylthio, ethylthio, propylthio, 1-methylethylthio, butylthio, 1-methylpropylthio, 2-methylpropylthio or 1,1-dimethylethylthio.

The term "C₁-C₁₀-alkylsulfinyl" (C₁-C₁₀-alkyl-S(=O)-), as used herein refers to a straight-chain or branched saturated hydrocarbon group (as mentioned above) having 1 to 10 carbon atoms bonded through the sulfur atom of the sulfinyl group at any bond in the alkyl group. Examples include C₁-C₆-alkylsulfinyl: SO-CH₃, SO-C₂H₅, n-propylsulfinyl, 1-methylethylsulfinyl, n-butylsulfinyl, 1-methylpropylsulfinyl, 2-methylpropylsulfinyl, 1,1-dimethylethylsulfinyl, n-pentylsulfinyl, 1-methylbutylsulfinyl, 2-methylbutylsulfinyl, 3-methylbutylsulfinyl, 1,1-dimethylpropylsulfinyl, 1,2-dimethylpropylsulfinyl, 2,2-dimethylpropylsulfinyl, 1-ethylpropylsulfinyl, n-hexylsulfinyl, 1-methylpentylsulfinyl, 2-methylpentylsulfinyl, 3-methylpentylsulfinyl, 4-methylpentylsulfinyl, 1,1-dimethylbutylsulfinyl, 1,2-dimethylbutylsulfinyl, 1,3-dimethylbutylsulfinyl, 2,2-dimethylbutylsulfinyl, 2,3-dimethylbutylsulfinyl, 3,3-dimethylbutylsulfinyl, 1-ethylbutylsulfinyl, 2-ethylbutylsulfinyl, 1,1,2-trimethylpropylsulfinyl, 1,2,2-trimethylpropylsulfinyl, 1-ethyl-1-methylpropylsulfinyl or 1-ethyl-2-methylpropylsulfinyl.

The term "C₁-C₁₀-alkylsulfonyl" (C₁-C₁₀-alkyl-S(=O)₂-) as used herein refers to a straight-chain or branched saturated alkyl group having 1 to 10 carbon atoms (as mentioned above) which is bonded via the sulfur atom of the sulfonyl group at any bond in the alkyl group. Examples include C₁-C₆-alkylsulfonyl such as SO₂-CH₃, SO₂-C₂H₅, n-propylsulfonyl, SO₂-CH(CH₃)₂, n-butylsulfonyl, 1-methylpropylsulfonyl, 2-methylpropylsulfonyl, SO₂-C(CH₃)₃, n-pentylsulfonyl, 1-methylbutylsulfonyl, 2-methylbutylsulfonyl, 3-methylbutylsulfonyl, 1,1-dimethylpropylsulfonyl, 1,2-dimethylpropylsulfonyl, 2,2-dimethylpropylsulfonyl, 1-ethylpropylsulfonyl, n-hexylsulfonyl, 1-methylpentylsulfonyl, 2-methylpentylsulfonyl, 3-methylpentylsulfonyl, 4-methylpentylsulfonyl, 1,1-dimethylbutylsulfonyl, 1,2-dimethylbutylsulfonyl, 1,3-dimethylbutylsulfonyl, 2,2-dimethylbutylsulfonyl, 2,3-dimethylbutylsulfonyl, 3,3-dimethylbutylsulfonyl, 1-ethylbutylsulfonyl, 2-ethylbutylsulfonyl, 1,1,2-trimethylpropylsulfonyl, 1,2,2-trimethylpropylsulfonyl, 1-ethyl-1-methylpropylsulfonyl or 1-ethyl-2-methylpropylsulfonyl and the like.

The term "C₂-C₁₀-alkenyl" as used herein refers to a straight-chain or branched unsaturated hydrocarbon group having 2 to 10 carbon atoms and a double bond in any position, such as ethenyl, 1-propenyl, 2-propenyl, 1-methyl-ethenyl, 1-butenyl, 2-butenyl, 3-butenyl, 1-methyl-1-propenyl, 2-methyl-1-propenyl, 1-methyl-2-propenyl, 2-methyl-2-propenyl; 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl.

The term "C₂-C₁₀-alkynyl" as used herein refers to a straight-chain or branched unsaturated hydrocarbon group having 2 to 10 carbon atoms and containing at least one triple bond, such as ethynyl, prop-1-yn-1-yl, prop-2-yn-1-yl, n-but-1-yn-1-yl, n-but-1-yn-3-yl, n-but-1-yn-4-yl, n-but-2-yn-1-yl, n-pent-1-yn-1-yl, n-pent-1-yn-3-yl, n-pent-1-yn-4-yl, n-pent-1-yn-5-yl, n-pent-2-yn-1-yl, n-pent-2-yn-4-yl, n-pent-2-yn-5-yl, 3-methylbut-1-yn-3-yl, 3-methylbut-1-yn-4-yl, n-hex-1-yn-1-yl, n-hex-1-yn-3-yl, n-hex-1-yn-4-yl, n-hex-1-yn-5-yl, n-hex-1-yn-6-yl, n-hex-2-yn-1-yl, n-hex-2-yn-4-yl, n-hex-2-yn-5-yl, n-hex-2-yn-6-yl, n-hex-3-yn-1-yl, n-hex-3-yn-2-yl, 3-methylpent-1-yn-1-yl, 3-methylpent-1-yn-3-yl, 3-methylpent-1-yn-4-yl, 3-methylpent-1-yn-5-yl, 4-methylpent-1-yn-1-yl, 4-methylpent-2-yn-4-yl or 4-methylpent-2-yn-5-yl and the like.

The term "C₃-C₁₀-cycloalkyl" as used herein refers to a monocyclic hydrocarbon radical having 3 to 10 carbon atoms, in particular 3 to 8 carbon atoms, e.g. cyclopropyl, cyclobutyl, cyclopentyl, cyclohexyl, cycloheptyl, cyclooctyl, cyclononyl or cyclodecyl.

The term "C₃-C₈-cycloalkyl-C₁-C₄-alkyl" as used herein refers to a C₁-C₄-alkyl which carries a C₃-C₈-cycloalkyl radical as defined above, for example cyclopropylmethyl, 1-cyclopropylethyl, 2-cyclopropylethyl, 1-cyclopropylprop-1-yl, 2-cyclopropylprop-1-yl, 3-cyclopropylprop-1-yl, 1-cyclopropylbut-1-yl, 2-cyclopropylbut-1-yl, 3-cyclopropylbut-1-yl, 4-cyclopropylbut-1-yl, 1-cyclopropylbut-2-yl, 2-cyclopropylbut-2-yl, 3-cyclopropylbut-2-yl, 3-cyclopropylbut-2-yl, 4-cyclopropylbut-2-yl, 1-(cyclopropylmethyl)-eth-1-yl, 1-(cyclopropylmethyl)-1-(methyl)-eth-1-yl, 1-(cyclopropylmethyl)-prop-1-yl, cyclobutylmethyl, 1-cyclobutylethyl, 2-cyclobutylethyl, 1-cyclobutylprop-1-yl, 2-cyclobutylprop-1-yl, 3-cyclobutylprop-1-yl, 1-cyclobutylbut-1-yl, 2-cyclobutylbut-1-yl, 3-cyclobutylbut-1-yl, 4-cyclobutylbut-1-yl, 1-cyclobutylbut-2-yl, 2-cyclobutylbut-2-yl, 3-cyclobutylbut-2-yl, 3-cyclobutylbut-2-yl, 4-cyclobutylbut-2-yl, 1-(cyclobutylmethyl)eth-1-yl, 1-(cyclobutylmethyl)-1-(methyl)-eth-1-yl, 1-(cyclobutylmethyl)prop-1-yl, cyclopentylmethyl, 1-cyclopentylethyl, 2-cyclopentylethyl, 1-cyclopentylprop-1-yl, 2-cyclopentylprop-1-yl, 3-cyclopentylprop-1-yl, 1-cyclopentylbut-1-yl, 2-cyclopentylbut-1-yl, 3-cyclopentylbut-1-yl, 4-cyclopentylbut-1-yl, 1-cyclopentylbut-2-yl, 2-cyclopentylbut-2-yl, 3-cyclopentylbut-2-yl, 3-cyclopentylbut-2-yl, 4-cyclopentylbut-2-yl, 1-(cyclopentylmethyl)-eth-1-yl, 1-(cyclopentylmethyl)-1-(methyl)-eth-1-yl, 1-(cyclopentylmethyl)-prop-1-yl, cyclohexylmethyl, 1-cyclohexylethyl, 2-cyclohexylethyl, 1-cyclohexylprop-1-yl, 2-cyclohexylprop-1-yl, 3-cyclohexylprop-1-yl, 1-cyclohexylbut-1-yl, 2-cyclohexylbut-1-yl, 3-cyclohexylbut-1-yl, 4-cyclohexylbut-1-yl, 1-cyclohexylbut-2-yl, 2-cyclohexylbut-2-yl, 3-cyclohexylbut-2-yl, 3-cyclohexylbut-2-yl, 4-cyclohexylbut-2-yl, 1-(cyclohexylmethyl)-eth-1-yl, 1-(cyclohexylmethyl)-1-(methyl)-eth-1-yl, 1-(cyclohexylmethyl)-prop-1-yl, cycloheptylmethyl, 1-cycloheptylethyl, 2-cycloheptylethyl, 1-cycloheptylprop-1-yl, 2-cycloheptylprop-1-yl, 3-cycloheptylprop-1-yl, 1-cycloheptylbut-1-yl, 2-cycloheptylbut-1-yl, 3-cycloheptylbut-1-yl, 4-cycloheptylbut-1-yl, 1-cycloheptylbut-2-yl, 2-cycloheptylbut-2-yl, 3-cycloheptylbut-2-yl, 3-cycloheptylbut-2-yl, 4-cycloheptylbut-2-yl, 1-(cycloheptylmethyl)-eth-1-yl, 1-(cycloheptylmethyl)-1-(methyl)-eth-1-yl, 1-(cycloheptylmethyl)-prop-1-yl, cyclooctylmethyl, 1-cyclooctylethyl, 2-cyclooctylethyl, 1-cyclooctylprop-1-yl, 2-cyclooctylprop-1-yl, 3-cyclooctylprop-1-yl, 1-cyclooctylbut-1-yl, 2-cyclooctylbut-1-yl, 3-cyclooctylbut-1-yl, 4-cyclooctylbut-1-yl, 1-cyclooctylbut-2-yl, 2-cyclooctylbut-2-yl, 3-cyclooctylbut-2-yl, 3-cyclooctylbut-2-yl, 4-cyclooctylbut-2-yl, 1-(cyclooctylmethyl)-eth-1-yl, 1-(cyclooctylmethyl)-1-(methyl)-eth-1-yl or 1-(cyclooctylmethyl)-prop-1-yl.

The term "phenyl-C₁-C₄-alkyl" as used herein refers to C₁-C₄-alkyl which is substituted by phenyl, which may for its part be unsubstituted or carries one, two or three substituents, such as benzyl, 1-phenylethyl, 2-phenylethyl, 1-phenylprop-1-yl, 2-phenylprop-1-yl, 3-phenylprop-1-yl, 1-phenylbut-1-yl, 2-phenylbut-1-yl, 3-phenylbut-1-yl, 4-phenylbut-1-yl, 1-phenylbut-2-yl, 2-phenylbut-2-yl, 3-phenylbut-2-yl, 4-phenylbut-2-yl, 1-(benzyl)eth-1-yl, 1-(benzyl)-1-(methyl)eth-1-yl or 1-(benzyl)-prop-1-yl. In one embodiment of the invention, "phenyl-C₁-C₄-alkyl" is selected from the group consisting of benzyl, 1-phenylethyl, 2-phenylethyl, (R)-1-phenylethyl and (S)-1-phenylethyl.

The term "heterocyclyl" as used herein refers to a 3- to 7-membered heterocyclic radical which has 3, 4, 5, 6 or 7 ring members, where 1, 2, 3 or 4 of these ring members are heteroatoms selected, independently from each other, from the group consisting of oxygen, nitrogen, sulfur and a group NR⁵, wherein R⁵ has the meanings as defined in R³ above. The heterocycle may be a carbon-bonded heterocycle or may be bonded via a heteroatom. The heterocycle may be aromatic (heteroaryl) or partially or fully saturated.

Moreover, the heterocyclyl radical may be fused to a 5-to 7-membered saturated, unsaturated or aromatic carbocyclic ring or to a 5- to 7-membered heterocyclic ring which may carry for their part one, two, three, four, five or six substituents which are selected, independently from one another from the group consisting of halogen such as fluorine, chlorine, bromine and C₁-C₄-alkyl such as methyl.

Examples for monocyclic heteroaromatic rings include triazinyl, pyrazinyl, pyrimidyl, pyridazinyl, pyridyl, thienyl, furyl, pyrrolyl, pyrazolyl, imidazolyl, triazolyl, tetrazolyl, thiazolyl, oxazolyl, thiadiazolyl, oxadiazolyl, isothiazolyl or isoxazolyl.

Examples for non-aromatic rings include pyrrolidinyl, pyrazolinyl, imidazolinyl, pyrrolinyl, pyrazolinyl, imidazolinyl, tetrahydrofuranyl, dihydrofuranyl, 1,3-dioxolanyl, dioxolenyl, thiolanyl, dihydrothiophenyl, oxazolidinyl, isoxazolidinyl, oxazolinyl, isoxazolinyl, thiazolinyl, isothiazolinyl, thiazolidinyl, isothiazolidinyl, oxathiolanyl, piperidinyl, piperazinyl, pyranyl, dihydropyranyl, tetrahydropyranyl, dioxanyl, thiopyranyl, dihydrothiopyranyl, tetrahydrothiopyranyl, morpholinyl, thiazinyl and the like.

The term "fused to a 5- to 7-membered saturated, unsaturated or aromatic carbocyclic ring or to a 5- to 7-membered heterocyclic ring" as used herein refers to a cyclic radical which carries a fused saturated C₅-C₇-carbocycle as defined above, a mono- or diunsaturated C₅-C₇-carbocycle or phenyl or to a 5-7-membered heterocyclic ring as defined above.

Examples for C₃-C₁₀-cycloalkyl fused with a 5- to 7-membered saturated, unsaturated or aromatic carbocyclic ring are indan-1-yl, indan-2-yl, 1,2,3,4-tetrahydronaphthalen-1-yl, 1,2,3,4-tetrahydronaphthalen-2-yl, decalin-1-yl, decalin-2-yl or hydrindanyl and the like.

Examples for phenyl fused with a 5- to 7-membered saturated, unsaturated or aromatic carbocyclic ring or to a 5- to 7-membered heterocyclic ring are indan-5-yl, indan-6-yl, dihydronaphthalen-5-yl, dihydronaphthalen-6-yl, 1,2,3,4-tetrahydronaphthalen-5-yl, 1,2,3,4-tetrahydronaphthalen-6-yl, quinolinyl, isoquinolinyl, indolyl, indolizinyl, isoindolyl, indazolyl, benzofuryl, benzthienyl, benzthiazolyl, benzoxazolyl, 1,3-benzodioxolyl, 1,4-benzodioxanyl, benzimidazolyl, dihydroindolyl, dihydroindolizinyl, dihydroisoindolyl, dihydrochinolinyl, dihydroisochinolinyl, chromenyl, chromanyl and the like.

Examples for 3- to 7-membered heterocyclyl carrying a fused-on 5- to 7-membered saturated, unsaturated or aromatic carbocyclic ring or a 5- to 7-membered heterocyclic ring are quinolinyl, isoquinolinyl, indolyl, indolizinyl, isoindolyl, indazolyl, benzofuryl, benzthienyl, benzthiazolyl, benzoxazolyl, 1,3-benzodioxolyl, 1,4-benzodioxanyl, benzimidazolyl, dihydroindolyl, dihydroindolizinyl, dihydroisoindolyl, dihydrochinolinyl, dihydroisochinolinyl, chromenyl, chromanyl and the like.

Embodiments of 6-halogeno-[1,2,4]-triazolo-[1,5a]-pyrimidine compounds used in the invention

One embodiment of the invention is given to the compounds for use wherein the substituents have the following meaning:
wherein compounds of formula I, A is a single bond.

wherein compounds of formula I, A is a C₁-C₃-alkylene chain. In one embodiment of the invention, the C₁-C₃ alkylene chain is selected from CH₂, C₂H₄, CH(CH₃), and CH(C₂H₅).

wherein in compounds of formula I, the radical R⁴ is selected from phenyl, phenyl-C₁-C₄-alkyl and C₃-C₈-cycloalkyl, wherein each phenyl and C₃-C₈-cycloalkyl group may be unsubstituted or may carry one or two substituents as defined above.

In one embodiment for the radical R⁴, the radical is cis-cyclohexyl when there is a substituent at the 4-position of the cyclohexyl, which may carry one or two further substituents as defined above.

In another embodiment for the radical R⁴, the radical is cyclohexyl, which carries a substituent in the 4-position such as C₁-C₄-alkyl or C₁-C₄-haloalkyl.

In yet another embodiment for the radical R⁴, the radical is 1-phenylethyl, which is an S-enantiomer.

In still another embodiment for the radical R⁴, the radical is 1-phenylethyl, which carries a substituent in the 4-position of the phenyl, wherein the substituent is selected from the group consisting of halogen, C₁-C₄-alkyl, C₁-C₄-alkoxy, C₁-C₄-haloalkyl and C₁-C₄-haloalkoxy.

In one embodiment for the radical R¹, the radical is selected from hydrogen, C₁-C₄-alkyl, C₁-C₄-haloalkyl, C₁-C₄-alkoxy, C₁-C₄-alkylthio, C₁-C₄-alkylsulfinyl and C₁-C₄-alkylsulfonyl. In another embodiment for the radical R¹, the radical is hydrogen.

In one embodiment for the radical R², the radical is selected from C₁-C₄-alkyl, C₁-C₄-alkoxy and C₁-C₄-haloalkyl. In another embodiment for the radical R², the radical is selected from C₁-C₂-alkyl, C₁-C₂-alkoxy and C₁-C₂-fluoroalkyl.

In one embodiment for the radical R³, the radical is selected from hydrogen or C₁-C₄-alkoxy-C₁-C₄-alkyl. In another embodiment for the radical R³, the radical is hydrogen.

In one embodiment of the invention, the radical X in the compounds of formula I is chlorine.

In another embodiment of the invention, the compounds of the invention are the 6-chloro-[1,2,4]triazolo-[1,5-a]pyrimidine compounds of formula I, wherein X is Cl and R¹ and R³ are H (as shown in formula la), and wherein R⁴-A and R² have the meanings given in Table A.

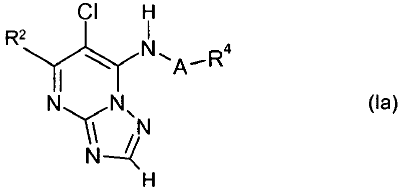

**TABLE A**

| No. | R² | R⁴-A |
|---|---|---|
| A-1. | CH₃ | 4-CH₃-cyclohexyl- |
| A-2. | CH₃ | cis-4-CH₃-cyclohexyl- |
| A-3. | CH₃ | trans-4-CH₃-cyclohexyl- |
| A-4. | CH₃ | 4-C₂H₅-cyclohexyl- |
| A-5. | CH₃ | cis-4-C₂H₅-cyclohexyl- |
| A-6. | CH₃ | trans-4-C₂H₅-cyclohexyl- |
| A-7. | CH₃ | 4-n-propyl-cyclohexyl- |
| A-8. | CH₃ | cis-4-n-propyl-cyclohexyl- |
| A-9. | CH₃ | trans-4-n-propyl-cyclohexyl- |
| A-10. | CH₃ | 4-isopropyl-cyclohexyl- |
| A-11. | CH₃ | cis-4-isopropyl-cyclohexyl- |
| A-12. | CH₃ | trans-4-isopropyl-cyclohexyl- |
| A-13. | CH₃ | 4-n-butylcyclohexyl- |
| A-14. | CH₃ | cis-4-n-butylcyclohexyl- |
| A-15. | CH₃ | trans-4-n-butylcyclohexyl- |
| A-16. | CH₃ | 4-tert-butylcyclohexyl- |
| A-17. | CH₃ | cis-4-tert-butylcyclohexyl- |
| A-18. | CH₃ | trans-4-tert-butylcyclohexyl- |
| A-19. | CH₃ | 4-(2-butyl)cyclohexyl- |
| A-20. | CH₃ | cis-4-(2-butyl)cyclohexyl- |
| A-21. | CH₃ | trans-4-(2-butyl)cyclohexyl- |
| A-22. | CH₃ | 4-(cyclohexyl-C(CH₃)₂)-cyclohexyl- |
| A-23. | CH₃ | cis-4-(cyclohexyl-C(CH₃)₂)-cyclohexyl- |
| A-24. | CH₃ | trans-4-(cyclohexyl-C(CH₃)₂)-cyclohexyl- |
| A-25. | CH₃ | C₆H₅- |
| A-26. | CH₃ | 4-F-C₆H₄- |
| A-27. | CH₃ | 4-Cl-C₆H₄- |
| A-28. | CH₃ | 4-Br-C₆H₄- |
| A-29. | CH₃ | 4-(C₆H₅)-C₆H₄- |
| A-30. | CH₃ | 4-phenoxyphenyl |
| A-31. | CH₃ | benzyl |
| A-32. | CH₃ | 4-CH₃-C₆H₄-CH₂- |
| A-33. | CH₃ | 4-C₂H₅-C₆H₄-CH₂- |
| A-34. | CH₃ | 4-n-C₃H₇-C₆H₄-CH₂- |
| A-35. | CH₃ | 4-isopropyl-C₆H₄-CH₂- |
| A-36. | CH₃ | 4-n-C₄H₉-C₆H₄-CH₂- |
| A-37. | CH₃ | 4-isobutyl-C₆H₄-CH₂- |
| A-38. | CH₃ | 4-tert-buyl-C₆H₄CH₂- |
| A-39. | CH₃ | 4-F₃C-C₆H₄CH₂- |
| A-40. | CH₃ | 4-methoxy-C₆H₄CH₂- |
| A-41. | CH₃ | 4-ethoxy-C₆H₄CH₂- |
| A-42. | CH₃ | 4-n-propoxy-C₆H₄CH₂- |
| A-43. | CH₃ | 4-isopropoxy-C₆H₄CH₂- |
| A-44. | CH₃ | 4-n-butoxy-C₆H₄CH₂- |
| A-45. | CH₃ | 4-tert-butoxy-C₆H₄CH₂- |
| A-46. | CH₃ | 4-F-C₆H₄CH₂- |
| A-47. | CH₃ | 4-Cl-C₆H₄CH₂- |
| A-48. | CH₃ | 4-Br-C₆H₄CH₂- |
| A-49. | CH₃ | 3,4-F₂-C₆H₃CH₂- |
| A-50. | CH₃ | 3,4-Cl₂-C₆H₃CH₂- |
| A-51. | CH₃ | 3,4-Br₂-C₆H₃CH₂- |
| A-52. | CH₃ | 4-(4-CH₃-C₆H₄-O)-C₆H₄-CH₂- |
| A-53. | CH₃ | 4-(4-C₂H₅-C₆H₄-O)-C₆H₄-CH₂- |
| A-54. | CH₃ | 4-(4-n-C₃H₇-C₆H₄-O)-C₆H₄-CH₂- |
| A-55. | CH₃ | 4-(4-iso-C₃H₇-C₆H₄-O)-C₆H₄-CH₂- |
| A-56. | CH₃ | 4-(4-n-C₄H₉-C₆H₄-O)-C₆H₄-CH₂- |
| A-57. | CH₃ | 4-(4-iso-C₄H₉-C₆H₄-O)-C₆H₄-CH₂- |
| A-58. | CH₃ | 4-(4-tert-C₄H₉-C₆H₄-O)-C₆H₄-CH₂- |
| A-59. | CH₃ | 4-(4-H₃C-O-C₆H₄-O)-C₆H₄-CH₂- |
| A-60. | CH₃ | 4-(4-H₃C-H₂C-O-C₆H₄-O)-C₆H₄-CH₂- |
| A-61. | CH₃ | 4-(4-H₃C-H₂C-H₂C-O-C₆H₄-O)-C₆H₄-CH₂- |
| A-62. | CH₃ | 4-(4-H₃C-H₂C-H₂C-H₂C-O-C₆H₄-O)-C₆H₄-CH₂- |
| A-63. | CH₃ | 4-(4-tert-butoxy-C₆H₄-O)-C₆H₄-CH₂- |
| A-64. | CH₃ | 4-(4-Cl-C₆H₄-O)-C₆H₄-CH₂- |
| A-65. | CH₃ | 4-(4-Br-C₆H₄-O)-C₆H₄-CH₂- |
| A-66. | CH₃ | 4-(3-CH₃-C₆H₄-O)-C₆H₄-CH₂- |
| A-67. | CH₃ | 4-(3-C₂H₅-C₆H₄-O)-C₆H₄-CH₂- |
| A-68. | CH₃ | 4-(3-n-C₃H₇-C₆H₄-O)-C₆H₄-CH₂- |
| A-69. | CH₃ | 4-(3-iso-C₃H₇-C₆H₄-O)-C₆H₄-CH₂- |
| A-70. | CH₃ | 4-(3-n-C₄H₉-C₆H₄-O)-C₆H₄-CH₂- |
| A-71. | CH₃ | 4-(3-iso-C₄H₉-C₆H₄-O)-C₆H₄-CH₂- |
| A-72. | CH₃ | 4-(3-tert-butyl-C₆H₄-O)-C₆H₄-CH₂- |
| A-73. | CH₃ | 4-(3-H₃C-O-C₆H₄-O)-C₆H₄-CH₂- |
| A-74. | CH₃ | 4-(3-H₃C-H₂C-O-C₆H₄-O)-C₆H₄-CH₂- |
| A-75. | CH₃ | 4-(3-H₃C-H₂C-H₂C-O-C₆H₄-O)-C₆H₄-CH₂- |
| A-76. | CH₃ | 4-(3-H₃C-H₂C-H₂C-H₂C-O-C₆H₄-O)-C₆H₄-CH₂- |
| A-77. | CH₃ | 4-(3-tert-butoxy-C₆H₄-O)-C₆H₄-CH₂- |
| A-78. | CH₃ | 4-(3-F-C₆H₄-O)-C₆H₄-CH₂- |
| A-79. | CH₃ | 4-(3-Cl-C₆H₄-O)-C₆H₄-CH₂- |
| A-80. | CH₃ | 4-(3-Br-C₆H₄-O)-C₆H₄-CH₂- |
| A-81. | CH₃ | 4-(2-CH₃-C₆H₄-O)-C₆H₄-CH₂- |
| A-82. | CH₃ | 4-(2-C₂H₅-C₆H₄-O)-C₆H₄-CH₂- |
| A-83. | CH₃ | 4-(2-n-C₃H₇-C₆H₄-O)-C₆H₄-CH₂- |
| A-84. | CH₃ | 4-(2-iso-C₃H₇-C₆H₄-O)-C₆H₄-CH₂- |
| A-85. | CH₃ | 4-(2-n-C₄H₉-C₆H₄-O)-C₆H₄-CH₂- |
| A-86. | CH₃ | 4-(2-iso-C₄H₉-C₆H₄-O)-C₆H₄-CH₂- |
| A-87. | CH₃ | 4-(2-tert-butyl-C₆H₄-O)-C₆H₄-CH₂- |
| A-88. | CH₃ | 4-(2-H₃C-O-C₆H₄-O)-C₆H₄-CH₂- |
| A-89. | CH₃ | 4-(2-H₃C-H₂C-O-C₆H₄-O)-C₆H₄-CH₂- |
| A-90. | CH₃ | 4-(2-H₃C-H₂C-H₂C-O-C₆H₄-O)-C₆H₄-CH₂- |
| A-91. | CH₃ | 4-(2-H₃C-H₂C-H₂C-H₂C-O-C₆H₄-O)-C₆H₄-CH₂- |
| A-92. | CH₃ | 4-(2-tert-butoxy-C₆H₄-O)-C₆H₄-CH₂- |
| A-93. | CH₃ | 4-(2-F-C₆H₄-O)-C₆H₄-CH₂- |
| A-94. | CH₃ | 4-(2-Cl-C₆H₄-O)-C₆H₄-CH₂- |
| A-95. | CH₃ | 4-(2-Br-C₆H₄-O)-C₆H₄-CH₂- |
| A-96. | CH₃ | 4-(3,4-F₂-C₆H₃-O)-C₆H₄-CH₂- |
| A-97. | CH₃ | 4-(3,4-Cl₂-C₆H₃-O)-C₆H₄-CH₂- |
| A-98. | CH₃ | 4-(3,4-(CH₃)₂-C₆H₃-O)-C₆H₄-CH₂- |
| A-99. | CH₃ | 4-(3-F-4-Cl-C₆H₃-O)-C₆H₄-CH₂- |
| A-100. | CH₃ | 4-(3-Cl-4-F-C₆H₃-O)-C₆H₄-CH₂- |
| A-101. | CH₃ | 4-(3-CH₃-4-Cl-C₆H₃-O)-C₆H₄-CH₂- |
| A-102. | CH₃ | 4-(3-Cl-4-CH₃-C₆H₃-O)-C₆H₄-CH₂- |
| A-103. | CH₃ | 4-(3-Cl-4-Br-C₆H₃-O)-C₆H₄-CH₂- |
| A-104. | CH₃ | 4-(3-CH₃-4-Br-C₆H₃-O)-C₆H₄-CH₂- |
| A-105. | CH₃ | (±) phenyl-CH(CH₃)- |
| A-106. | CH₃ | (R) phenyl-CH(CH₃)- |
| A-107. | CH₃ | (S) phenyl-CH(CH₃)- |
| A-108. | CH₃ | (±) 4-F-phenyl-CH(CH₃)- |
| A-109. | CH₃ | (R) 4-F-phenyl-CH(CH₃)- |
| A-110. | CH₃ | (S) 4-F-phenyl-CH(CH₃)- |
| A-111. | CH₃ | (±) 4-Cl-phenyl-CH(CH₃)- |
| A-112. | CH₃ | (R) 4-Cl-phenyl-CH(CH₃)- |
| A-113. | CH₃ | (S) 4-Cl-phenyl-CH(CH₃)- |
| A-114. | CH₃ | (±) 4-Br-phenyl-CH(CH₃)- |
| A-115. | CH₃ | (R) 4-Br-phenyl-CH(CH₃)- |
| A-116. | CH₃ | (S) 4-Br-phenyl-CH(CH₃)- |
| A-117. | CH₃ | (±) 4-Cl-2-F-phenyl-CH(CH₃)- |
| A-118. | CH₃ | (R) 4-Cl-2-F-phenyl-CH(CH₃)- |
| A-119. | CH₃ | (S) 4-Cl-2-F-phenyl-CH(CH₃)- |
| A-120. | CH₃ | (±) 6-Cl-2-F-phenyl-CH(CH₃)- |
| A-121. | CH₃ | (R) 6-Cl-2-F-phenyl-CH(CH₃)- |
| A-122. | CH₃ | (S) 6-Cl-2-F-phenyl-CH(CH₃)- |
| A-123. | CH₃ | (±) 2-F-phenyl-CH(CH₃)- |
| A-124. | CH₃ | (R) 2-F-phenyl-CH(CH₃)- |
| A-125. | CH₃ | (S) 2-F-phenyl-CH(CH₃)- |
| A-126. | CH₃ | (S) 2,4-F₂-phenyl-CH(CH₃)- |
| A-127. | CH₃ | (±) 2,4-F₂-phenyl-CH(CH₃)- |
| A-128. | CH₃ | (R) 2,4-F₂-phenyl-CH(CH₃)- |
| A-129. | CH₃ | (S) 2,5-F₂-phenyl-CH(CH₃)- |
| A-130. | CH₃ | (±) 2,5-F₂-phenyl-CH(CH₃)- |
| A-131. | CH₃ | (R) 2,5-F₂-phenyl-CH(CH₃)- |
| A-132. | CH₃ | (S) 2,6-F₂-phenyl-CH(CH₃)- |
| A-133. | CH₃ | (±) 2,6-F₂-phenyl-CH(CH₃)- |
| A-134. | CH₃ | (R) 2,6-F₂-phenyl-CH(CH₃)- |
| A-135. | CH₃ | (±) 2-CH₃O-phenyl-CH(CH₃)- |
| A-136. | CH₃ | (R) 2-CH₃O-phenyl-CH(CH₃)- |
| A-137. | CH₃ | (S) 2-CH₃O-phenyl-CH(CH₃)- |
| A-138. | CH₃ | (±) 4-CH₃O-phenyl-CH(CH₃)- |
| A-139. | CH₃ | (R) 4-CH₃O-phenyl-CH(CH₃)- |
| A-140. | CH₃ | (S) 4-CH₃O-phenyl-CH(CH₃)- |
| A-141. | CH₃ | (±) 4-H₅C₂-O-phenyl-CH(CH₃)- |
| A-142. | CH₃ | (R) 4-H₅C₂-O-phenyl-CH(CH₃)- |
| A-143. | CH₃ | (S) 4-H₅C₂-O-phenyl-CH(CH₃)- |
| A-144. | CH₃ | (±) 4-n-propoxy-phenyl-CH(CH₃)- |
| A-145. | CH₃ | (R) 4-n-propoxy-phenyl-CH(CH₃)- |
| A-146. | CH₃ | (S) 4-n-propoxy-phenyl-CH(CH₃)- |
| A-147. | CH₃ | (±) 4-n-butoxy-phenyl-CH(CH₃)- |
| A-148. | CH₃ | (R) 4-n-butoxy-phenyl-CH(CH₃)- |
| A-149. | CH₃ | (S) 4-n-butoxyphenyl-CH(CH₃)- |
| A-150. | CH₃ | (±) 4-tert-butoxy-phenyl-CH(CH₃)- |
| A-151. | CH₃ | (R) 4-tert-butoxy-phenyl-CH(CH₃)- |
| A-152. | CH₃ | (S) 4-tert-butoxyphenyl-CH(CH₃)- |
| A-153. | CH₃ | (±) 4-CH₃-phenyl-CH(CH₃)- |
| A-154. | CH₃ | (R) 4-CH₃-phenyl-CH(CH₃)- |
| A-155. | CH₃ | (S) 4-CH₃-phenyl-CH(CH₃)- |
| A-156. | CH₃ | (±) 4-C₂H₅-phenyl-CH(CH₃)- |
| A-157. | CH₃ | (R) 4-C₂H₅-phenyl-CH(CH₃)- |
| A-158. | CH₃ | (S) 4-C₂H₅-phenyl-CH(CH₃)- |
| A-159. | CH₃ | (±) 4-n-C₃H₇-phenyl-CH(CH₃)- |
| A-160. | CH₃ | (R) 4-n-C₃H₇-phenyl-CH(CH₃)- |
| A-161. | CH₃ | (S) 4-n-C₃H₇-phenyl-CH(CH₃)- |
| A-162. | CH₃ | (±) 4-iso-C₃H₇-phenyl-CH(CH₃)- |
| A-163. | CH₃ | (R) 4-iso-C₃H₇-phenyl-CH(CH₃)- |
| A-164. | CH₃ | (S) 4-iso-C₃H₇-phenyl-CH(CH₃)- |
| A-165. | CH₃ | (±) 4-n-C₄H₉-phenyl-CH(CH₃)- |
| A-166. | CH₃ | (R) 4-n-C₄H₉-phenyl-CH(CH₃)- |
| A-167. | CH₃ | (S) 4-n-C₄H₉-phenyl-CH(CH₃)- |
| A-168. | CH₃ | (±) 4-tert-C₄H₉-phenyl-CH(CH₃)- |
| A-169. | CH₃ | (R) 4-tert-C₄H₉-phenyl-CH(CH3)- |
| A-170. | CH₃ | (S) 4-tert-C₄H₉-phenyl-CH(CH₃)- |
| A-171. | CH₃ | (±) 4-cycl.-C₆H₉phenyl-CH(CH₃)- |
| A-172. | CH₃ | (R) 4-cycl.-C₆H₁₁-phenyl-CH(CH₃)- |
| A-173. | CH₃ | (S) 4-cycl.-C₆H₉phenyl-CH(CH₃)- |
| A-174. | CH₃ | (±) 4-OCF₃-phenyl-CH(CH₃)- |
| A-175. | CH₃ | (R) 4-OCF₃-phenyl-CH(CH₃)- |
| A-176. | CH₃ | (S) 4-OCF₃-phenyl-CH(CH₃)- |
| A-177. | CH₃ | (±) 4-CF₃-phenyl-CH(CH₃)- |
| A-178. | CH₃ | (R) 4-CF₃-phenyl-CH(CH₃)- |
| A-179. | CH₃ | (S) 4-CF₃-phenyl-CH(CH3)- |
| A-180. | CH₃ | (±) 3-F-phenyl-CH(CH₃)- |
| A-181. | CH₃ | (R) 3-F-phenyl-CH(CH₃)- |
| A-182. | CH₃ | (S) 3-F-phenyl-CH(CH₃)- |
| A-183. | CH₃ | (±) 3-Cl-phenyl-CH(CH₃)- |
| A-184. | CH₃ | (R) 3-Cl-phenyl-CH(CH₃)- |
| A-185. | CH₃ | (S) 3-Cl-phenyl-CH(CH₃)- |
| A-186. | CH₃ | (±) 3-Br-phenyl-CH(CH₃)- |
| A-187. | CH₃ | (R) 3-Br-phenyl-CH(CH₃)- |
| A-188. | CH₃ | (S) 3-Br-phenyl-CH(CH₃)- |
| A-189. | CH₃ | (±) 3-CF₃-phenyl-CH(CH₃)- |
| A-190. | CH₃ | (R) 3-CF₃-phenyl-CH(CH₃)- |
| A-191. | CH₃ | (S) 3-CF₃-phenyl-CH(CH₃)- |
| A-192. | CH₃ | (±) 3,4-F₂-phenyl-CH(CH₃)- |
| A-193. | CH₃ | (R) 3,4-F₂-phenyl-CH(CH₃)- |
| A-194. | CH₃ | (S) 3,4-F₂-phenyl-CH(CH₃)- |
| A-195. | CH₃ | (±) 3,4-Cl₂-phenyl-CH(CH₃)- |
| A-196. | CH₃ | (R) 3,4-Cl₂-phenyl-CH(CH₃)- |
| A-197. | CH₃ | (S) 3,4-Cl₂-phenyl-CH(CH₃)- |
| A-198. | CH₃ | (±) 3,4-Br₂-phenyl-CH(CH₃)- |
| A-199. | CH₃ | (R) 3,4-Br₂-phenyl-CH(CH₃)- |
| A-200. | CH₃ | (S) 3,4-Br₂-phenyl-CH(CH₃)- |
| A-201. | CH₃ | (±) 4-Difluoromethoxyphenyl-CH(CH₃)- |
| A-202. | CH₃ | (R) 4-Difluoromethoxyphenyl-CH(CH₃)- |
| A-203. | CH₃ | (S) 4-Difluoromethoxyphenyl-CH(CH₃)- |
| A-204. | CH₃ | (±) 4-(1,1,2,2-tetrafluoroethoxy)phenyl-CH(CH₃)- |
| A-205. | CH₃ | (R) 4-(1,1,2,2-tetrafluoroethoxy)phenyl-CH(CH₃)- |
| A-206. | CH₃ | (S) 4-(1,1,2,2-tetrafluoroethoxy)phenyl-CH(CH₃)- |
| A-207. | CH₃ | (±) (5,5,7,7-tetramethylindan-2-yl)-CH(CH₃)- |
| A-208. | CH₃ | (R) (5,5,7,7-tetramethylindan-2-yl)-CH(CH₃)- |
| A-209. | CH₃ | (S) (5,5,7,7-tetramethylindan-2-yl)-CH(CH₃)- |
| A-210. | CH₃ | (±) (1,1,4,4,-tetramethyl-1,2,3,4-tetrahydronaphthalin-6-yl)-CH(CHs)- |
| A-211. | CH₃ | (R) (1,1,4,4,-tetramethyl-1,2,3,4-tetrahydronaphthalin-6-yl)-CH(CH₃)- |
| A-212. | CH₃ | (S) (1,1,4,4,-tetramethyl-1,2,3,4-tetrahydronaphthalin-6-yl)-CH(CH₃)- |
| A-213. | CH₃ | (±) (1,1-dimethyl-1,2,3,4-tetrahydronaphthalin-6-yl)-CH(CH₃)- |
| A-214. | CH₃ | (R) (1,1-dimethyl-1,2,3,4-tetrahydronaphthalin-6-yl)-CH(CH₃)- |
| A-215. | CH₃ | (S) (1,1-dimethyl-1,2,3,4-tetrahydronaphthalin-6-yl)-CH(CH₃)- |
| A-216. | CH₃ | (±) (1,1,4,4,7-pentamethyl-1,2,3,4-tetrahydronaphthalin-6-yl)-CH(CH₃)- |
| A-217. | CH₃ | (R) (1,1,4,4,7-pentamethyl-1,2,3,4-tetrahydronaphthalin-6-yl)-CH(CH₃)- |
| A-218. | CH₃ | (S) (1,1,4,4,7-pentamethyl-1,2,3,4-tetrahydronaphthalin-6-yl)-CH(CH₃)- |
| A-219. | CH₃ | (±) (1,1,7-trimethyl-1,2,3,4-tetrahydronaphthalin-6-yl)-CH(CH₃)- |
| A-220. | CH₃ | (R) (1,1,7-trimethyl-1,2,3,4-tetrahydronaphthalin-6-yl)-CH(CH₃)- |
| A-221. | CH₃ | (S) (1,1,7-trimethyl-1,2,3,4-tetrahydronaphthalin-6-yl)-CH(CH₃)- |
| A-222. | CH₃ | (±) (2-methy)-1,3-dioxan-2-yl)-CH(CH₃)- |
| A-223. | CH₃ | (R) 2-methyl-1,3-dioxan-2-yl)-CH(CH₃)- |
| A-224. | CH₃ | (S) 2-methyl-1,3-dioxan-2-yl)-CH(CH₃)- |
| A-225. | CH₃ | (±) (2,5,5-trimethyl-1,3-dioxan-2-yl)-CH(CH₃)- |
| A-226. | CH₃ | (R) 2,5,5-trimethyl-1,3-dioxan-2-yl)-CH(CH₃) |
| A-227. | CH₃ | (S) 2,5,5-trimethyl-1,3-dioxan-2-yl)-CH(CH₃)- |
| A-228. | CH₃ | (±) (2,2-difluorobenzodioxole-5-yl)CH(C₂H₅)- |
| A-229. | CH₃ | (R) (2,2-difluorobenzodioxole-5-yl)CH(C₂H₅)- |
| A-230. | CH₃ | (S) (2,2-difluorobenzodioxole-5-yl)CH(C₂H₅)- |
| A-231. | CH₃ | C₆H₅CH₂CH₂- |
| A-232. | CH₃ | 4-F-C₆H₄CH₂CH₂- |
| A-233. | CH₃ | 4-Cl-C₆H₄CH₂CH₂- |
| A-234. | CH₃ | 4-Br-C₆H₄CH₂CH₂- |
| A-235. | CH₃ | 4-CH₃O-C₆H₄CH₂CH₂- |
| A-236. | CH₃ | 4-C₂H₅O-C₆H₄CH₂CH₂- |
| A-237. | CH₃ | 4-n-C₃H₇O-C₆H₄CH₂CH₂- |
| A-238. | CH₃ | 4-n-C₄H₉O-C₆H₄CH₂CH₂- |
| A-239. | CH₃ | 4-t-C₄H₉O-C₆H₄CH₂CH₂- |
| A-240. | CH₃ | 3,4-(CH₃O)₂-C₆H₄CH₂CH₂- |
| A-241. | CH₃ | 4-H₃C-C₆H₄CH₂CH₂- |
| A-242. | CH₃ | 4-H₃C-H₂C-C₆H₄CH₂CH₂- |
| A-243. | CH₃ | 4-H₃C-H₂C-H₂C-C₆H₄CH₂CH₂- |
| A-244. | CH₃ | 4-H₃C-H₂C-H₂C-H₂C-C₆H₄CH₂CH₂- |
| A-245. | CH₃ | 4-(CH₃)₃C-C₆H₄CH₂CH₂- |
| A-246. | CH₃ | 4-F₃CO-C₆H₄CH₂CH₂- |
| A-247. | CH₃ | 4-F₃C-C₆H₄CH₂CH₂- |
| A-248. | CH₃ | 3-F₃C-C₆H₄CH₂CH₂- |
| A-249. | CH₃CH₂ | 4-CH₃-cyclohexyl- |
| A-250. | CH₃CH₂ | cis-4-CH3-cyclohexyl- |
| A-251. | CH₃CH₂ | trans-4-CH₃-cyclohexyl- |
| A-252. | CH₃CH₂ | 4-C₂H₅-cyclohexyl- |
| A-253. | CH₃CH₂ | cis-4-C₂H₅-cyclohexyl- |
| A-254. | CH₃CH₂ | trans-4-C₂H₅-cyclohexyl- |
| A-255. | CH₃CH₂ | 4-n-propyl-cyclohexyl- |
| A-256. | CH₃CH₂ | cis-4-n-propyl-cyclohexyl- |
| A-257. | CH₃CH₂ | trans-4-n-propyl-cyclohexyl- |
| A-258. | CH₃CH₂ | 4-isopropyl-cyclohexyl- |
| A-259. | CH₃CH₂ | cis-4-isopropyl-cyclohexyl- |
| A-260. | CH₃CH₂ | trans-4-isopropyl-cyclohexyl- |
| A-261. | CH₃CH₂ | 4-n-butylcyclohexyl- |
| A-262. | CH₃CH₂ | cis-4-n-butylcyclohexyl- |
| A-263. | CH₃CH₂ | trans-4-n-butylcyclohexyl- |
| A-264. | CH₃CH₂ | 4-(2-butyl)cyclohexyl- |
| A-265. | CH₃CH₂ | cis-4-(2-butyl)cyclohexyl- |
| A-266. | CH₃CH₂ | trans-4-(2-butyl)cyclohexyl- |
| A-267. | CH₃CH₂ | 4-tert-butylcyclohexyl- |
| A-268. | CH₃CH₂ | cis-4-tert-butylcyclohexyl- |
| A-269. | CH₃CH₂ | trans-4-tert-butylcyclohexyl- |
| A-270. | CH₃CH₂ | 4-(cyclohexyl-C(CH₃)₂)-cyclohexyl- |
| A-271. | CH₃CH₂ | cis-4-(cyclohexyl-C(CH₃)2)-cyclohexyl- |
| A-272. | CH₃CH₂ | trans-4-(cyclohexyl-C(CH₃)₂)-cyclohexyl- |
| A-273. | CH₃CH₂ | C₆H₅- |
| A-274. | CH₃CH₂ | 4-F-C₆H₄- |
| A-275. | CH₃CH₂ | 4-Cl-C₆H₄- |
| A-276. | CH₃CH₂ | 4-Br-C₆H₄- |
| A-277. | CH₃CH₂ | 4-(C₆H₅)-C₆H₄- |
| A-278. | CH₃CH₂ | 4-phenoxyphenyl |
| A-279. | CH₃CH₂ | benzyl |
| A-280. | CH₃CH₂ | 4-CH₃-C₆H₄-CH₂- |
| A-281. | CH₃CH₂ | 4-C₂H₅-C₆H₄-CH₂- |
| A-282. | CH₃CH₂ | 4-n-C₃H₇-C₆H₄-CH₂- |
| A-283. | CH₃CH₂ | 4-isopropyl-C₆H₄-CH₂- |
| A-284. | CH₃CH₂ | 4-n-C₄H₉-C₆H₄-CH₂- |
| A-285. | CH₃CH₂ | 4-isobutyl-C₆H₄-CH₂- |
| A-286. | CH₃CH₂ | 4-tert-buyl-C₆H₄CH₂- |
| A-287. | CH₃CH₂ | 4-F₃C-C₆H₄CH₂- |
| A-288. | CH₃CH₂ | 4-methoxy-C₆H₄CH₂- |
| A-289. | CH₃CH₂ | 4-ethoxy-C₆H₄CH₂- |
| A-290. | CH₃CH₂ | 4-n-propoxy-C₆H₄CH₂- |
| A-291. | CH₃CH₂ | 4-isopropoxy-C₆H₄CH₂- |
| A-292. | CH₃CH₂ | 4-n-butoxy-C₆H₄CH₂- |
| A-293. | CH₃CH₂ | 4-tert-butoxy-C₆H₄CH₂- |
| A-294. | CH₃CH₂ | 4-F-C₆H₄CH₂- |
| A-295. | CH₃CH₂ | 4-Cl-C₆H₄CH₂- |
| A-296. | CH₃CH₂ | 4-Br-C₆H₄CH₂- |
| A-297. | CH₃CH₂ | 3,4-F₂-C₆H₃CH₂- |
| A-298. | CH₃CH₂ | 3,4-Cl₂-C₆H₃CH₂- |
| A-299. | CH₃CH₂ | 3,4-Br₂-C₆H₃CH₂- |
| A-300. | CH₃CH₂ | 4-(4-CH₃-C₆H₄-O)-C₆H₄-CH₂- |
| A-301. | CH₃CH₂ | 4-(4-C₂H₅-C₆H₄-O)-C₆H₄-CH₂- |
| A-302. | CH₃CH₂ | 4-(4-n-C₃H₇-C₆H₄-O)-C₆H₄-CH₂- |
| A-303. | CH₃CH₂ | 4-(4-iso-C₃H₇-C₆H₄-O)-C₆H₄-CH₂- |
| A-304. | CH₃CH₂ | 4-(4-n-C₄H₉-C₆H₄-O)-C₆H₄-CH₂- |
| A-305. | CH₃CH₂ | 4-(4-iso-C₄H₉-C₆H₄-O)-C₆H₄-CH₂- |
| A-306. | CH₃CH₂ | 4-(4-tert-C₄H₉-C₆H4-O)-C₆H₄-CH₂- |
| A-307. | CH₃CH₂ | 4-(4-H₃C-O-C₆H₄-O)-C₆H₄-CH₂- |
| A-308. | CH₃CH₂ | 4-(4-H₃C-H₂C-O-C₆H₄-O)-C₆H₄-CH₂- |
| A-309. | CH₃CH₂ | 4-(4-H₃C-H₂C-H₂C-O-C₆H₄-O)-C₆H₄-CH₂- |
| A-310. | CH₃CH₂ | 4-(4-H₃C-H₂C-H₂C-H₂C-O-C₆H₄-O)-C₆H₄-CH₂- |
| A-311. | CH₃CH₂ | 4-(4-tert-butoxy-C₆H₄-O)-C₆H₄-CH₂- |
| A-312. | CH₃CH₂ | 4-(4-Cl-C₆H₄-O)-C₆H₄-CH₂- |
| A-313. | CH₃CH₂ | 4-(4-Br-C₆H₄-O)-C₆H₄-CH₂- |
| A-314. | CH₃CH₂ | 4-(3-CH₃-C₆H₄-O)-C₆H₄-CH₂- |
| A-315. | CH₃CH₂ | 4-(3-C₂H₅-C₆H₄-O)-C₆H₄-CH₂- |
| A-316. | CH₃CH₂ | 4-(3-n-C₃H₇-C₆H₄-O)-C₆H₄-CH₂- |
| A-317. | CH₃CH₂ | 4-(3-iso-C₃H₇-C₆H₄-O)-C₆H₄-CH₂- |
| A-318. | CH₃CH₂ | 4-(3-n-C₄H₉-C₆H₄-O)-C₆H₄-CH₂- |
| A-319. | CH₃CH₂ | 4-(3-iso-C₄H₉-C₆H4-O)-C₆H₄-CH₂- |
| A-320. | CH₃CH₂ | 4-(3-tert-butyl-C₆H₄-O)-C₆H₄-CH₂- |
| A-321. | CH₃CH₂ | 4-(3-H₃C-O-C₆H₄-O)-C₆H₄-CH₂- |
| A-322. | CH₃CH₂ | 4-(3-H₃C-H₂C-O-C₆H₄-O)-C₆H₄-CH2- |
| A-323. | CH₃CH₂ | 4-(3-H₃C-H₂C-H₂C-O-C₆H₄-O)-C₆H₄-CH₂- |
| A-324. | CH₃CH₂ | 4-(3-H₃C-H₂C-H₂C-H₂C-O-C₆H₄-O)-C₆H₄-CH₂- |
| A-325. | CH₃CH₂ | 4-(3-tert-butoxy-C₆H₄-O)-C₆H₄-CH2- |
| A-326. | CH₃CH₂ | 4-(3-F-C₆H₄-O)-C₆H₄-CH₂- |
| A-327. | CH₃CH₂ | 4-(3-Cl-C₆H₄-O)-C₆H₄-CH₂- |
| A-328. | CH₃CH₂ | 4-(3-Br-C₆H₄-O)-C₆H₄-CH₂- |
| A-329. | CH₃CH₂ | 4-(2-CH₃-C₆H₄-O)-C₆H₄-CH₂- |
| A-330. | CH₃CH₂ | 4-(2-C₂H₅-C₆H₄-O)-C₆H₄-CH₂- |
| A-331. | CH₃CH₂ | 4-(2-n-C₃H₇-C₆H₄-O)-C₆H₄-CH₂- |
| A-332. | CH₃CH₂ | 4-(2-iso-C₃H₇-C₆H₄-O)-C₆H₄-CH₂- |
| A-333. | CH₃CH₂ | 4-(2-n-C₄H₉-C₆H₄-O)-C₆H₄-CH₂- |
| A-334. | CH₃CH₂ | 4-(2-iso-C₄H₉-C₆H₄-O)-C₆H₄-CH₂- |
| A-335. | CH₃CH₂ | 4-(2-tert-butyl-C₆H₄-O)-C₆H₄-CH₂- |
| A-336. | CH₃CH₂ | 4-(2-H₃C-O-C₆H₄-O)-C₆H₄-CH₂- |
| A-337. | CH₃CH₂ | 4-(2-H₃C-H₂C-O-C₆H₄-O)-C₆H₄-CH₂- |
| A-338. | CH₃CH₂ | 4-(2-H₃C-H₂C-H₂C-O-C₆H₄-O)-C₆H₄-CH₂- |
| A-339. | CH₃CH₂ | 4-(2-H₃C-H₂C-H₂C-H₂C-O-C₆H₄-O)-C₆H₄-CH₂- |
| A-340. | CH₃CH₂ | 4-(2-tert-butoxy-C₆H₄-O)-C₆H₄-CH2- |
| A-341. | CH₃CH₂ | 4-(2-F-C₆H₄-O)-C₆H₄-CH₂- |
| A-342. | CH₃CH₂ | 4-(2-Cl-C₆H₄-O)-C₆H₄-CH₂- |
| A-343. | CH₃CH₂ | 4-(2-Br-C₆H₄-O)-C₆H₄-CH₂- |
| A-344. | CH₃CH₂ | 4-(3,4-F₂-C₆H₃-O)-C₆H₄-CH₂- |
| A-345. | CH₃CH₂ | 4-(3,4-Cl₂-C₆H₃-O)-C₆H₄-CH₂- |
| A-346. | CH₃CH₂ | 4-(3,4-(CH₃)₂-C₆H₃-O)-C₆H₄-CH₂- |
| A-347. | CH₃CH₂ | 4-(3-F-4-Cl-C₆H₃-O)-C₆H₄-CH₂- |
| A-348. | CH₃CH₂ | 4-(3-Cl-4-F-C₆H₃-O)-C₆H₄-CH₂- |
| A-349. | CH₃CH₂ | 4-(3-CH₃-4-Cl-C₆H₃-O)-C₆H₄-CH₂- |
| A-350. | CH₃CH₂ | 4-(3-Cl-4-CH₃-C₆H₃-O)-C₆H₄-CH₂- |
| A-351. | CH₃CH₂ | 4-(3-Cl-4-Br-C₆H₃-O)-C₆H₄-CH₂- |
| A-352. | CH₃CH₂ | 4-(3-CH₃-4-Br-C₆H₃-O)-O₆H₄-CH₂- |
| A-353. | CH₃CH₂ | (±) phenyl-CH(CH₃)- |
| A-354. | CH₃CH₂ | (R) phenyl-CH(CH₃)- |
| A-355. | CH₃CH₂ | (S) phenyl-CH(CH₃)- |
| A-356. | CH₃CH₂ | (±) 4-F-phenyl-CH(CH₃)- |
| A-357. | CH₃CH₂ | (R) 4-F-phenyl-CH(CH₃)- |
| A-358. | CH₃CH₂ | (S) 4-F-phenyl-CH(CH₃)- |
| A-359. | CH₃CH₂ | (±) 4-Cl-phenyl-CH(CH₃)- |
| A-360. | CH₃CH₂ | (R) 4-Cl-phenyl-CH(CH₃)- |
| A-361. | CH₃CH₂ | (S) 4-Cl-phenyl-CH(CH₃)- |
| A-362. | CH₃CH₂ | (±) 4-Br-phenyl-CH(CH₃)- |
| A-363. | CH₃CH₂ | (R) 4-Br-phenyl-CH(CH₃)- |
| A-364. | CH₃CH₂ | (S) 4-Br-phenyl-CH(CH₃)- |
| A-365. | CH₃CH₂ | (±) 4-Cl-2-F-phenyl-CH(CH₃)- |
| A-366. | CH₃CH₂ | (R) 4-Cl-2-F-phenyl-CH(CH₃)- |
| A-367. | CH₃CH₂ | (S) 4-Cl-2-F-phenyl-CH(CH₃)- |
| A-368. | CH₃CH₂ | (±) 6-Cl-2-F-phenyl-CH(CH₃)- |
| A-369. | CH₃CH₂ | (R) 6-Cl-2-F-phenyl-CH(CH₃)- |
| A-370. | CH₃CH₂ | (S) 6-Cl-2-F-phenyl-CH(CH₃)- |
| A-371. | CH₃CH₂ | (±) 2-F-phenyl-CH(CH₃)- |
| A-372. | CH₃CH₂ | (R) 2-F-phenyl-CH(CH₃)- |
| A-373. | CH₃CH₂ | (S) 2-F-phenyl-CH(CH₃)- |
| A-374. | CH₃CH₂ | (S) 2,4-F₂-phenyl-CH(CH₃)- |
| A-375. | CH₃CH₂ | (±) 2,4-F₂-phenyl-CH(CH₃)- |
| A-376. | CH₃CH₂ | (R) 2,4-F₂-phenyl-CH(CH₃)- |
| A-377. | CH₃CH₂ | (S) 2,5-F₂-phenyl-CH(CH₃)- |
| A-378. | CH₃CH₂ | (±) 2,5-F₂-phenyl-CH(CH₃)- |
| A-379. | CH₃CH₂ | (R) 2,5-F₂-phenyl-CH(CH3)- |
| A-380. | CH₃CH₂ | (S) 2,6-F₂-phenyl-CH(CH₃)- |
| A-381. | CH₃CH₂ | (±) 2,6-F₂-phenyl-CH(CH₃)- |
| A-382. | CH₃CH₂ | (R) 2,6-F₂-phenyl-CH(CH₃)- |
| A-383. | CH₃CH₂ | (±) 2-CH₃O-phenyl-CH(CH3)- |
| A-384. | CH₃CH₂ | (R) 2-CH₃O-phenyl-CH(CH₃)- |
| A-385. | CH₃CH₂ | (S) 2-CH₃O-phenyl-CH(CH₃)- |
| A-386. | CH₃CH₂ | (±) 4-CH₃O-phenyl-CH(CH₃)- |
| A-387. | CH₃CH₂ | (R) 4-CH₃O-phenyl-CH(CH₃)- |
| A-388. | CH₃CH₂ | (S) 4-CH₃O-phenyl-CH(CH₃)- |
| A-389. | CH₃CH₂ | (±) 4-H₅C₂-O-phenyl-CH(CH₃)- |
| A-390. | CH₃CH₂ | (R) 4-H₅C₂-O-phenyl-CH(CH₃)- |
| A-391. | CH₃CH₂ | (S) 4-H₅C₂-O-phenyl-CH(CH₃)- |
| A-392. | CH₃CH₂ | (±) 4-n-propoxy-phenyl-CH(CH₃)- |
| A-393. | CH₃CH₂ | (R) 4-n-propoxy-phenyl-CH(CH₃)- |
| A-394. | CH₃CH₂ | (S) 4-n-propoxy-phenyl-CH(CH₃)- |
| A-395. | CH₃CH₂ | (±) 4-n-butoxy-phenyl-CH(CH₃)- |
| A-396. | CH₃CH₂ | (R) 4-n-butoxyx-phenyl-CH(CH₃)- |
| A-397. | CH₃CH₂ | (S) 4-n-butoxyphenyl-CH(CH₃)- |
| A-398. | CH₃CH₂ | (±) 4-tert-butoxy-phenyl-CH(CH₃)- |
| A-399. | CH₃CH₂ | (R) 4-tert-butoxyx-phenyl-CH(CH₃)- |
| A-400. | CH₃CH₂ | (S) 4-tert-butoxyphenyl-CH(CH₃)- |
| A-401. | CH₃CH₂ | (±) 4-CH₃-phenyl-CH(CH₃)- |
| A-402. | CH₃CH₂ | (R) 4-CH₃-phenyl-CH(CH₃)- |
| A-403. | CH₃CH₂ | (S) 4-CH₃-phenyl-CH(CH₃)- |
| A-404. | CH₃CH₂ | (±) 4-C₂H₅-phenyl-CH(CH3)- |
| A-405. | CH₃CH₂ | (R) 4-C₂H₅-phenyl-CH(CH₃)- |
| A-406. | CH₃CH₂ | (S) 4-C₂Hs-phenyl-CH(CH₃)- |
| A-407. | CH₃CH₂ | (±) 4-n-C₃H₇-phenyl-CH(CH3)- |
| A-408. | CH₃CH₂ | (R) 4-n-C₃H₇-phenyl-CH(CH₃)- |
| A-409. | CH₃CH₂ | (S) 4-n-C₃H₇-phenyl-CH(CH₃)- |
| A-410. | CH₃CH₂ | (±) 4-iso-C₃H₇-phenyl-CH(CH₃)- |
| A-411. | CH₃CH₂ | (R) 4-iso-C₃H₇-phenyl-CH(CH₃)- |
| A-412. | CH₃CH₂ | (S) 4-iso-C₃H₇-phenyl-CH(CH₃)- |
| A-413. | CH₃CH₂ | (±) 4-n-C₄H₉-phenyl-CH(CH₃)- |
| A-414. | CH₃CH₂ | (R) 4-n-C₄H₉-phenyl-CH(CH₃)- |
| A-415. | CH₃CH₂ | (S) 4-n-C₄H₉-phenyl-CH(CH₃)- |
| A-416. | CH₃CH₂ | (±) 4-tert-C₄H₉-phenyl-CH(CH₃)- |
| A-417. | CH₃CH₂ | (R) 4-tert-C₄H₉-phenyl-CH(CH₃)- |
| A-418. | CH₃CH₂ | (S) 4-tert-C₄H₉-phenyl-CH(CH₃)- |
| A-419. | CH₃CH₂ | (±) 4-cycl.-C₆H₁₁-phenyl-CH(CH₃)- |
| A-420. | CH₃CH₂ | (R) 4-cycl.-C₆H₁₁-phenyl-CH(CH₃)- |
| A-421. | CH₃CH₂ | (S) 4-cycl.-C₆H₁₁-phenyl-CH(CH₃)- |
| A-422. | CH₃CH₂ | (±) 4-OCF₃-phenyl-CH(CH₃)- |
| A-423. | CH₃CH₂ | (R) 4-OCF₃-phenyl-CH(CH₃)- |
| A-424. | CH₃CH₂ | (S) 4-OCF₃-phenyl-CH(CH₃)- |
| A-425. | CH₃CH₂ | (±) 4-CF₃-phenyl-CH(CH₃)- |
| A-426. | CH₃CH₂ | (R) 4-CF₃-phenyl-CH(CH₃)- |
| A-427. | CH₃CH₂ | (S) 4-CF₃-phenyl-CH(CH₃)- |
| A-428. | CH₃CH₂ | (±) 3-F-phenyl-CH(CH₃)- |
| A-429. | CH₃CH₂ | (R) 3-F-phenyl-CH(CH₃)- |
| A-430. | CH₃CH₂ | (S) 3-F-phenyl-CH(CH₃)- |
| A-431. | CH₃CH₂ | (±) 3-Cl-phenyl-CH(CH₃)- |
| A-432. | CH₃CH₂ | (R) 3-Cl-phenyl-CH(CH₃)- |
| A-433. | CH₃CH₂ | (S) 3-Cl-phenyl-CH(CH₃)- |
| A-434. | CH₃CH₂ | (±) 3-Br-phenyl-CH(CH₃)- |
| A-435. | CH₃CH₂ | (R) 3-Br-phenyl-CH(CH₃)- |
| A-436. | CH₃CH₂ | (S) 3-Br-phenyl-CH(CH₃)- |
| A-437. | CH₃CH₂ | (±) 3-CF₃-phenyl-CH(CH₃)- |
| A-438. | CH₃CH₂ | (R) 3-CF₃-phenyl-CH(CH₃)- |
| A-439. | CH₃CH₂ | (S) 3-CF₃-phenyl-CH(CH₃)- |
| A-440. | CH₃CH₂ | (±) 3,4-F₂-phenyl-CH(CH₃)- |
| A-441. | CH₃CH₂ | (R) 3,4-F₂-phenyl-CH(CH₃)- |
| A-442. | CH₃CH₂ | (S) 3,4-F₂-phenyl-CH(CH3)- |
| A-443. | CH₃CH₂ | (±) 3,4-Cl₂-phenyl-CH(CH₃)- |
| A-444. | CH₃CH₂ | (R) 3,4-Cl₂-phenyl-CH(CH₃)- |
| A-445. | CH₃CH₂ | (S) 3,4-Cl₂-phenyl-CH(CH₃)- |
| A-446. | CH₃CH₂ | (±) 3,4-Br₂-phenyl-CH(CH₃)- |
| A-447. | CH₃CH₂ | (R) 3,4-Br₂-phenyl-CH(CH₃)- |
| A-448. | CH₃CH₂ | (S) 3,4-Br₂-phenyl-CH(CH₃)- |
| A-449. | CH₃CH₂ | (±) 4-Difluoromethoxyphenyl-CH(CH₃)- |
| A-450. | CH₃CH₂ | (R) 4-Difluoromethoxyphenyl-CH(CH₃)- |
| A-451. | CH₃CH₂ | (S) 4-Difluoromethoxyphenyl-CH(CH₃)- |
| A-452. | CH₃CH₂ | (±) 4-(1,1,2,2-tetrafluoroethoxy)phenyl-CH(CH₃)- |
| A-453. | CH₃CH₂ | (R) 4-(1,1,2,2-tetrafluoroethoxy)phenyl-CH(CH₃)- |
| A-454. | CH₃CH₂ | (S) 4-(1,1,2,2-tetrafluoroethoxy)phenyl-CH(CH₃)- |
| A-455. | CH₃CH₂ | (±) (5,5,7,7-tetramethylindan-2-yl)-CH(CH₃)- |
| A-456. | CH₃CH₂ | (R) (5,5,7,7-tetramethylindan-2-yl)-CH(CH₃)- |
| A-457. | CH₃CH₂ | (S) (5,5,7,7-tetramethylindan-2-yl)-CH(CH₃)- |
| A-458. | CH₃CH₂ | (±) (1,1,4,4,-tetramethyl-1,2,3,4-tetrahydronaphthalin-6-yl)-CH(CH₃)- |
| A-459. | CH₃CH₂ | (R) (1,1,4,4,-tetramethyl-1,2,3,4-tetrahydronaphthalin-6-yl)-CH(CH₃)- |
| A-460. | CH₃CH₂ | (S) (1,1,4,4,-tetramethyl-1,2,3,4-tetrahydronaphthalin-6-yl)-CH(CH₃)- |
| A-461. | CH₃CH₂ | (±) (1,1-dimethyl-1,2,3,4-tetrahydronaphthalin-6-yl)-CH(CH₃)- |
| A-462. | CH₃CH₂ | (R) (1,1-dimethyl-1,2,3,4-tetrahydronaphthalin-6-yl)-CH(CH₃)- |
| A-463. | CH₃CH₂ | (S) (1,1-dimethyl-1,2,3,4-tetrahydronaphthalin-6-yl)-CH(CH₃)- |
| A-464. | CH₃CH₂ | (±) (1,1,4,4,7-pentamethyl-1,2,3,4-tetrahydronaphthalin-6-yl)-CH(CH₃)- |
| A-465. | CH₃CH₂ | (R) (1,1,4,4,7-pentamethyl-1,2,3,4-tetrahydronaphthalin-6-yl)-CH(CH₃)- |
| A-466. | CH₃CH₂ | (S) (1,1,4,4,7-pentamethyl-1,2,3,4-tetrahydronaphthalin-6-yl)-CH(CH₃)- |
| A-467. | CH₃CH₂ | (±) (1,1,7-trimethyl-1,2,3,4-tetrahydronaphthalin-6-yl)-CH(CH₃)- |
| A-468. | CH₃CH₂ | (R) (1,1,7-trimethyl-1,2,3,4-tetrahydronaphthalin-6-yl)-CH(CH₃)- |
| A-469. | CH₃CH₂ | (S) (1,1,7-trimethyl-1,2,3,4-tetrahydronaphthalin-6-yl)-CH(CH₃)- |
| A-470. | CH₃CH₂ | (±) (2-methyl-1,3-dioxan-2-yl)-CH(CH₃)- |
| A-471. | CH₃CH₂ | (R) 2-methyl-1,3-dioxan-2-yl)-CH(CH₃)- |
| A-472. | CH₃CH₂ | (S) 2-methyl-1,3-dioxan-2-yl)-CH(CH₃)- |
| A-473. | CH₃CH₂ | (±) (2,5,5-trimethyl-1,3-dioxan-2-yl)-CH(CH₃)- |
| A-474. | CH₃CH₂ | (R) 2,5,5-trimethyl-1,3-dioxan-2-yl)-CH(CH₃)- |
| A-475. | CH₃CH₂ | (S) 2,5,5-trimethyl-1,3-dioxan-2-yl)-CH(CH₃)- |
| A-476. | CH₃CH₂ | (±) (2,2-difluorobenzodioxole-5-yl)CH(C₂H₅)- |
| A-477. | CH₃CH₂ | (R) (2,2-difluorobenzodioxole-5-yl)CH(C₂H₅)- |
| A-478. | CH₃CH₂ | (S) (2,2-difluorobenzodioxole-5-yl)CH(C₂H₅)- |
| A-479. | CH₃CH₂ | C₆H₅CH₂CH₂- |
| A-480. | CH₃CH₂ | 4-F-C₆H₄CH₂CH₂- |
| A-481. | CH₃CH₂ | 4-Cl-C₆H₄CH₂CH₂- |
| A-482. | CH₃CH₂ | 4-Br-C₆H₄CH₂CH₂- |
| A-483. | CH₃CH₂ | 4-CH₃O-C₆H₄CH₂CH₂- |
| A-484. | CH₃CH₂ | 4-C₂H₅O-C₆H₄CH₂CH₂- |
| A-485. | CH₃CH₂ | 4-n-C₃H₇O-C₆H₄CH₂CH₂- |
| A-486. | CH₃CH₂ | 4-n-C₄H₉O-C₆H₄CH₂CH₂- |
| A-487. | CH₃CH₂ | 4-t-C₄H₉O-C₆H₄CH₂CH₂- |
| A-488. | CH₃CH₂ | 3,4-(CH₃O)₂-C₆H₄CH₂CH₂- |
| A-489. | CH₃CH₂ | 4-H₃C-C₆H₄CH₂CH₂- |
| A-490. | CH₃CH₂ | 4-H₃C-H₂C-C₆H₄CH₂CH₂- |
| A-491. | CH₃CH₂ | 4-H₃C-H₂C-H₂C-C₆H₄CH₂CH₂- |
| A-492. | CH₃CH₂ | 4-H₃C-H₂C-H₂C-H₂C-C₆H₄CH₂CH₂- |
| A-493. | CH₃CH₂ | 4-(CH₃)₃C-C₆H₄CH₂CH₂- |
| A-494. | CH₃CH₂ | 4-F₃CO-C₆H₄CH₂CH₂- |
| A-495. | CH₃CH₂ | 4-F₃C-CeH₄CH₂CH₂- |
| A-496. | CH₃CH₂ | 3-F₃C-C₆H₄CH₂CH₂- |
| A-497. | CH₃CHF | 4-CH₃-cyclohexyl- |
| A-498. | CH₃CHF | cis-4-CH₃-cyclohexyl- |
| A-499. | CH₃CHF | trans-4-CH₃-cyclohexyl- |
| A-500. | CH₃CHF | 4-C₂H₅-cyclohexyl- |
| A-501. | CH₃CHF | cis-4-C₂H₅-cyclohexyl- |
| A-502. | CH₃CHF | trans-4-C₂H₅-cyclohexyl- |
| A-503. | CH₃CHF | 4-n-propyl-cyclohexyl- |
| A-504. | CH₃CHF | cis-4-n-propyl-cyclohexyl- |
| A-505. | CH₃CHF | trans-4-n-propyl-cyclohexyl- |
| A-506. | CH₃CHF | 4-isopropyl-cyclohexyl- |
| A-507. | CH₃CHF | cis-4-isopropyl-cyclohexyl- |
| A-508. | CH₃CHF | trans-4-isopropyl-cyclohexyl- |
| A-509. | CH₃CHF | 4-n-butylcyclohexyl- |
| A-510. | CH₃CHF | cis-4-n-butylcyclohexyl- |
| A-511. | CH₃CHF | trans-4-n-butylcyclohexyl- |
| A-512. | CH₃CHF | 4-tert-butylcyclohexyl- |
| A-513. | CH₃CHF | cis-4-tert-butylcyclohexyl- |
| A-514. | CH₃CHF | trans-4-tert-butylcyclohexyl- |
| A-515. | CH₃CHF | 4-(2-butyl)cyclohexyl- |
| A-516. | CH₃CHF | cis-4-(2-butyl)cyclohexyl- |
| A-517. | CH₃CHF | trans-4-(2-butyl)cyclohexyl- |
| A-518. | CH₃CHF | 4-(cyclohexyl-C(CH₃)₂)-cyclohexyl- |
| A-519. | CH₃CHF | cis-4-(cyclohexyl-C(CH₃)₂)-cyclohexyl- |
| A-520. | CH₃CHF | trans-4-(cyclohexyl-C(CH₃)₂)-cyclohexyl- |
| A-521. | CH₃CHF | C₆H₅- |
| A-522. | CH₃CHF | 4-F-C₆H₄- |
| A-523. | CH₃CHF | 4-Cl-C₆H₄- |
| A-524. | CH₃CHF | 4-Br-C₆H₄- |
| A-525. | CH₃CHF | 4-(C₆H₅)-C₆H₄- |
| A-526. | CH₃CHF | 4-phenoxyphenyl |
| A-527. | CH₃CHF | benzyl |
| A-528. | CH₃CHF | 4-CH₃-C₆H₄-CH₂- |
| A-529. | CH₃CHF | 4-C₂H₅-C₆H₄-CH₂- |
| A-530. | CH₃CHF | 4-n-C₃H₇-C₆H₄-CH₂- |
| A-531. | CH₃CHF | 4-isopropyl-C₆H₄-CH₂- |
| A-532. | CH₃CHF | 4-n-C₄H₉-C₆H₄-CH₂- |
| A-533. | CH₃CHF | 4-isobutyl-C₆H₄-CH₂- |
| A-534. | CH₃CHF | 4-tert-buyl-C₆H₄CH₂- |
| A-535. | CH₃CHF | 4-F₃C-C₆H₄CH₂- |
| A-536. | CH₃CHF | 4-methoxy-C₆H₄CH₂- |
| A-537. | CH₃CHF | 4-ethoxy-C₆H₄CH₂- |
| A-538. | CH₃CHF | 4-n-propoxy-C₆H₄CH₂- |
| A-539. | CH₃CHF | 4-isopropoxy-C₆H₄CH₂- |
| A-540. | CH₃CHF | 4-n-butoxy-C₆H₄CH₂- |
| A-541. | CH₃CHF | 4-tert-butoxy-C₆H₄CH₂- |
| A-542. | CH₃CHF | 4-F-C₆H₄CH₂- |
| A-543. | CH₃CHF | 4-Cl-C₆H₄CH₂- |
| A-544. | CH₃CHF | 4-Br-C₆H₄CH₂- |
| A-545. | CH₃CHF | 3,4-F₂-C₆H₃CH₂- |
| A-546. | CH₃CHF | 3,4-Cl₂-C₆H₃CH₂- |
| A-547. | CH₃CHF | 3,4-Br₂-C₆H₃CH₂- |
| A-548. | CH₃CHF | 4-(4-CH₃-C₆H₄-O)-C₆H₄-CH₂- |
| A-549. | CH₃CHF | 4-(4-C₂H₅-C₆H₄-O)-C₆H₄-CH₂- |
| A-550. | CH₃CHF | 4-(4-n-C₃H₇-C₆H₄-O)-C₆H₄-CH₂- |
| A-551. | CH₃CHF | 4-(4-iso-C₃H₇-C₆H₄-O)-C₆H₄-CH₂- |
| A-552. | CH₃CHF | 4-(4-n-C₄H₉-C₆H₄-O)-C₆H₄-CH₂- |
| A-553. | CH₃CHF | 4-(4-iso-C₄H₉-C₆H₄-O)-C₆H₄-CH₂- |
| A-554. | CH₃CHF | 4-(4-tert-C₄H₉-C₆H₄-O)-C₆H₄-CH₂- |
| A-555. | CH₃CHF | 4-(4-H₃C-O-C₆H₄-O)-C₆H₄-CH₂- |
| A-556. | CH₃CHF | 4-(4-H₃C-H₂C-O-C₆H₄-O)-C₆H₄-CH₂- |
| A-557. | CH₃CHF | 4-(4-H₃C-H₂C-H₂C-O-C₆H₄-O)-C₆H₄-CH₂- |
| A-558. | CH₃CHF | 4-(4-H₃C-H₂C-H₂C-H₂C-O-C₆H₄-O)-C₆H₄-CH₂- |
| A-559. | CH₃CHF | 4-(4-tert-butoxy-C₆H₄-O)-C₆H₄-CH₂- |
| A-560. | CH₃CHF | 4-(4-Cl-C₆H₄-O)-C₆H₄-CH₂- |
| A-561. | CH₃CHF | 4-(4-Br-C₆H₄-O)-C₆H₄-CH₂- |
| A-562. | CH₃CHF | 4-(3-CH₃-C₆H₄-O)-C₆H₄-CH₂- |
| A-563. | CH₃CHF | 4-(3-C₂H₅-C₆H₄-O)-C₆H₄-CH₂- |
| A-564. | CH₃CHF | 4-(3-n-C₃H₇-C₆H₄-O)-C₆H₄-CH₂- |
| A-565. | CH₃CHF | 4-(3-iso-C₃H₇-C₆H₄-O)-C₆H₄-CH₂- |
| A-566. | CH₃CHF | 4-(3-n-C₄H₉-C₆H₄-O)-C₆H₄-CH₂- |
| A-567. | CH₃CHF | 4-(3-iso-C₄H₉-C₆H₄-O)-C₆H₄-CH₂- |
| A-568. | CH₃CHF | 4-(3-tert-butyl-C₆H₄-O)-C₆H₄-CH₂- |
| A-569. | CH₃CHF | 4-(3-H₃C-O-C₆H₄-O)-C₆H₄-CH₂- |
| A-570. | CH₃CHF | 4-(3-H₃C-H₂C-O-C₆H₄-O)-C₆H₄-CH₂- |
| A-571. | CH₃CHF | 4-(3-H₃C-H₂C-H₂C-O-C₆H₄-O)-C₆H₄-CH₂- |
| A-572. | CH₃CHF | 4-(3-H₃C-H₂C-H₂C-H₂C-O-C₆H₄-O)-C₆H₄-CH₂- |
| A-573. | CH₃CHF | 4-(3-tert-butoxy-C₆H₄-O)-C₆H₄-CH₂- |
| A-574. | CH₃CHF | 4-(3-F-C₆H₄-O)-C₆H₄-CH₂- |
| A-575. | CH₃CHF | 4-(3-Cl-C₆H₄-O)-C₆H₄-CH₂- |
| A-576. | CH₃CHF | 4-(3-Br-C₆H₄-O)-C₆H₄-CH₂- |
| A-577. | CH₃CHF | 4-(2-CH₃-C₆H₄-O)-C₆H₄-CH₂- |
| A-578. | CH₃CHF | 4-(2-C₂H₅-C₆H₄-O)-C₆H₄-CH₂- |
| A-579. | CH₃CHF | 4-(2-n-C₃H₇-C₆H₄-O)-C₆H₄-CH₂- |
| A-580. | CH₃CHF | 4-(2-iso-C₃H₇-C₆H₄-O)-C₆H₄-CH₂- |
| A-581. | CH₃CHF | 4-(2-n-C₄H₉-C₆H₄-O)-C₆H₄-CH₂- |
| A-582. | CH₃CHF | 4-(2-iso-C₄H₉-C₆H₄-O)-C₆H₄-CH₂- |
| A-583. | CH₃CHF | 4-(2-tert-butyl-C₆H₄-O)-C₆H₄-CH₂- |
| A-584. | CH₃CHF | 4-(2-H₃C-O-C₆H₄-O)-C₆H₄-CH₂- |
| A-585. | CH₃CHF | 4-(2-H₃C-₂C-O-C₆H₄-O)-C₆H₄-CH₂- |
| A-586. | CH₃CHF | 4-(2-H₃C-H₂C-H₂C-O-C₆H₄-O)-C₆H₄-CH₂- |
| A-587. | CH₃CHF | 4-(2-H₃C-H₂C-H₂C-H₂C-O-C₆H₄-O)-C₆H₄-CH₂- |
| A-588. | CH₃CHF | 4-(2-tert-butoxy-C₆H₄-O)-C₆H₄-CH₂- |
| A-589. | CH₃CHF | 4-(2-F-C₆H₄-O)-C₆H₄-CH₂- |
| A-590. | CH₃CHF | 4-(2-Cl-C₆H₄-O)-C₆H₄-CH₂- |
| A-591. | CH₃CHF | 4-(2-Br-C₆H₄-O)-C₆H₄-CH₂- |
| A-592. | CH₃CHF | 4-(3,4-F₂-C₆H₃-O)-C₆H₄-CH₂- |
| A-593. | CH₃CHF | 4-(3,4-Cl₂-C₆H₃-O)-C₆H₄-CH₂- |
| A-594. | CH₃CHF | 4-(3,4-(CH₃)₂-C₆H₃-O)-C₆H₄-CH₂- |
| A-595. | CH₃CHF | 4-(3-F--4-Cl-C₆H₃-O)-C₆H₄-CH₂- |
| A-596. | CH₃CHF | 4-(3-Cl-4-F-C₆H₃-O)-C₆H₄-CH₂- |
| A-597. | CH₃CHF | 4-(3-CH₃-4-Cl-C₆H₃-O)-C₆H₄-CH₂- |
| A-598. | CH₃CHF | 4-(3-Cl-4-CH₃-C₆H₃-O)-C₆H₄-CH₂- |
| A-599. | CH₃CHF | 4-(3-Cl-4-Br-C₆H₃-O)-C₆H₄-CH₂- |
| A-600. | CH₃CHF | 4-(3-CH₃-4-Br-C₆H₃-O)-C₆H₄-CH₂- |
| A-601. | CH₃CHF | (±) phenyl-CH(CH₃)- |
| A-602. | CH₃CHF | (R) phenyl-CH(CH₃)- |
| A-603. | CH₃CHF | (S) phenyl-CH(CH₃)- |
| A-604. | CH₃CHF | (±) 4-F-phenyl-CH(CH₃)- |
| A-605. | CH₃CHF | (R) 4-F-phenyl-CH(CH₃)- |
| A-606. | CH₃CHF | (S) 4-F-phenyl-CH(CH₃)- |
| A-607. | CH₃CHF | (±) 4-Cl-phenyl-CH(CH₃)- |
| A-608. | CH₃CHF | (R) 4-Cl-phenyl-CH(CH₃)- |
| A-609. | CH₃CHF | (S) 4-Cl-phenyl-CH(CH₃)- |
| A-610. | CH₃CHF | (±) 4-Br-phenyl-CH(CH₃)- |
| A-611. | CH₃CHF | (R) 4-Br-phenyl-CH(CH₃)- |
| A-612. | CH₃CHF | (S) 4-Br-phenyl-CH(CH₃)- |
| A-613. | CH₃CHF | (±) 4-Cl-2-F-phenyl-CH(CH₃)- |
| A-614. | CH₃CHF | (R) 4-Cl-2-F-phenyl-CH(CH₃)- |
| A-615. | CH₃CHF | (S) 4-Cl-2-F-phenyl-CH(CH₃)- |
| A-616. | CH₃CHF | (±) 6-Cl-2-F-phenyl-CH(CH₃)- |
| A-617. | CH₃CHF | (R) 6-Cl-2-F-phenyl-CH(CH₃)- |
| A-618. | CH₃CHF | (S) 6-Cl-2-F-phenyl-CH(CH₃)- |
| A-619. | CH₃CHF | (±) 2-F-phenyl-CH(CH₃)- |
| A-620. | CH₃CHF | (R) 2-F-phenyl-CH(CH₃)- |
| A-621. | CH₃CHF | (S) 2-F-phenyl-CH(CH₃)- |
| A-622. | CH₃CHF | (S) 2,4-F₂-phenyl-CH(CH₃)- |
| A-623. | CH₃CHF | (±) 2,4-F₂-phenyl-CH(CH₃)- |
| A-624. | CH₃CHF | (R) 2,4-F₂-phenyl-CH(CH₃)- |
| A-625. | CH₃CHF | (S) 2,5-F₂-phenyl-CH(CH₃)- |
| A-626. | CH₃CHF | (±) 2,5-F₂-phenyl-CH(CH₃)- |
| A-627. | CH₃CHF | (R) 2,5-F₂-phenyl-CH(CH₃)- |
| A-628. | CH₃CHF | (S) 2,6-F₂-phenyl-CH(CH₃)- |
| A-629. | CH₃CHF | (±) 2,6-F₂-phenyl-CH(CH₃)- |
| A-630. | CH₃CHF | (R) 2,6-F₂-phenyl-CH(CH₃)- |
| A-631. | CH₃CHF | (±) 2-CH₃O-phenyl-CH(CH3)- |
| A-632. | CH₃CHF | (R) 2-CH₃O-phenyl-CH(CH₃)- |
| A-633. | CH₃CHF | (S) 2-CH₃O-phenyl-CH(CH₃)- |
| A-634. | CH₃CHF | (±) 4-CH₃O-phenyl-CH(CH₃)- |
| A-635. | CH₃CHF | (R) 4-CH₃O-phenyl-CH(CH₃)- |
| A-636. | CH₃CHF | (S) 4-CH₃O-phenyl-CH(CH₃)- |
| A-637. | CH₃CHF | (±) 4-H₅C₂-O-phenyl-CH(CH₃)- |
| A-638. | CH₃CHF | (R) 4-H₅C₂-O-phenyl-CH(CH₃)- |
| A-639. | CH₃CHF | (S) 4-H₅C₂-O-phenyl-CH(CH₃)- |
| A-640. | CH₃CHF | (±) 4-n-propoxy-phenyl-CH(CH₃)- |
| A-641. | CH₃CHF | (R) 4-n-propoxy-phenyl-CH(CH₃)- |
| A-642. | CH₃CHF | (S) 4-n-propoxy-phenyl-CH(CH₃)- |
| A-643. | CH₃CHF | (±) 4-n-butoxy-phenyl-CH(CH₃)- |
| A-644. | CH₃CHF | (R) 4-n-butoxyx-phenyl-CH(CH₃)- |
| A-645. | CH₃CHF | (S) 4-n-butoxyphenyl-CH(CH₃)- |
| A-646. | CH₃CHF | (±) 4-tert-butoxy-phenyl-CH(CH₃)- |
| A-647. | CH₃CHF | (R) 4-tert-butoxyx-phenyl-CH(CH₃)- |
| A-648. | CH₃CHF | (S) 4-tert-butoxyphenyl-CH(CH₃)- |
| A-649. | CH₃CHF | (±) 4-CH₃-phenyl-CH(CH₃)- |
| A-650. | CH₃CHF | (R) 4-CH₃-phenyl-CH(CH₃)- |
| A-651. | CH₃CHF | (S) 4-CH₃-phenyl-CH(CH₃)- |
| A-652. | CH₃CHF | (±) 4-C₂H₅-phenyl-CH(CH₃)- |
| A-653. | CH₃CHF | (R) 4-C₂H₅-phenyl-CH(CH₃)- |
| A-654. | CH₃CHF | (S) 4-C₂H₅-phenyl-CH(CH₃)- |
| A-655. | CH₃CHF | (±) 4-n-C₃H₇-phenyl-CH(CH₃)- |
| A-656. | CH₃CHF | (R) 4-n-C₃H₇-phenyl-CH(CH₃)- |
| A-657. | CH₃CHF | (S) 4-n-C₃H₇-phenyl-CH(CH₃)- |
| A-658. | CH₃CHF | (±) 4-iso-C₃H₇-phenyl-CH(CH₃)- |
| A-659. | CH₃CHF | (R) 4-iso-C₃H₇-phenyl-CH(CH₃)- |
| A-660. | CH₃CHF | (S) 4-iso-C₃H₇-phenyl-CH(CH₃)- |
| A-661. | CH₃CHF | (±) 4-n-C₄H₉-phenyl-CH(CH₃)- |
| A-662. | CH₃CHF | (R) 4-n-C₄H₉-phenyl-CH(CH₃)- |
| A-663. | CH₃CHF | (S) 4-n-C₄H₉-phenyl-CH(CH₃)- |
| A-664. | CH₃CHF | (±) 4-tert-C₄H₉-phenyl-CH(CH₃)- |
| A-665. | CH₃CHF | (R) 4-tert-C₄H₉-phenyl-CH(CH₃)- |
| A-666. | CH₃CHF | (S) 4-tert-C₄H₉-phenyl-CH(CH₃)- |
| A-667. | CH₃CHF | (±) 4-cycl.-C₆H₁₁-phenyl-CH(CH₃)- |
| A-668. | CH₃CHF | (R) 4-cycl.-C₆H₁₁-phenyl-CH(CH₃)- |
| A-669. | CH₃CHF | (S) 4-cycl.-C₆H₁₁-phenyl-CH(CH₃)- |
| A-670. | CH₃CHF | (±) 4-OCF₃-phenyl-CH(CH₃)- |
| A-671. | CH₃CHF | (R) 4-OCF₃-phenyl-CH(CH₃)- |
| A-672. | CH₃CHF | (S) 4-OCF₃-phenyl-CH(CH₃)- |
| A-673. | CH₃CHF | (±) 4-CF₃-phenyl-CH(CH₃)- |
| A-674. | CH₃CHF | (R) 4-CF₃-phenyl-CH(CH₃)- |
| A-675. | CH₃CHF | (S) 4-CF₃-phenyl-CH(CH₃)- |
| A-676. | CH₃CHF | (±) 3-F-phenyl-CH(CH₃)- |
| A-677. | CH₃CHF | (R) 3-F-phenyl-CH(CH₃)- |
| A-678. | CH₃CHF | (S) 3-F-phenyl-CH(CH₃)- |
| A-679. | CH₃CHF | (±) 3-Cl-phenyl-CH(CH₃)- |
| A-680. | CH₃CHF | (R) 3-Cl-phenyl-CH(CH₃)- |
| A-681. | CH₃CHF | (S) 3-Cl-phenyl-CH(CH₃)- |
| A-682. | CH₃CHF | (±) 3-Br-phenyl-CH(CH₃)- |
| A-683. | CH₃CHF | (R) 3-Br-phenyl-CH(CH₃)- |
| A-684. | CH₃CHF | (S) 3-Br-phenyl-CH(CH₃)- |
| A-685. | CH₃CHF | (±) 3-CF₃-phenyl-CH(CH₃)- |
| A-686. | CH₃CHF | (R) 3-CF₃-phenyl-CH(CH₃)- |
| A-687. | CH₃CHF | (S) 3-CF₃-phenyl-CH(CH₃)- |
| A-688. | CH₃CHF | (±) 3,4-F₂-phenyl-CH(CH₃)- |
| A-689. | CH₃CHF | (R) 3,4-F₂-phenyl-CH(CH₃)- |
| A-690. | CH₃CHF | (S) 3,4-F₂-phenyl-CH(CH₃)- |
| A-691. | CH₃CHF | (±) 3,4-Cl₂-phenyl-CH(CH₃)- |
| A-692. | CH₃CHF | (R) 3,4-Cl₂-phenyl-CH(CH₃)- |
| A-693. | CH₃CHF | (S) 3,4-Cl₂-phenyl-CH(CH₃)- |
| A-694. | CH₃CHF | (±) 3,4-Br₂-phenyl-CH(CH₃)- |
| A-695. | CH₃CHF | (R) 3,4-Br₂-phenyl-CH(CH₃)- |
| A-696. | CH₃CHF | (S) 3,4-Br₂-phenyl-CH(CH₃)- |
| A-697. | CH₃CHF | (±) 4-Difluoromethoxyphenyl-CH(CH₃)- |
| A-698. | CH₃CHF | (R) 4-Difluoromethoxyphenyl-CH(CH₃)- |
| A-699. | CH₃CHF | (S) 4-Difluoromethoxyphenyl-CH(CH₃)- |
| A-700. | CH₃CHF | (±) 4-(1,1,2,2-tetrafluoroethoxy)phenyl-CH(CH₃)- |
| A-701. | CH₃CHF | (R) 4-(1,1,2,2-tetrafluoroethoxy)phenyl-CH(CH₃)- |
| A-702. | CH₃CHF | (S) 4-(1,1,2,2-tetrafluoroethoxy)phenyl-CH(CH₃)- |
| A-703. | CH₃CHF | (±) (5,5,7,7-tetramethylindan-2-yl)-CH(CH₃)- |
| A-704. | CH₃CHF | (R)(5,5,7,7-tetramethylindan-2-yl)-CH(CH₃)- |
| A-705. | CH₃CHF | (S) (5,5,7,7-tetramethylindan-2-yl)-CH(CH₃)- |
| A-706. | CH₃CHF | (±) (1,1,4,4,-tetramethyl-1,2,3,4- tetrahydronaphthalin-6-yl)-CH(CH₃)- |
| A-707. | CH₃CHF | (R) (1,1,4,4,-tetramethyl-1,2,3,4-tetrahydronaphthalin-6-yl)-CH(CH₃)- |
| A-708. | CH₃CHF | (S) (1,1,4,4,-tetramethyl-1,2,3,4-tetrahydronaphthalin-6-yl)-CH(CH₃)- |
| A-709. | CH₃CHF | (±) (1,1-dimethyl-1,2,3,4-tetrahydronaphthalin-6-yl)-CH(CH₃)- |
| A-710. | CH₃CHF | (R) (1,1-dimethyl-1,2,3,4-tetrahydronaphthalin-6-yl)-CH(CH₃)- |
| A-711. | CH₃CHF | (S) (1,1-dimethyl-1,2,3,4-tetrahydronaphthalin-6-yl)-CH(CH₃)- |
| A-712. | CH₃CHF | (±) (1,1,4,4,7-pentamethyl-1,2,3,4-tetrahydronaphthalin-6-yl)-CH(CH₃)- |
| A-713. | CH₃CHF | (R) (1,1,4,4,7-pentamethyl-1,2,3,4-tetrahydronaphthalin-6-yl)-CH(CH₃)- |
| A-714. | CH₃CHF | (S) (1,1,4,4,7-pentamethyl-1,2,3,4-tetrahydronaphthalin-6-yl)-CH(CH₃)- |
| A-715. | CH₃CHF | (±) (1,1,7-trimethyl-1,2,3,4-tetrahydronaphthalin-6-yl)-CH(CH₃)- |
| A-716. | CH₃CHF | (R) (1,1,7-trimethyl-1,2,3,4-tetrahydronaphthalin-6-yl)-CH(CH₃)- |
| A-717. | CH₃CHF | (S) (1,1,7-trimethyl-1,2,3,4-tetrahydronaphthalin-6-yl)-CH(CH₃)- |
| A-718. | CH₃CHF | (±) (2-methyl-1,3-dioxan-2-y)-CH(CH₃)- |
| A-719. | CH₃CHF | (R) 2-methyl-1,3-dioxan-2-yl)-CH(CH₃)- |
| A-720. | CH₃CHF | (S) 2-methyl-1,3-dioxan-2-yl)-CH(CH₃)- |
| A-721. | CH₃CHF | (±) (2,5,5-trimethyl-1,3-dioxan-2-yl)-CH(CH₃)- |
| A-722. | CH₃CHF | (R) 2,5,5-trimethyl-1,3-dioxan-2-yl)-CH(CH₃)- |
| A-723. | CH₃CHF | (S) 2,5,5-trimethyl-1,3-dioxan-2-yl)-CH(CH₃)- |
| A-724. | CH₃CHF | (±) (2,2-difluorobenzodioxole-5-yl)CH(C₂H₅)- |
| A-725. | CH₃CHF | (R) (2,2-difluorobenzodioxole-5-yl)CH(C₂H₅)- |
| A-726. | CH₃CHF | (S) (2,2-difluorobenzodioxole-5-yl)CH(C₂H₅)- |
| A-727. | CH₃CHF | C₆H₅CH₂CH₂- |
| A-728. | CH₃CHF | 4-F-C₆H₄CH₂CH₂- |
| A-729. | CH₃CHF | 4-Cl-C₆H₄CH₂CH₂- |
| A-730. | CH₃CHF | 4-Br-C₆H₄CH₂CH₂- |
| A-731. | CH₃CHF | 4-CH₃O-C₆H₄CH₂CH₂- |
| A-732. | CH₃CHF | 4-C₂H₅O-C₆H₄CH₂CH₂- |
| A-733. | CH₃CHF | 4-n-C₃H₇O-C₆H₄CH₂CH₂- |
| A-734. | CH₃CHF | 4-n-C₄H₉O-C₆H₄CH₂CH₂- |
| A-735. | CH₃CHF | 4-t-C₄H₉O-C₆H₄CH₂CH₂- |
| A-736. | CH₃CHF | 3,4-(CH₃O)₂-C₆H₄CH₂CH₂- |
| A-737. | CH₃CHF | 4-H₃C-C₆H₄CH₂CH₂- |
| A-738. | CH₃CHF | 4-H₃C-H₂C-C₆H₄CH₂CH₂- |
| A-739. | CH₃CHF | 4-H₃C-H₂C-H₂C-C₆H₄CH₂CH₂- |
| A-740. | CH₃CHF | 4-H₃C-H₂C-H₂C-H₂C-C₆H₄CH₂CH₂- |
| A-741. | CH₃CHF | 4-(CH₃)₃C-C₆H₄CH₂CH₂- |
| A-742. | CH₃CHF | 4-F₃CO-C₆H₄CH₂CH₂- |
| A-743. | CH₃CHF | 4-F₃C-C₆H₄CH₂CH₂- |
| A-744. | CH₃CHF | 3-F₃C-C₆H₄CH₂CH₂- |

Examples of one group of used compounds are the 6-chloro-[1,2,4]triazolo-[1,5-a]pyrimidines of formula I, wherein X is Cl, R¹ is CH₃ and R³ is H and R⁴-A and R² together have the meanings given in Table A.

Examples of a second group of used compounds are the 6-chloro-[1,2,4]triazolo-[1,5-a]pyrimidines of formula I, wherein X is Cl, R¹ is OCH₃, R³ is H and R⁴-A and R² together have the meanings given in Table A.

Examples of a third group of used compounds also are the 6-chloro-[1,2,4]triazolo-[1,5-a]pyrimidines of formula I, wherein X is Cl, R¹ is SCH₃, R³ is H and R⁴-A and R² together have the meanings given in Table A.

Examples of a fourth group of used compounds also are the 6-chloro-[1,2,4]triazolo-[1,5-a]pyrimidines of formula I, wherein X is Cl, R¹ is SOCH₃, R³ is H and R⁴-A and R² together have the meanings given in Table A.

Examples of a fifth group of used compounds also are the 6-chloro-[1,2,4]triazolo-[1,5-a]pyrimidines of formula I, wherein X is Cl, R¹ is SO₂CH₃, R³ is H and R⁴-A and R² together have the meanings given in Table A.

Examples of a sixth group of used compounds also are the 6-chloro-[1,2,4]triazolo-[1,5-a]pyrimidines of formula I, wherein X is Cl, R¹ is CF₃, R³ is H and R⁴-A and R² together have the meanings given in Table A.

Examples of a seventh group of used compounds also are the 6-chloro-[1,2,4]triazoto-[1,5-a]pyrimidines of formula I, wherein X is Cl, R¹ is CN, R³ is H and R⁴-A and R² together have the meanings given in Table A.

Examples of an eighth group of used compounds also are the 6-chloro-[1,2,4]triazolo-[1,5-a]pyrimidines of formula I, wherein X is Cl, R¹ is OH, R³ is H and R⁴-A and R² together have the meanings given in Table A.

Examples of a ninth group of used compounds also are the 6-chloro-[1,2,4]triazolo-[1,5-a]pyrimidines of formula I, wherein X is Cl, R¹ is OCHF₂, R³ is H and R⁴-A and R² together have the meanings given in Table A.

Examples of a tenth group of used compounds also are the 6-chloro-[1,2,4]triazolo-[1,5-a]pyrimidines of formula I, wherein X is Cl, R¹ is OCF₂CHF₂, R³ is H and R⁴-A and R² together have the meanings given in Table A.

Examples of an eleventh group of used compounds also are the 6-chloro-[1,2,4]triazolo-[1,5-a]pyrimidines of formula I, wherein X is Cl, R¹ is OCF₃, R³ is H and R⁴-A and R² together have the meanings given in Table A.

Examples of a twelfth group of compounds also are the 6-chloro-[1,2,4]triazolo-[1,5-a]pyrimidines of formula I, wherein X is Cl, R¹ is OCH₂C₆H₅, R³ is H and R⁴-A and R² together have the meanings given in Table A.

Examples of a thirteenth group of used compounds also are the 6-chloro-[1,2,4]triazolo-[1,5-a]pyrimidines of formula I, wherein X is Cl, R¹ is OCH₂CH₃, R³ is H and R⁴-A and R² together have the meanings given in Table A.

Examples of a fourteenth group of used compounds also are the 6-chloro-[1,2,4]triazolo-[1,5-a]pyrimidines of formula I, wherein X is Cl, R¹ is OCH₂C≡CH, R³ is H and R⁴-A and R² together have the meanings given in Table A.

Examples of a fifteenth group of used compounds also are the 6-chloro-[1,2,4]triazolo-[1,5-a]pyrimidines of formula I, wherein X is Cl, R¹ is OCH(CH₃)₂, R³ is H and R⁴-A and R² together have the meanings given in Table A.

Examples of a sixteenth group of used compounds also are the 6-chloro-[1,2,4]triazolo-[1,5-a]pyrimidines of formula I, wherein X is Cl, R¹ is O-CH₂CH₂-O-CH₃, R³ is H and R⁴-A and R² together have the meanings given in Table A.

Examples of a seventeenth group of used compounds also are the 6-chloro-[1,2,4]triazolo-[1,5-a]pyrimidines of formula I, wherein X is Cl, R¹ is O-CH₂CH₂-O-CH₂CH₂-O-CH₃, R³ is H and R⁴-A and R² together have the meanings given in Table A.

Examples of an eighteenth group of used compounds also are the 6-chloro-[1,2,4]triazolo-[1,5-a]pyrimidines of formula I, wherein X is Cl, R¹ is S-CH₂CH₂-O-CH₃, R³ is H and R⁴-A and R² together have the meanings given in Table A.

Examples of a nineteenth group of used compounds also are the 6-chloro-[1,2,4]triazolo-[1,5-a]pyrimidines of formula I, wherein X is Cl, R¹ is S-CH₂C(O)-O-CH₃, R³ is H and R⁴-A and R² together have the meanings given in Table A.

Examples of a twentieth group of used compounds also are the 6-chloro-[1,2,4]triazolo-[1,5-a]pyrimidines of formula I, wherein X is Cl, R¹ is S-CH₂CH₂C(O)-O-CH₃, R³ is H and R⁴-A and R² together have the meanings given in Table A.

Examples of a twenty-first group of used compounds also are the 6-chloro-[1,2,4]triazolo-[1,5-a]pyrimidines of formula I, wherein X is Cl, R¹ is S-CH₂C(O)-OH, R³ is H and R⁴-A and R² together have the meanings given in Table A.

### Method of preparing 6-halogeno-[1,2,4]-triazolo-[1,5-a]-pyrimidine compounds

The 6-halogeno-[1,2,4]triazolo-[1,5-a]pyrimidines of formula I according to the present invention can be prepared as described in WO2005/025315 (U.S. Patent Publication 2006/264446). They can be prepared for example, similarly to a process described in Pharmazie, 1971, 26, 534 ff or in DD 99 794 or any other method coming within the competence of a person skilled in the art who is a specialist in chemical synthesis. For the chemical preparation of the products of the invention, the person skilled in the art is regarded as having available, inter alia, all the contents of "Chemical Abstracts" and of the documents which are cited therein, organic chemistry texts such as March's Advanced Organic Chemistry - Reactions, Mechanisms and Structure (6th Edition), Wiley-Interscience, (2007); Vogel's Textbook of Practical Organic Chemistry (5th Edition), Longman Scientific & Technical, (1989); Protective Groups in Organic Synthesis (3rd Edition), Wiley Interscience, (1999); etc.

### Embodiments of Parasites

Parasite, within the meaning of the present invention, is understood to mean not only true parasites, but also insects or other vermin capable of soiling occasional or permanent hosts or of otherwise harming them. The parasites targeted by the invention are mainly composed of arthropods which includes insects and Arachnida, including Acarina.

One embodiment of the description is a method for removing parasites of vertebrates and which is inclusive of, but not exclusive of, ectoparasites of vertebrates which include but are not limited to mammals, wherein the ectoparasites include arthropods, such as insects and Arachnida; wherein an effectively parasiticidal amount of a compound of formula I as defined above is administered to the animal via an administration route which makes possible good effectiveness of the compound against said parasites.

Another embodiment of the description isa method for the removal of fleas which includes Ctenocephalides spp. and the species felis; ticks, which includes Rhipicephalus spp., and the species sanguineus and Boophilus spp., which includes the species microplus; myiasis-causing parasites or mange, which includes Sarcoptes spp., and the species cabiei; and lice, which includes Damalinia spp. and Linognathus spp., in companion animals (such as dogs and cats), cattle, goats and sheep, and Suidae (which includes pigs).

Compounds of formula I or the enantiomers or veterinarily acceptable salts thereof and compositions comprising them are preferably for use in controlling and preventing infestations and infections in animals including warm-blooded animals (including humans) and fish. They are for example suitable for controlling and preventing infestations and infections in mammals including but not limited to cattle, sheep, swine, camels, deer, horses, pigs, poultry, Ilamas, alpacas, rabbits, goats, dogs and cats, water buffalo, donkeys, fallow deer and reindeer, and also in fur-bearing animals including but not limited to mink, chinchilla and raccoon, birds such as hens, geese, turkeys and ducks and fish including but not limited to fresh- and salt-water fish such as trout, carp and eels.

In one embodiment of the invention, the compounds of formula I or the enantiomers or veterinarily acceptable salts thereof and compositions comprising them are for use in controlling and preventing infestations and Infections in domestic animals, such as dogs or cats.

Infestations in warm-blooded animals and fish include, but are not limited to, lice, biting lice, ticks, nasal bots, keds, biting files, muscold files, files, mylasitic fly larvae, chiggers, gnats, mosquitoes and fleas,

The compounds of formula I or the enantiomers or veterinarily acceptable salts thereof and compositions comprising them are suitable for systemic and/or non-systemic control of ecto- and/or endoparasites. They are active against all or some stages of development of parasites.

As described herein, the compounds of formula I are for use in combating ectoparasites.

As described herein, the compounds of formula I are for use in combating parasites of the following orders and species, respectively:
fleas (Siphonaptera), e.g. Ctenocephalides fells, Ctenocephalides canis, Xenopsylla cheopis, Pulex irritans, Tunga penetrans, and Nosopsyllus fasciatus,
cockroaches (Blattaria - Blattodea), e.g. Blattella germanica, Blattella asahinae, Periplaneta americana, Periplaneta japonica, Periplaneta brunnea, Periplaneta fuligginosa, Periplaneta australasiae, and Blatta orlentalls,
flies, mosquitoes (Diptera), e.g. Aedes aegypti, Aedes albopictus, Aedes vexans, Anastrepha ludens, Anopheles maculipennls, Anopheles cruclans, Anopheles albimanus, Anopheles gambiae, Anopheles freebornl, Anopheles leucosphyrus, Anopheles minimus, Anopheles quadrimaculatus, Calliphora vicitia, Ceratitis capitata, Chrysomya bezziana, Chrysomya hominivorax, Chrysomya macellaria, Chrysops discalls, Chrysops silacea, Chrysops atlanticus, Cochilomyla hominivorax, Cordylobia anthropophaga, Culicoides furens, Culex pipiens, Culex nigripalpus, Culex quinquefasciatus, Culex tarsalis, Culiseta inornata, Culiseta melanura, Dermatobla hominis, Fannia canicularis, Gasterophilus intestinalis, Glossina morsitans, Glossina palpalis, Glossina fuscipes, Glossina tachinoides, Haematobia irritans, Hapiodiplosis equestris, Hippelates spp,, Hypoderma lineata, Leptoconops torrens, Lucilla caprina, Lucilia cuprina, Lucilia sericata, Lycoria pectoralis, Mansonia spp., Musca domestica, Muscina stabulans, Oestrus ovis, Phlebotomus argentipes, Psorophora columbiae, Psorophora discolor, Prosimullum mixtum, Sarcophaga haemorrhoidalis, Sarcophaga sp., Simulium vittatum, Stomoxys calcitrans, Tabanus bovinus, Tabanus atratus, Tabanus lineola, and Tabanus similis,
lice (Phthiraptera), e.g. Pediculus humanus capitis, Pediculus humanus corporis, Pthirus pubis, Haematopinus eurysternus, Haematopinus suis, Linognathus vituli, Bovicola bovis, Menopon gallinae, Menacanthus stramineus and Solenopotes capillatus.

ticks and parasitic mites (Parasitiformes): ticks (Ixodida), e.g. Ixodes scapularis, Ixodes holocyclus, Ixodes pacificus, Rhiphicephalus sanguineus, Dermacentor andersoni, Dermacentor variabilis, Amblyomma americanum, Ambryomma maculatum, Ornithodorus hermsi, Ornithodorus turicata and parasitic mites (Mesostigmata), e.g. Ornithonyssus bacoti and Dermanyssus gallinae,

Actinedida (Prostigmata) and Acaridida (Astigmata) e.g. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp.,Knemidocoptes spp., Cytodites spp., and Laminosioptes spp,

Bugs (Heteropterida): Cimex lectularius, Cimex hemipterus, Reduvius senilis, Triatoma spp., Rhodnius ssp., Panstrongylus ssp. and Arilus critatus,

Anoplurida, e.g. Haematopinus spp., Linognathus spp., Pediculus spp., Phtirus spp., and Solenopotes spp,

Mallophagida (suborders Arnblycerina and Ischnocerina), e.g. Trimenopon spp., Menopon spp., Trinoton spp., Bovicola spp., Werneckiella spp., Lepikentron spp., Trichodectes spp., and Felicola spp,

Roundworms (Nematoda):
Whipworms, e.g. Trichinellidae (Trichinella spp.), Trichuridae (Trichuris spp., Capillaria spp.),
Rhabditida, e.g. Rhabditis spp, Strongyloides spp., Helicephalobus spp,
Strongylida, e.g. Strongylus spp., Ancylostoma spp., Necator americanus, Bunostomum spp. (Hookworm), Trichostrongylus spp., Haemonchus contortus., Ostertagia spp., Cooperia spp., Nematodirus spp., Dictyocaulus spp., Cyathostoma spp., Oesophagostomum spp., Stephanurus dentatus, Ollulanus spp., Chabertia spp., Stephanurus dentatus, Syngamus trachea, Ancylostoma spp., Uncinaria spp., Globocephalus spp., Necator spp., Metastrongylus spp., Muellerius capillaris, Protostrongylus spp., Anglostrongylus spp., Parelaphostrongylus spp. Aleurostrongylus abstrusus, and Dioctophyma renale,

Intestinal roundworms (Ascaridida), e.g. Ascaris lumbricoides, Ascaris suum, Ascaridia galli, Parascaris equorum, Enteroblus vermicuiaris (Threadworm), Toxocara canis, Toxascaris leonine, Skrjabinema spp., and Oxyuris equl,

### Camallanida, e.g. Dracunculus medinensis (guinea worm)

Spirurida, e.g. Thelazia spp., Wuchereria spp., Brugia spp., Onchocerca spp., Dirofllari spp.a, Dipetalonema spp., Setaria spp., Elaeophora spp., Spirocerca lupi, and Habronema spp.,

Thorny headed worms (Acanthocephala), e.g. Acanthocephalus spp., Macracanthorhynchus hirudinaceus and Oncicola spp,

Planarians (Plathelminthes):
Flukes (Trematoda), e.g. Faciola spp., Fascloloides magna, Paragonimus spp.,
Dicrocoelium spp., Fasciolopsis buskl, Clonorchls sinensis, Schistosoma spp.,
Trlchobilharzia spp., Alaria alata, Paragonimus spp., and Nanocyetes spp,

Cercomeromorpha, In particular Cestoda (Tapeworms), e.g. Diphyllobothrium spp,, Tenia spp., Echinococcus spp., Dipylidium caninum, Multiceps spp., Hymenolepis spp., Mesocestoides spp., Vampirolepis spp., Monlezia spp., Anoplocephala spp., Sirometra spp., Anoplocephala spp., and Hymenolepis spp.

The compounds of formula I and compositions containing them are useful for the control of pests from the orders Diptera, Siphonaptera and ixodida.

As described herein, the compounds of formula I and compositions containing them are for use in combating mosquitoes.

As described herein, the compounds of formula I and compositions containing them are for use in combating flies.

As described herein, the compounds of formula I and compositions containing them are for use In combating fleas.

As described herein, the compounds of formula I and compositions containing them are for use In combating ticks.

The compounds of formula I also useful for combating endoparasites which Include but are not limited to roundworms (Nematoda), thorny headed worms and planarlans.

### Applications

Administration can be carried out both prophylactically and therapeutically.

Administration of the active compounds is carried out directly or in the form of suitable preparations, orally, topically/dermally or parenterally.

For oral administration to warm-blooded animals, the formula I compounds may be formulated as animal feeds, animal feed premixes, animal feed concentrates, pills, solutions, pastes, suspensions, drenches, gels, tablets, boluses and capsules. In addition, the formula I compounds may be administered to the animals In their drinking water.

For oral administration, the dosage form chosen should provide the animal with an amount of about 0.01 mg/kg to about 100 mg/kg of animal body weight per day of the formula I compound. In another embodiment of oral administration, the dosage form provides the animal with an amount of about 0.5 mg/kg to about 100 mg/kg of animal body weight per day.

Alternatively, the formula I compounds may be for administration to animals parenterally, for example, by intraruminal, intramuscular, Intravenous or subcutaneous injection. The formula I compounds may be dispersed or dissolved in a physiologically acceptable carrier for subcutaneous injection. Alternatively, the formula I compounds may be formulated into an implant for subcutaneous administration. In addition, the formula I compound may be for transdermal administration to animals. For parenteral administration, the dosage form chosen should provide the animal with about 0.01 mg/kg to about 100 mg/kg of animal body weight per day of the formula I compound.

The formula I compounds may also be for topical application to the animals in the form of dips, dusts, powders, collars, medallions, sprays, shampoos, spot-on and pour-on formulations and in ointments or oil-in-water or water-in-oil emulsions. For topical application, dips and sprays usually contain an amount of formula I compound selected from the ranges of about 0.5 ppm to about 5,000 ppm and about 1 ppm to about 3,000 ppm. In addition, the formula I compounds may be formulated as ear tags for animals, including quadrupeds such as cattle and sheep.

Another aspect of the description is therapeutic methods intended for the treatment or prevention of parasitoses having pathogenic consequences, wherein, for example, the compounds of formula I are applied in the elimination of myiasis-causing parasites, in animals such as cattle, horses, goats or sheep, in regions where a significant pressure from these myiasis-causing parasites exists.

Another aspect of the description is therapeutic methods intended for the treatment or prevention of parasitoses having pathogenic consequences, wherein for example, the compounds of formula I are applied in the elimination of ticks, in animals such as cattle or dogs, in regions where the pressure from ticks is of such a nature as to result in pathogenic consequences on a significant scale.

Another subject of the description is methods with a non-therapeutic purpose, e.g., cleaning the coats of animals such as dogs and other companion animals, which are thus rid of parasites such as fleas, and their waste and excreta. The treated animal exhibits a coat which is pleasing to the eye and pleasant to the touch.

Other non-therapeutic methods according to the description are applied, for example, in combating harmful flies, in companion animals or income-producing animals. In another embodiment of the non-therapeutic method, the animals are raised under intensive conditions or in herds.

### Composition forms

Another subject of the description is compositions for the implementation of methods with a therapeutic purpose according to the invention.

Another subject of the description is compositions for the implementation of non-therapeutic methods according to the invention, in particular for cleaning coats.

Suitable composition forms include but are not limited to:
Solutions such as oral solutions, concentrates for oral administration after dilution,
solutions for use on the skin or in body cavities, pouring-on formulations, gels; Emulsions and suspensions for oral or dermal administration; semi-solid preparations;
Formulations in which the active compound is processed in an ointment base or in an oil-in-water or water-in-oil emulsion base;
Solid preparations such as powders, premixes or concentrates, granules, pellets, tablets, boluses, capsules; aerosols and inhalants, and active compound-containing shaped articles.

Compositions suitable for injection are prepared by dissolving the active ingredient in a suitable solvent and optionally adding further ingredients such as acids, bases, buffer salts, preservatives, and solubilizers. The solutions are filtered and filled sterile.
Suitable solvents are physiologically tolerable solvents such as water, alkanols such as ethanol, butanol, benzyl alcohol, glycerol, propylene glycol, polyethylene glycols, N-methyl-pyrrolidone, 2-pyrrolidone, and mixtures thereof.

The parasiticidal compounds used in this invention can optionally be dissolved in physiologically tolerable vegetable or synthetic oils which are suitable for injection.

Suitable solubilizers are solvents which promote the dissolution of the active compound in the main solvent or prevent its precipitation. Examples are polyvinylpyrrolidone, polyvinyl alcohol, polyoxyethylated castor oil, and polyoxyethylated sorbitan ester.

Suitable preservatives are benzyl alcohol, trichlorobutanol, p-hydroxybenzoic acid esters, and n-butanol.

Oral solutions are administered directly. Concentrates are administered orally after prior dilution to the use concentration. Oral solutions and concentrates are prepared according to the state of the art and as described above for injection solutions, sterile procedures not being necessary.

Solutions for use on the skin are trickled on, spread on, rubbed in, sprinkled on or sprayed on.

Solutions for use on the skin are prepared according to the state of the art and according to what is described above for injection solutions, sterile procedures not being necessary. Further suitable solvents are polypropylene glycol, phenyl ethanol, phenoxy ethanol, ester such as ethyl or butyl acetate, benzyl benzoate, ethers such as alkyleneglycol alkylether, e.g. dipropylene glycol monomethyl ether, ketones such as acetone, methylethylketone, aromatic hydrocarbons, vegetable and synthetic oils, dimethylformamide, dimethylacetamide, transcutol, solketal, propylencarbonate, and mixtures thereof.

It may be advantageous to add thickeners during preparation. Suitable thickeners are inorganic thickeners such as bentonites, colloidal silicic acid, aluminium monostearate, and organic thickeners such as cellulose derivatives, polyvinyl alcohols and their copolymers, acrylates and methacrylates.

Gels are applied to or spread on the skin or introduced into body cavities. Gels are prepared by treating solutions which have been prepared as described in the case of the injection solutions with sufficient thickener that a clear material having an ointment-like consistency results. The thickeners employed are the thickeners given above.

Pour-on formulations are poured or sprayed onto limited areas of the skin, the active compound penetrating the skin and acting systemically.

Pour-on formulations are prepared by dissolving, suspending or emulsifying the active compound in a suitable skin-compatible solvent or a solvent mixture. If appropriate, other auxiliaries such as colorants, bioabsorption-promoting substances, antioxidants, light stabilizers, adhesives are added.

Suitable solvents are:
water, alkanols, glycols, polyethylene glycols, polypropylene glycols, glycerol, aromatic alcohols such as benzyl alcohol, phenylethanol, phenoxyethanol, esters such as ethyl acetate, butyl acetate, benzyl benzoate, ethers such as alkylene glycol alkyl ethers such as dipropylene glycol monomethyl ether, diethylene glycol mono-butyl ether, ketones such as acetone, methyl ethyl ketone, cyclic carbonates such as propylene carbonate, ethylene carbonate, aromatic and/or aliphatic hydrocarbons, vegetable or synthetic oils, DMF, dimethylacetamide, n-alkylpyrrolidones such as methylpyrrolidone, n-butylpyrrolidone or n-octylpyrrolidone, N-methylpyrrolidone, 2-pyrrolidone, 2,2-dimethyl-4-oxy-methylene-1,3-dioxolane and glycerol formal.

Suitable colorants are all colorants permitted for use on animals and which can be dissolved or suspended.

Suitable absorption-promoting substances are, for example, DMSO, spreading oils such as isopropyl myristate, dipropylene glycol pelargonate, silicone oils and copolymers thereof with polyethers, fatty acid esters, triglycerides, fatty alcohols.

Suitable antioxidants are sulfites or metabisulfites such as potassium metabisulfite, ascorbic acid, butylhydroxytoluene (BHT), butylhydroxyanisole, tocopherol.

Suitable light stabilizers are, for example, novantisolic acid.

Suitable adhesives are, for example, cellulose derivatives, starch derivatives, polyacrylates, natural polymers such as alginates, gelatin.

Emulsions can be administered orally, dermally or as injections.

Emulsions are either of the water-in-oil (W/O), oil-in-water (O/W), water-in-oil-in water (W/O/W), oil in-water in oil (O/W/O), Pickering emulsions or emulsions which are emulsifier-free.

They are prepared by dissolving the active compound either in the hydrophobic or in the hydrophilic phase and homogenizing this with the solvent of the other phase with the aid of suitable emulsifiers and, if appropriate, other auxiliaries such as colorants, absorption-promoting substances, preservatives, antioxidants, light stabilizers, viscosity-enhancing substances.

Suitable hydrophobic phases (oils) are:
liquid paraffins, silicone oils, natural vegetable oils such as sesame oil, almond oil, castor oil, synthetic triglycerides such as caprylic/capric biglyceride, triglyceride mixture with vegetable fatty acids of the chain length C₈-C₁₂ or other specially selected natural fatty acids, partial glyceride mixtures of saturated or unsaturated fatty acids possibly also containing hydroxyl groups, mono- and diglycerides of the C₈-C₁₀ fatty acids,
fatty acid esters such as ethyl stearate, di-n-butyryl adipate, hexyl laurate, dipropylene glycol perlargonate, esters of a branched fatty acid of medium chain length with saturated fatty alcohols of chain length C₁₆-C₁₈, isopropyl myristate, isopropyl palmitate, caprylic/capric acid esters of saturated fatty alcohols of chain length C₁₂-C₁₈, isopropyl stearate, oleyl oleate, decyl oleate, ethyl oleate, ethyl lactate, waxy fatty acid esters such as synthetic duck coccygeal gland fat, dibutyl phthalate, diisopropyl adipate, and ester mixtures related to the latter, fatty alcohols such as isotridecyl alcohol, 2-octyldodecanol, cetylstearyl alcohol, oleyl alcohol, and fatty acids such as oleic acid and mixtures thereof.

Suitable hydrophilic phases are:
water, alcohols such as propylene glycol, glycerol, sorbitol and mixtures thereof.

Suitable emulsifiers are:
non-ionic surfactants, e.g. polyethoxylated castor oil, polyethoxylated sorbitan monooleate,
sorbitan monostearate, glycerol monostearate, polyoxyethyl stearate, alkylphenol polyglycol ether;
ampholytic surfactants such as di-sodium N-lauryl-p-iminodipropionate or lecithin; anionic surfactants, such as sodium lauryl sulfate, fatty alcohol ether sulfates, mono/dialkyl polyglycol ether orthophosphoric acid ester monoethanolamine salt;
cation-active surfactants, such as cetyltrimethylammonium chloride.

Suitable further auxiliaries are:
substances which enhance the viscosity and stabilize the emulsion, such as carboxymethylcellulose, methylcellulose and other cellulose and starch derivatives, polyacrylates, alginates, gelatin, gum arabic, polyvinylpyrrolidone, polyvinyl alcohol, copolymers of methyl vinyl ether and maleic anhydride, polyethylene glycols, waxes, colloidal silicic acid or mixtures of the substances mentioned.

Suspensions can be administered orally or topically/dermally. They are prepared by suspending the active compound in a suspending agent, if appropriate with addition of other auxiliaries such as wetting agents, colorants, bioabsorption-promoting substances, preservatives, antioxidants, light stabilizers.

Liquid suspending agents are all homogeneous solvents and solvent mixtures.

Suitable wetting agents (dispersants) are the emulsifiers given above.

Other auxiliaries which may be mentioned are those given above.

Semi-solid preparations can be administered orally or topically/dermally. They differ from the suspensions and emulsions described above only by their higher viscosity.

For the production of solid preparations, the active compound is mixed with suitable excipients, if appropriate with addition of auxiliaries, and brought into the desired form.

Suitable excipients are all physiologically tolerable solid inert substances. Those used are inorganic and organic substances. Inorganic substances are, for example, sodium chloride, carbonates such as calcium carbonate, hydrogencarbonates, aluminium oxides, titanium oxide, silicic acids, argillaceous earths, precipitated or colloidal silica, or phosphates. Organic substances are, for example, sugar, cellulose, foodstuffs and feeds such as milk powder, animal meal, grain meals and shreds, starches.

Suitable auxiliaries are preservatives, antioxidants, and/or colorants which have been mentioned above.

Other suitable auxiliaries are lubricants and glidants such as magnesium stearate, stearic acid, talc, bentonites, disintegration-promoting substances such as starch or crosslinked polyvinylpyrrolidone, binders such as starch, gelatin or linear polyvinylpyrrolidone, and dry binders such as microcrystalline cellulose.

In general, "parasiticidally effective amount" means the amount of active ingredient needed to achieve an observable effect on growth, including the effects of necrosis, death, retardation, prevention, and removal, destruction, or otherwise diminishing the occurrence and activity of the target organism. The parasiticidally effective amount can vary for the various compounds/compositions used in the invention. A parasiticidally effective amount of the compositions will also vary according to the prevailing conditions such as desired parasiticidal effect and duration, target species, mode of application, and the like. A parasiticidally effective amount should result in blood and/or tissue concentrations, or residual concentrations on/in skin which are toxic by ingestion or contact by parasitic arthropods, which include but are not limited to biting, sucking or gnawing arthropods.

According to one embodiment of the description the administration of the effective dose to the animal is carried out once or a very small number of times for a duration of activity of at least one month, which can advantageously be two or three months or even six months. In other words, this embodiment is about a permanent combating method in an environment in which the animal is subjected to strong parasitic pressure, wherein a systemic or topical administration is carried out at a frequency well below a daily administration, such as, for example, a monthly administration, or even less than a monthly frequency, for example quarterly or bi-annually.

Moreover, it is particularly noteworthy and surprising to be able to act effectively and for such long periods of time against ectoparasites which live in the integuments or on the fur of the animal by the administration of relatively low and non-toxic doses by systemic or topical administration, without requiring the employment of controlled and long-lasting release means. While not wishing to be bound by theory, it appears that the long-term effectiveness is related to the combination of a very long persistence of the compound with an exceptional toxicity by ingestion and/or contact by the parasite.

The effective dose administered in the method according to the description can be a range selected from the group consisting of between about 0.001 mg/kg and about 100 mg/kg, about 0.01 mg/kg and about 100 mg/kg and about 1 to about 50 mg/kg of animal weight.

For the majority of host species, the dose and the composition for systemic administration are chosen so as to maintain a serum level of a compound according to formula I of greater than or equal to about 1 ng/ml, for example, about 1 to about 50 ng/ml.

Another embodiment of the method for combating parasites according to the description is the combating against fleas and ticks of small companion animals, such as dogs and cats. Serum levels for systemic administration are selected from the group of ranges consisting of about 10 to about 50 ng/ml and about 20 to about 30 ng/ml against fleas and about 10 to about 75 ng/ml and about 30 to about 50 ng/ml against ticks.

Yet another embodiment of the method for combating parasites according to the description is the combating against ticks, flies and myiasis-causing parasites which parasitize large animals such as cattle, goats and sheep.

Another embodiment of the method for combating parasites according to the description is the treatment of porcine mange.

One embodiment for the administration of the composition is by the oral or parenteral route or by a topical formulation with or without a transcutaneous effect.

In another embodiment for the administration of the composition, the compositions according to the description are provided for administration in a single dose or a dose repeated a small number of times and comprise a dose of compound of formula (I) selected from the ranges consisting of between about 0.01 and about 100 mg/kg and about 1 to about 50 mg/kg of body weight of the animal.

The compositions are effective over a fairly wide range of doses, which makes it possible to provide the same dosages for small animals having relatively different sizes.

For administration by the oral route, the composition can optionally be prepared at the time of use, for example by simple mixing of a powdered or dissolved preparation of a composition containing compound of formula I into the food of the animal such as food prepared for dogs or cats.

The composition can, however, also be provided in any other form suitable for oral administration, such as, for example, solutions or suspensions to be taken orally, emulsions, microemulsions, creams, pellets, tablets, gelatin capsules or others.

In one embodiment for the excipient which forms part of the composition for oral administration, the excipient facilitates release of the parasiticidal compounds into the intestines. In another embodiment of the excipient used, a gastroprotected gelatin capsule or gastroresistant tablet is formed.

In an embodiment of the description for large animals, the compositions are delivered in the form of powders, or rumen-resistant compositions, boli or intraruminal devices.

For administration by the parenteral route, such as the subcutaneous or intramuscular route, the compound of formula I can be contained in a liquid excipient which is biologically suitable for injection in the solution, suspension, emulsion or microemulsion form.

The parenteral composition can also be produced in the particulate form, such as nanoparticles and nanocapsules, microparticles, microcapsules or liposomes, or alternatively in the form of an implant.

The composition according to the description can be presented in the form of a single dose without controlled release means. In this case, the dose is between about 1 and about 20 mg/kg of body weight, which makes possible a long-lasting activity of several weeks to several months, which is altogether remarkable.

The compound of formula I can also be contained in a material which provides for controlled release. For example, the compound according to the invention can be contained in microspheres, granules or implants which make possible release by diffusion and/or erosion.

A releasable composition with a dose selected from the ranges consisting of between about 1 and about 50 mg/kg, about 10 to about 30 mg/kg, and 20 mg/kg of body weight, consequently makes possible a long-lasting activity for several months, even a year.

In an embodiment of the description for administration by the parenteral route, the controlled-release formulations are by injection which is suitable for the case of cats or other animals with rapid metabolization.

In an embodiment of the description for transcutaneous-passage compositions, the composition can comprise the abovementioned particulate or liposomal forms, in combination with an absorption promoter.

In one embodiment of the description the composition according to the description can also comprise at least one other relevant parasiticidal ingredient, such as an insecticide, acaricide, parasiticide, etc.

In one embodiment of the description the other parasiticide is an endectocidal parasiticide of macrocyclic lactone type. This macrocyclic lactone type parasiticide includes but is not limited to avermectins and derivatives thereof, which includes but is not limited to abamectin, dimadectin, doramectin, emamectin, eprinomectin, ivermectin, latidectin, lepimectin, selamectin, and milbemycin and derivatives thereof including but not limited to milbemectin, moxidectin, nemadectin and milbemycin D. '

The avermectin and milbemycin series of compounds are potent anthelmintic and antiparasitic agents against a wide range of internal and external parasites. The compounds which belong to this series are either natural products or are semi-synthetic derivatives thereof. The structure of these two series of compounds are closely related and they both share a complex 16-membered macrocyclic lactone ring; however, the milbemycin do not contain the aglycone substituent in the 13-position of the lactone ring. The natural product avermectins are disclosed in U.S. Patent 4,310,519 to Albers-Schonberg, et al., and the 22, 23-dihydro avermectin compounds are disclosed in Chabala, et al., U.S. Patent 4,199,569. For a general discussion of avermectins, which include a discussion of their uses in humans and animals, see "Ivermectin and Abamectin," W.C. Campbell, ed., Springer-Verlag, New York (1989). Naturally occurring milbemycins are described in Aoki et al., U.S. Patent 3,950,360 as well as in the various references cited in "The Merck Index" 12^{th} ed., S. Budavari, Ed., Merck & Co., Inc. Whitehouse Station, New Jersey (1996). Semisynthetic derivatives of these classes of compounds are well known in the art and are described, for example, in U.S. Patent 5,077,308, U.S. Patent 4,859,657, U.S. Patent 4,963,582, U.S. Patent 4,855,317, U.S. Patent 4,871,719, U.S. Patent 4,874,749, U.S. Patent 4,427,663, U.S. Patent 4,310,519, U.S. Patent 4,199,569, U.S. Patent 5,055,596, U.S. Patent 4,973,711, U.S. Patent 4,978,677, and U.S. Patent 4,920,148. Especially preferred compounds include ivermectin, emamectin, abamectin, eprinonectin and selamectin.

Another class of compounds that may be included in the inventive formulations or methods is insect growth regulators (IGR). Compounds belonging to this group are well known to the practitioner and represent a wide range of different chemical classes. These compounds all act by interfering with the development or growth of the insect pests. Compounds with an ovicidal and/or larvicidal effect on the immature stages of various ectoparasites are already known, for example from U.S. Patent No. 5,439,924. Among these compounds described are those IGR compounds which act either by blocking the development of the immature stages (eggs and larvae) into adult stages, or by inhibiting the synthesis of chitin. Insect growth regulators are described, for example, in U.S. Patent 3,748,356; U.S. Patent 3,818,047; U.S. Patent 4,225,598; U.S. Patent 4,798,837; and U.S. Patent 4,751,225, as well as in EP 179,022 or U.K. 2,140,010. French Patent No. A-2,713,889 which generally describes an IGR combination comprising at least one compound with juvenile hormone activity and chitin synthesis inhibitors, with at least one of three N-aryldiazole compounds, in particular fipronil, to control many harmful insects belonging to very varied orders.

The effective amount of the endectocide in a dose is selected from a range consisting of between about 0.1 mg/kg and about 200 mg/kg and from about 1 to about 200 mg/kg of animal weight.

Examples of IGR which may be used in the formulation of the present invention include compounds which mimic juvenile hormones, in particular:
Azadirchtin - Agridyne
Diofenolan (Ciba Geigy)
Fenoxycarb (Ciba Geigy)
Hydroprene (Sandoz)
Kinoprene (Sandoz)
Methoprene (Sandoz)
Pyriproxyfen (Sumitomo/Mgk)
Tetrahydroazadirachtin (Agridyne)
4-chloro-2-(2-chloro-2-methylpropyl)-5-(6-iodo-3-pyridylmethoxy)pyridizin-3(2 H)-one and chitin-synthesis inhibitors, in particular:
chlorfluazuron (Ishihara Sangyo)
cyromazine (Ciba Geigy)
diflubenzuron (Solvay Duphar)
fluazuron (Ciba Geigy)
flucycloxuron (Solvay Duphar)
flufenoxuron (Cyanamid)
hexaflumuron (Dow Elanco)
lufenuron (Ciba Geigy)
tebufenozide (Rohm & Haas)
teflubenzuron (Cyanamid)
triflumuron (Bayer)
these compounds being defined by their international common name (The Pesticide Manual, 10th edition, 1994, Ed. Clive Tomlin, Great Britain).

Chitin-synthesis inhibitors also include compounds such as 1-(2,6-difluorobenzoyl)-3-(2-fluoro-4-((trifluoromethyl) phenylurea, 1-(2,6-difluorobenzoyl)-3-(2-fluoro-4-(1,1,2,2-tetrafluoroethoxy)phenylurea and 1-(2,6-difluorobenzoyl)-3-(2-fluoro-4-trifluoromethyl)phenylurea. Novaluron (Isagro, Italian company) is also an example of an IGR.

Especially preferred IGR include methoprenes, pyriproxyfens, hydroprene, cyromazine, lufenuron, 1-(2,6-difluorobenzoyl)-3-(2-fluoro-4-(trifluoromethyl)phenylurea and novaluron.

Also contemplated are, for example, nodulisporic acid or nodulisporic acid derivatives. Nodulisporic acid and nodulisporic acid derivatives are known in the art as a class of compounds that are potent endo- and ectoantiparasitic agents. These compounds are based upon three structures, A, B or C, which have the following structures: 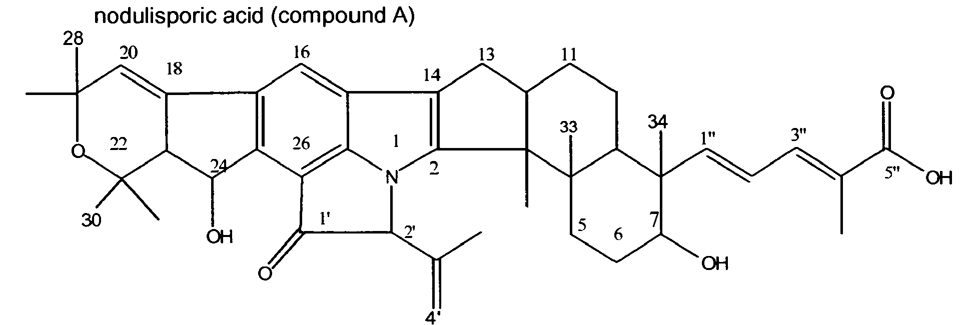 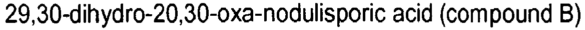 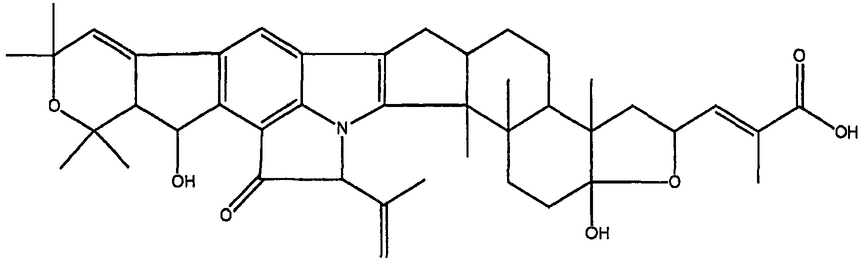 and

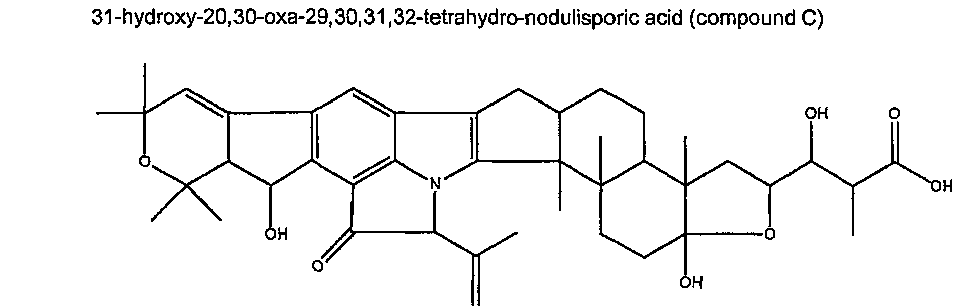

These compounds were obtained from the fermentation culture of Nodulisporium sp. MF-5954 (ATCC 74245) and the isolation and purification of the three nodulisporic acids are disclosed in US Patent 5,399,582. Derivatives of these compounds are described in WO 96/29073 and US Patent Nos. 5,945,317; 5,962,499; 5,834,260; 6,399,796; 6,221,894; 6,136,838; 5,595,991; 5,299,582; and 5,614,546.

This description includes all nodulisporic acid derivatives known in the art, including all stereoisomers, such as those described in the prior publications described above. Especially preferred are spot-on formulations comprising nordulisporic acid derivates of the formula. 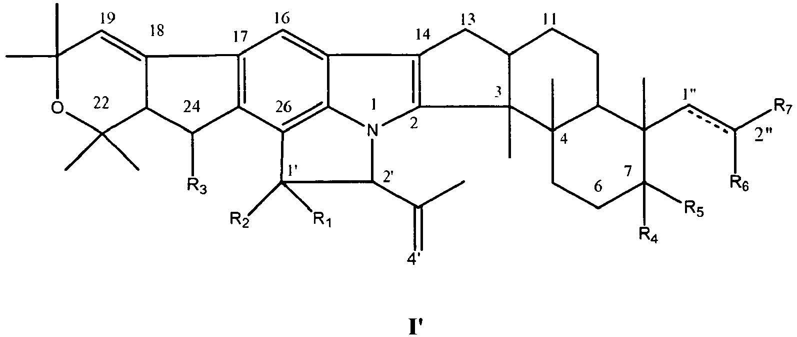 wherein
R₁ is
   (1) hydrogen,
   (2) optionally substituted alkyl,
   (3) optionally substituted alkenyl,
   (4) optionally substituted alkynyl,
   (5) optionally substituted cycloalkyl,
   (6) optionally substituted cycloalkenyl, where the substituents on the alkyl, alkenyl, alkynyl, cycloalkyl and cycloalkenyl are 1 to 3 groups independently selected from
      (i) alkyl,
      (ii) alkyl, where X is O or S(O)ₘ.
      (iii) cycloalkyl,
      (iv) hydroxy,
      (v) halogen,
      (vi) cyano,
      (vii) carboxy,
      (viii) NY¹Y², where Y¹ and Y² are independently H or alkyl,
      (ix) alkanoylamino, and
      (x) aroylamino wherein said aroyl is optionally substituted with 1 to 3 groups independently selected from R^{f}
   (7) aryl or arylalkyl, wherein said aryl is optionally substituted with 1 to 3 groups independently selected from R^{f},
   (8) perfluoroalkyl
   (9) a 5- or 6-member heterocycle containing from 1 to 4 heteroatoms independently selected from oxygen, sulfur and nitrogen atoms optionally substituted by 1 to 3 groups independently selected from hydroxy, oxo, alkyl and halogen, and which may be saturated or partly unsaturated,
R₂, R₃, and R₄ are independently OR^{a}, OCO₂R^{b}, OC(O)NR^{c}R^{d}; or
R¹ and R² represent =0, =NOR^{a} or =N-NR^{c}R^{d};
R₅ and R₆ are H; or
R₅ and R₆ together represent -0-;
R₇ is
   (1) CHO, or
   (2) the fragment 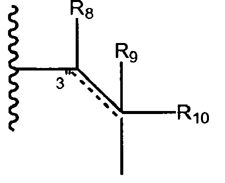
R₈ is
   (1) H,
   (2) OR^{a}, or
   (3) NR^{c}R^{d}
R₉ is
   (1) H, or
   (2) OR^{a};
R₁₀ is
   (1) CN,
   (2) C(O)OR^{b},
   (3) C(O)N(OR^{b})R^{c},
   (4) C(O)NR^{c}R^{d},
   (5) NHC(O)OR^{b},
   (6) NHC(O)NRCR^{d},
   (7) CH₂OR^{a},
   (8) CH₂OCO₂R^{b},
   (9) CH₂OC(O)NR^{c}R^{d},
   (10) C(O)NR^{c}NR^{c}R^{d}, or
   (11) C(O)NR^{c}SO₂R^{b}; ------ represents a single or a double bond;
R^{a} is
   (1) hydrogen,
   (2) optionally substituted alkyl,
   (3) optionally substituted alkenyl,
   (4) optionally substituted alkynyl,
   (5) optionally substituted alkanoyl,
   (6) optionally substituted alkenoyl,
   (7) optionally substituted alkynoyl,
   (8) optionally substituted aroyl,
   (9) optionally substituted aryl,
   (10) optionally substituted cycloalkanoyl,
   (11) optionally substituted cycloalkenoyl,
   (12) optionally substituted alkylsulfonyl
   (13) optionally substituted cycloalkyl
   (14) optionally substituted cycloalkenyl where the substituents on the alkyl, alkenyl, alkynyl, alkanoyl, alkenoyl, alkynoyl, aroyl, aryl, cycloalkanoyl, cycloalkenoyl, alkylsulfonyl, cycloalkyl and cycloalkenyl are from 1 to 10 groups independently selected from hydroxy, alkoxy, cycloalkyl, arylalkoxy, NR^{g}R^{h}, CO₂R_{b}, CONR^{c}R^{d} and halogen,
   (15) perfluoroalkyl,
   (16) arylsulfonyl optionally substituted with 1 to 3 groups independently selected from alkyl, perfluoroalkyl, nitro, halogen and cyano,
   (17) a 5- or 6-member heterocycle containing 1 to 4 heteroatoms selected from oxygen, sulfur and nitrogen optionally substituted by 1 to 4 groups independently selected from alkyl, alkenyl, perfluoroalkyl, amino, C(O)NR^{c}R^{d}, cyano, CO₂R^{b} and halogen, and which may be saturated or partly unsaturated;
R^{b} is
   (1) H,
   (2) optionally substituted aryl,
   (3) optionally substituted alkyl,
   (4) optionally substituted alkenyl,
   (5) optionally substituted alkynyl,
   (6) optionally substituted cycloalkyl,
   (7) optionally substituted cycloalkenyl, or
   (8) optionally substituted
      heterocycle containing from 1 to 4 heteroatoms independently selected from oxygen, sulfur and nitrogen; where the substituents on the aryl, alkyl, alkenyl, cycloalkyl, cycloalkenyl, heterocycle, or alkynyl are from 1 to 10 groups
      independently selected from
      (i) hydroxy,
      (ii) alkyl,
      (iii) oxo,
      (iv) SO₂NR^{g}R^{h},
      (v) arylalkoxy,
      (vi) hydroxyalkyl,
      (vii) alkoxy,
      (viii) hydroxyalkoxy,
      (ix) aminoalkoxy,
      (x) cyano,
      (xi) mercapto,
      (xii) alkyl-S(O)ₘ,
      (xiii) cycloalkyl optionally substituted with 1 to 4 groups independently selected from R^{e},
      (xiv) cycloalkenyl,
      (xv) halogen,
      (xvi) alkanoyloxy,
      (xvii) C(O)NR^{g}R^{h},
      (xviii) CO₂Rⁱ,
      (xix) formyl,
      (xx) -NR^{g}R^{h},
      (xxi) 5-to 9-member heterocycle, which may be saturated or partially unsaturated, containing from 1 to 4 heteroatoms independently selected from oxygen, sulfur and nitrogen, and optionally substituted with 1 to 5 groups independently selected from R^{e},
      (xxii) optionally substituted aryl, wherein the aryl substituents are 1,2-methylenedioxy or 1 to 5 groups independently selected from R^{e},
      (xxiii) optionally substituted arylalkoxy, wherein the aryl substituents are 1,2-methylenedioxy or 1 to 5 groups independently selected from R^{e}, and
      (xxiv) perfluoroalkyl;
R^{c} and R^{d} are independently selected from R^{b}; or
R^{c} and R^{d} together with the N to which they are attached form a 3- to 10-member ring containing 0 to 2 additional heteroatoms selected from O, S(O)ₘ, and N, optionally substituted with 1 to 3 groups independently selected from R^{g}, hydroxy, thioxo and oxo;
R^{e} is
   (1) halogen,
   (2) alkyl,
   (3) perfluoroalkyl,
   (4) -S(O)ₘRⁱ,
   (5) cyano,
   (6) nitro,
   (7) R₁₀(CH₂)v-,
   (8) RⁱCO₂(CH₂)v-,
   (9) RⁱOCO(CH₂)v-,
   (10) optionally substituted aryl where the substituents are from 1 to 3 of halogen, alkyl, alkoxy, or hydroxy,
   (11) SO₂NR^{g}R^{h}, or
   (12) amino;
R^{f} is
   (1) alkyl,
   (2) X-C₁-C₄ alkyl, where X is O or S(O)ₘ,
   (3) alkenyl,
   (4) alkynyl,
   (5) perfluoroalkyl,
   (6) NY¹Y², where Y¹ and Y² are independently H or alkyl,
   (7) hydroxy,
   (8) halogen, and
   (9) alkanoylamino,
R^{g} and R^{h} are independently
   (1) hydrogen,
   (2) alkyl optionally substituted with hydroxy, amino, or CO₂R^{l}
   (3) aryl optionally substituted with halogen, 1,2-methylenedioxy, alkoxy, alkyl or perfluoroalkyl,
   (4) arylalkyl, wherein the aryl is optionally substituted with perfluorolkyl or 1,2-methylenedioxy;
   (5) alkoxycarbonyl,
   (6) alkanoyl,
   (7) alkanoylalkyl,
   (9) aryl alkoxycarbonyl,
   (10) aminocarbonyl,
   (11) monoalkylaminocarbonyl
   (12) dialkylaminocarbonyl; or
R^{g} and R^{h} together with the N to which they are attached form a 3- to 7-member ring containing 0 to 2 additional heteroatoms selected from O, S(O)ₘ, and N, optionally substituted with 1 to 3 groups independently selected from R^{e} and oxo;
R^{l} is
   (1) hydrogen,
   (2) perfluoroalkyl,
   (3) alkyl,
   (4) optionally substituted aryl or arylalkyl, where the aryl substituents are from 1 to 3 groups independently selected from halogen, alkyl, alkoxy, and hydroxy;
m is 0 to 2; and
v is 0 to 3; or
a pharmaceutically acceptable salt thereof.

In a preferred embodiment, the present description provides formulations comprising compounds of Formula I' 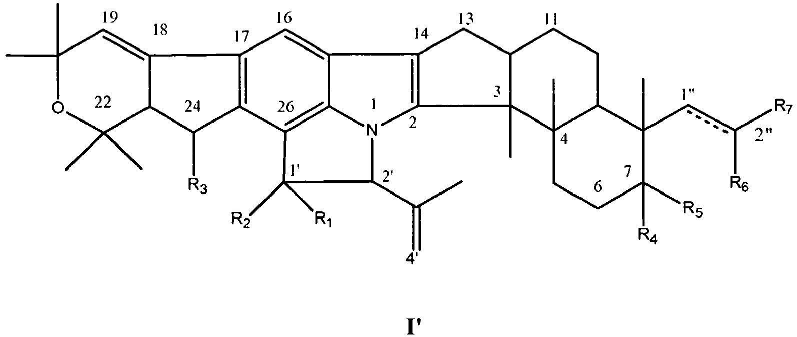 wherein
R₁ is
   (1) hydrogen,
   (2) optionally substituted alkyl,
   (3) optionally substituted alkenyl,
   (4) optionally substituted alkynyl,
   (5) optionally substituted cycloalkyl,
   (6) optionally substituted cycloalkenyl where the substituents on the alkyl, alkenyl, alkynyl, cycloalkyl and cycloalkenyl are 1 to 3 groups independently selected from
      (i) alkyl,
      (ii) X-C₁-C₆ alkyl, where X is O or S(O)ₘ,
      (iii) cycloalkyl,
      (iv) hydroxy,
      (v) halogen,
      (vi) cyano,
      (vii) carboxy, and
      (viii) NY¹Y², where Y¹ and Y² are independently H or alkyl,
   (7) aryl or arylalkyl wherein said aryl is optionally substituted with 1 to 3 groups independently selected from R^{f},
   (8) perfluoroalkyl,
   (9) a 5- or 6-member heterocycle containing from 1 to 4 heteroatoms independently selected from oxygen, sulfur and nitrogen atoms optionally substituted by 1 to 3 groups independently selected from hydroxy, oxo, alkyl and halogen, and which may be saturated or partly unsaturated,
R₈ is
   (1) H,
   (2) OH, or
   (3) NH₂;
R₉ is
   (1) H or
   (2) OH;
R₁₀ is
   (1) C(O)OR^{b},
   (2) C(O)N(OR^{b})R^{c},
   (3) C(O)NR^{c}R^{d},
   (4) NHC(O)OR^{b},
   (5) NHC(O)NR^{c}R^{d},
   (6) CH₂OR^{a},
   (7) CH₂OCO₂R^{b},
   (8) CH₂OC(O)NR^{c}R^{d},
   (9) C(O)NR^{c}NR^{c}R^{d}, or
   (10) C(O)NR^{c}SO₂R^{b};
R^{a} is
   (1) hydrogen,
   (2) optionally alkyl,
   (3) optionally substituted alkenyl,
   (4) optionally substituted alkynyl,
   (5) optionally substituted alkanoyl,
   (6) optionally substituted alkenoyl,
   (7) optionally substituted alkynoyl,
   (8) optionally substituted aroyl,
   (9) optionally substituted aryl,
   (10) optionally substituted cycloalkanoyl,
   (11) optionally substituted cycloalkenoyl,
   (12) optionally substituted alkylsulfonyl
   (13) optionally substituted cycloalkyl
   (14) optionally substituted cycloalkenyl where the substituents on the alkyl, alkenyl, alkynyl, alkanoyl, alkenoyl, alkynoyl, aroyl, aryl, cycloalkanoyl, cycloalkenoyl, alkylsulfonyl, cycloalkyl and cycloalkenyl are from 1 to 10 groups independently selected from hydroxy, alkoxy, cycloalkyl, aryl alkoxy, NR^{g}R^{h}, CO₂R_{b}, CONR^{c}R^{d} and halogen,
   (15) perfluoroalkyl,
   (16) arylsulfonyl optionally substituted with 1 to 3 groups independently selected from alkyl, perfluoroalkyl, halogen and cyano,
   (17) a 5- or 6-member heterocycle containing 1 to 4 heteroatoms selected from oxygen, sulfur and nitrogen optionally substituted by 1 to 4 groups independently selected from alkyl, alkenyl, perfluoroalkyl, amino, C(O)NR^{c}R^{d}, cyano, CO₂R^{b} and halogen, and which may be saturated or partly unsaturated;
R^{b} is
   (1) H,
   (2) optionally substituted aryl,
   (3) optionally substituted alkyl,
   (4) optionally substituted alkenyl,
   (5) optionally substituted alkynyl,
   (6) optionally substituted cycloalkyl,
   (7) optionally substituted cycloalkenyl, or
   (8) optionally substituted 5- to 10-member heterocycle containing from 1 to 4 heteroatoms independently selected from oxygen, sulfur and nitrogen; where the substituents on the aryl, alkyl, alkenyl, cycloalkyl, cycloalkenyl, heterocycle, or alkynyl are from 1 to 10 groups
      independently selected from
      (i) hydroxy,
      (ii) C₁-C₃ alkyl,
      (iii) oxo,
      (iv) SO₂NR^{g}R^{h},
      (v) aryl alkoxy,
      (vi) hydroxy alkyl,
      (vii) alkoxy,
      (viii) hydroxyalkoxy,
      (ix) aminoalkoxy,
      (x) cyano,
      (xi) perfluoroalkyl,
      (xii) alkyl-S(O)ₘ,
      (xiii) cycloalkyl optionally substituted with 1 to 4 groups independently selected from R^{e},
      (xiv) cycloalkenyl,
      (xv) halogen,
      (xvi) alkanoyloxy,
      (xvii) C(O)NR^{g}R^{h},
      (xviii) CO₂Rⁱ,
      (xix) optionally substituted arylalkoxy,
         wherein the aryl substituents are 1,2-methylenedioxy or 1 to 5 groups independently selected from R^{e},
      (xx) -NR^{g}R^{h},
      (xxi) 5 to 6-member heterocycle, which may be saturated or partially unsaturated, containing from 1 to 4 heteroatoms independently selected from oxygen, sulfur and nitrogen, and optionally substituted with 1 to 5 groups independently selected from R^{e}, and
      (xxii) optionally substituted aryl, wherein the aryl substituents are 1,2-methylenedioxy or 1 to 5 groups independently selected from R^{e};
R^{e} is
   (1) halogen,
   (2) alkyl,
   (3) perfluoroalkyl,
   (4) -S(O)ₘRⁱ,
   (5) cyano,
   (6) amino,
   (7) RⁱO(CH₂)ᵥ-,
   (8) RⁱCO₂(CH₂)ᵥ-,
   (9) RⁱOCO(CH₂)ᵥ-,
   (10) optionally substituted aryl where the substituents are from 1 to 3 of halogen, alkyl, alkoxy, or hydroxy, or
   (11) SO₂NR^{g}R^{h};
R^{f} is
   (1) methyl,
   (2) X-C1-C2 alkyl, where X is O or S(O)ₘ,
   (3) halogen,
   (4) acetylamino,
   (5) trifluoromethyl,
   (6) NY¹Y², where Y¹ and Y² are independently H or methyl, and
   (7) hydroxy;
R^{g} and R^{h} are independently
   (1) hydrogen,
   (2) alkyl optionally substituted with hydroxy, amino, or CO₂Rⁱ
   (3) aryl optionally substituted with halogen, 1,2-methylenedioxy, alkoxy, alkyl or perfluoroalkyl,
   (4) arylalkyl, wherein the aryl is optionally substituted with perfluorolkyl or 1,2-methylenedioxy;
   (5) alkoxycarbonyl,
   (6) alkanoyl,
   (7) alkanoyl alkyl,
   (9) arylalkoxycarbonyl,
   (10) aminocarbonyl,
   (11) monoalkylaminocarbonyl
   (12) dialkylaminocarbonyl; or
R^{g} and R^{h} together with the N to which they are attached form a 5- to 6-member ring containing 0 to 2 additional heteroatoms selected from O, S(O)ₘ, and N, optionally substituted with 1 to 3 groups independently selected from R^{e} and oxo;
Rⁱ is
   (1) hydrogen,
   (2) perfluoroalkyl,
   (3) alkyl,
   (4) optionally substituted arylalkyl, where the aryl substituents are from 1 to 3 groups independently selected from halogen, alkyl, alkoxy, and hydroxyl.

In another preferred embodiment, the present description provides compositions comprising compounds of Formula I' 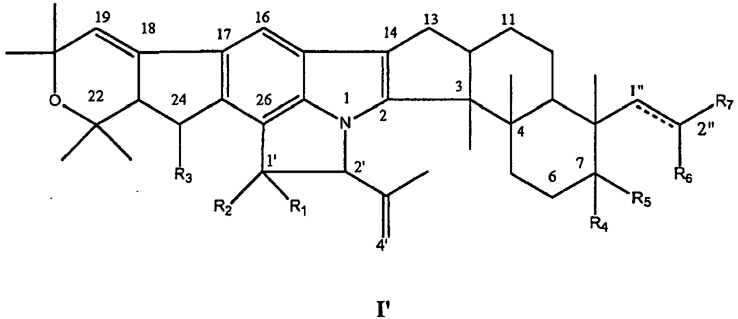 wherein
Rⁱ is
   (1) hydrogen,
   (2) optionally substituted alkyl,
   (3) optionally substituted alkenyl,
   (4) optionally substituted alkynyl, where the substituents on the alkyl, alkenyl, and alkynyl are 1 to 3 groups independently selected from
      (i) methyl,
      (ii) X-methyl, where X is O or S(O)ₘ and
      (iii) halogen,
   (5) aryl or arylalkyl wherein said aryl is optionally substituted with 1 to 3 groups independently selected from R^{f}.
   (6) trifluoromethyl
R₈ is
   (1) H,
   (2) OH, or
   (3) NH₂
R₉ is
   (1) H, or
   (2) OH;
R₁₀ is
   (1) C(O)OR^{b},
   (2) C(O)N(OR^{b})R^{c},
   (3) C(O)NR^{c}R^{d},
   (4) NHC(O)OR^{b},
   (5) NHC(O)NR^{c}R^{d},
   (6) CH₂OR^{a},
   (7) CH₂OCO₂R^{b},
   (8) CH₂OC(O)NR^{c}R^{d},
   (9) C(O)NR^{c}NR^{c}R^{d}, or
   (10) C(O)NR^{c}SO₂R^{b};
R^{a} is
   (1) hydrogen,
   (2) optionally substituted alkyl,
   (3) optionally substituted alkenyl,
   (4) optionally substituted alkynyl,
   (5) optionally substituted alkanoyl,
   (6) optionally substituted aroyl,
   (7) optionally substituted cycloalkanoyl,
   (8) optionally substituted cycloalkenoyl,
   (9) optionally substituted alkylsulfonyl where the substituents on the alkyl, alkenyl, alkynyl, alkanoyl, aroyl, cycloalkanoyl, cycloalkenoyl, and alkylsulfonyl, are from 1 to 5 groups independently selected from hydroxy, alkoxy, aryl alkoxy, NR^{g}R^{h}, CO₂R_{b}, CONR^{c}R^{d} and halogen,
   (10) trifluoromethyl,
   (11) arylsulfonyl optionally substituted with 1 to 3 groups independently selected from methyl, trifluoromethyl and halogen,
   (12) a 5- or 6-member heterocycle containing 1 to 4 heteroatoms selected from oxygen, sulfur and nitrogen optionally substituted by 1 to 4 groups independently selected from methyl, trifluoromethyl, C(O)NR^{c}R^{d}, CO₂R^{b}
      and halogen, and which may be saturated or partly unsaturated;
R_{b} is
   (1) H,
   (2) optionally substituted aryl,
   (3) optionally substituted alkyl,
   (4) optionally substituted alkenyl,
   (5) optionally substituted alkynyl,
   (6) optionally substituted cycloalkyl,
   (7) optionally substituted cycloalkenyl, or
   (8) optionally substituted 5- to 6-member heterocycle containing from 1 to 4 heteroatoms independently selected from oxygen, sulfur and nitrogen; where the substituents on the aryl, alkyl, alkenyl, cycloalkyl, cycloalkenyl, heterocycle, or alkynyl are from 1 to 10 groups independently selected from
      (i) hydroxy,
      (ii) alkyl,
      (iii) oxo,
      (iv) SO₂NR^{g}R^{h},
      (v) arylalkoxy,
      (vi) hydroxyalkyl,
      (vii) alkoxy,
      (viii) hydroxy alkoxy,
      (ix) amino alkoxy,
      (x) cyano,
      (xi) alkyl-S(O)ₘ,
      (xii) cycloalkyl optionally substituted with 1 to 4 groups independently selected from R^{e},
      (xiii) cycloalkenyl,
      (xiv) halogen,
      (xv) alkanoyloxy,
      (xvi) C(O)NR^{g}R^{h},
      (xvii) CO₂Rⁱ,
      (xvii) -NR^{g}R^{h},
      (xix) 5 to 6-member heterocycle, which may be saturated or partially unsaturated, containing from 1 to 4 heteroatoms independently selected from oxygen, sulfur and nitrogen, and optionally substituted with 1 to 5 groups independently selected from R^{e},
      (xx) optionally substituted aryl, wherein the aryl substituents are 1,2-methylenedioxy or 1 to 5 groups independently selected from R^{e},
      (xxi) optionally substituted aryl alkoxy, wherein the aryl substituents are 1,2-methylenedioxy or 1 to 5 groups independently selected from R^{e}, and
      (xxii) perfluoroalkyl;
R^{e} is
   (1) halogen,
   (2) alkyl,
   (3) perfluoroalkyl,
   (4) -S(O)ₘRⁱ,
   (5) cyano,
   (6) RⁱO(CH₂)ᵥ-,
   (7) RⁱCO2(CH₂)ᵥ-,
   (8) R₁₀CO(CH₂)ᵥ-,
   (9) optionally substituted aryl where the substituents are from 1 to 3 of halogen, alkyl, alkoxy, or hydroxy,
   (10) SO₂NR^{g}R^{h}, or
   (11) amino;
R^{f} is
   (1) methyl,
   (2) X-C₁-C₂ alkyl, where X is O or S(O)ₘ,
   (3) trifluoromethyl,
   (4) NY¹Y², where Y¹ and Y² are independently H or methyl,
   (5) hydroxy,
   (6) halogen, and
   (7) acetylamino,
R^{g} and R^{h} are independently
   (1) hydrogen,
   (2) alkyl optionally substituted with hydroxy, amino, or CO₂Rⁱ
   (3) aryl optionally substituted with halogen, 1,2-methylenedioxy, alkoxy, alkyl or perfluoroalkyl,
   (4) arylalkyl, wherein the aryl is optionally substituted with perfluorolkyl or 1,2-methylenedioxy;
   (5) alkoxycarbonyl,
   (6) alkanoyl,
   (7) alkanoylalkyl,
   (9) arylalkoxycarbonyl,
   (10) aminocarbonyl,
   (11) monoalkylaminocarbonyl
   (12) dialkylaminocarbonyl; or
R^{g} and R^{h} together with the N to which they are attached form a 5- to 6-membered ring containing 0 to 2 additional heteroatoms selected from O, S(O)ₘ, and N, optionally substituted with 1 to 3 groups independently selected from R^{e} and oxo;
Rⁱ is
   (1) hydrogen,
   (2) perfluoroalkyl,
   (3) alkyl,
(4) optionally substituted aryl or arylalkyl, where the aryl substituents are from 1 to 3 groups independently selected from halogen, alkyl, alkoxy, and hydroxy; and all other variables are as defined under Formula I' above. Most especially preferred are formulations, wherein the composition comprises nodulisporic acid derivatives which are nodulisporamides, which are compounds of the formula 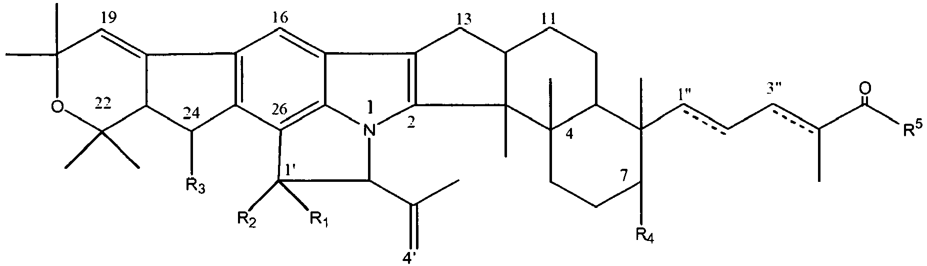 Wherein R₁ is
   (1) hydrogen,
   (2) optionally substituted C₁-C₁₀ alkyl,
   (3) optionally substituted C₂-C₁₀ alkenyl,
   (4) optionally substituted C₂-C₁₀ alkynyl,
   (5) optionally substituted C₃-C₈ cycloalkyl,
   (6) optionally substituted C₅-C₈ cycloalkenyl where the substituents on the alkyl, alkenyl, alkynyl, cycloalkyl and cycloalkenyl are 1 to 3 groups independently selected from C₁-C₅ alkyl, C₁-C₁₀ alkoxy, C₁-C₁₀ alkylthio, C₁- C₁₀ alkylsulfonyl, C₃-C₈ cycloalkyl, hydroxy, halogen, cyano, carboxy, amino, C₁-C₁₀ monoalkylamino, C₁-C₁₀ dialkylamino, C₁-C₁₀ alkanoyl amino and benzoyl amino wherein said benzoyl is optionally substituted with 1 to 3 groups independently selected from C₁-C₄ alkyl, C₁-C₄ alkoxy, C₁-C₄ alkylthio, C₂-C₄ alkenyl, C₂-C₄ alkynyl, C₁-C₃- perfluoroalkyl, amino, hydroxy, halogen, C₁-C₅ monoalkylamino, C₁-C₅ dialkylamino and C₁-C₅ alkanoyl amino,
   (7) phenyl C₀-C₅ alkyl wherein said phenyl is optionally substituted with 1 to 3 groups independently selected from C₁-C₄ alkyl, C₁-C₄ alkoxy, C₁-C₄ alkylthio, C₂-C₄ alkenyl, C₂-C₄ alkynyl, C₁-C₃- perfluoroalkyl, amino, hydroxy, carboxy, halogen, C₁-C₅ monoalkylamino, C₁-C₅ dialkylamino and C₁-C₅ alkanoyl amino,
   (8) C₁-C₅ perfluoroalkyl,
   (9) a 5- or 6-member ring selected from morpholino, pyridyl and piperazino, optionally substituted by 1 to 3 groups independently selected from hydroxy, oxo, C₁-C₁₀ alkyl and halogen,
R², R³, and R⁴ are independently OR^{a}, OCO₂R^{b}, OC(O)NR^{c}R^{d}; or
R¹ and R² together represent =O, =NOR^{a} or =N-NR^{c}R^{d};
R⁵ is NR^{c}R^{d},
R^{a} is
   (1) hydrogen,
   (2) optionally substituted C₁-C₁₀ alkyl,
   (3) optionally substituted C₃-C₁₀ alkenyl,
   (4) optionally substituted C₃-C₁₀ alkynyl,
   (5) optionally substituted C₁-C₁₀ alkanoyl,
   (6) optionally substituted C₁-C₁₀ alkenoyl,
   (7) optionally substituted C₁-C₁₀ alkynoyl,
   (8) optionally substituted benzoyl,
   (9) optionally substituted phenyl,
   (10) optionally substituted C₁-C₇ cycloalkanoyl,
   (11) optionally substituted C₄-C₇ cycloalkenoyl,
   (12) optionally substituted C₁-C₁₀ alkylsulfonyl
   (13) optionally substituted C₃-C₈ cycloalkyl
   (14) optionally substituted C₅-C₈ cycloalkenyl
      where the substituents on the alkyl, alkenyl, alkynyl, alkanoyl, alkenoyl, alkynoyl, benzoyl, phenyl, cycloalkanoyl, cycloalkenoyl, alkylsulfonyl, cycloalkyl and cycloalkenyl are from 1 to 5 groups independently selected from hydroxy, C₁-C₆ alkoxy, C₃-C₇ cycloalkyl, aryl C₁-C₃ alkoxy, NR^{g} R^{h}, CO₂R^{b}, CONR^{c} R^{d} and halogen,
   (15) C₁-C₅ perfluoroalkyl,
   (16) phenylsulfonyl optionally substituted with 1 to 3 groups independently selected from C₁-C₅ alkyl, C₁-C₅ perfluoroalkyl, nitro, halogen or cyano,
   (17) a 5- or 6-member ring selected from piperidino, morpholino, pyridyl and piperazino optionally substituted by 1 to 4 groups independently selected from C₁-C₅ alkyl, C₁-C₅ alkenyl, C₁-C₅ perfluoroalkyl, amino, C(O)R^{c} R^{d}, cyano, CO₂R^{b} or halogen;
R^{b} is
   (1) H,
   (2) optionally substituted phenyl,
   (3) optionally substituted C₁-C₁₀ alkyl,
   (4) optionally substituted C₃-C₁₀ alkenyl, or
   (5) optionally substituted C₃-C₁₀ alkynyl,
   where the substituents on the phenyl, alkyl, alkenyl or alkynyl are from 1 to 5 groups
   independently selected from hydroxy, C₁-C₆ alkoxy, C₃-C₇ cycloalkyl, halogen, C₁-C₅ alkanoyloxy, C(O)NR^{c}R^{d}, CO₂R^{b}, formyl, -NR^{g} R^{h}, optionally substituted phenyl, and optionally substituted phenyl C₁-C₃ alkoxy, wherein the phenyl substituents are 1 to 3 groups independently selected from R^{e} ;
R^{c} and R^{d} are independently R^{b} ; or
R^{c} and R^{d} together with the N to which they are attached form a piperidino, morpholino or piperazino optionally substituted with 1 to 3 groups independently selected from R^{g} and oxo;
R^{e} is
   (1) halogen,
   (2) C₁-C₇ alkyl,
   (3) C₁-C₃ perfluoroalkyl,
   (4) -S(O)ₘRⁱ,
   (5) cyano,
   (6) nitro,
   (7) R^{j}O(CH₂)ᵥ-,
   (8) R^{j}CO₂ (CH₂)ᵥ,
   (9) R^{j}OCO(CH₂)ᵥ-,
   (10) optionally substituted phenyl where the substituents are from 1 to 3 halogen, C₁-C₆ alkyl, C₁-C₆alkoxy, or hydroxy;
v is 0 to 3;
R^{g} and R^{h} are independently
   (1) hydrogen,
   (2) C₁-C₆ alkyl,
   (3) aryl,
   (4) aryl C₁-C₆ alkyl,
   (5) C₁-C₅ alkoxycarbonyl,
   (6) C₁-C₅ alkylcarbonyl, or
   (7) C₁-C₅ alkanoyl C₁-C₅ alkyl; or
R^{g} and R^{h} together with the N to which they are attached form a piperidino, morpholino or piperazino optionally substituted with 1 to 3 groups independently selected from R^{g} and oxo;
Rⁱ and R^{j} are independently
   (1) hydrogen,
   (2) C₁-C₃ perfluoroalkyl,
   (3) optionally substituted C₁-C₆ alkyl, where the substituents are aryl or substituted phenyl;
   (4) phenyl or substituted phenyl where the substituents are from 1 to 3 groups independently selected from halogen, C₁-C₆ alkyl, C₁-C₆ alkoxy, or hydroxy;
m is 0 to 2; or a pharmaceutically acceptable salt thereof. Most especially preferred are compositions comprising compounds of the formula 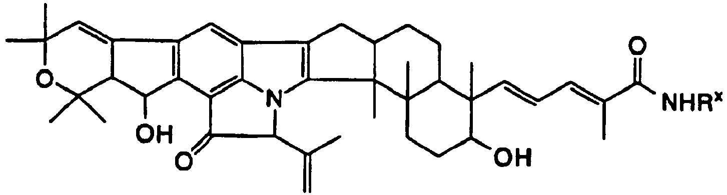 wherein Rx is selected from the group consisting of: H, CH₃, CH₂CH₃, C(CH₃)₃, CH₂CH₂CH₃, CH₂CH₂OH, CH(CO₂CH₃)CH₂OH, CH₂CO₂CH₃, CH₂CH(OCH₂CH₃)₂, CH₂CH₂OCH₂CH₂OH, CH(CH₃)(CH₂)₃C(CH₃)₂OH, (CH₂)₃OH, (CH₂)₄OH, (CH₂)SOH, CH(CH₂OH)CH₂CH₃, NHC(CH₃)₃, CH₂CN, (CH₂)₆OH, CH₂CH(OH)CH₃, CH(CH₂OH)CH₂CH₂CH₃, CH₂CH₂SCH₃, CH₂CH₂SCH₂CH₃, CH₂CONH, CH(CH₃)(CH₂OH)₂, CH₂CH₂NHCH₂CH₂OH, CH(CH₂OH)(CH₂)₃CH₃, CH(CH₂OCH₃)CH₃, (CH₂)₂SH, (CH₂)₄NH₂, CH₂CH₂SO₂CH₃, CH₂CH₂S(O)CH₃, CH(CH(CH₃)₂)CH₂OH, (CH₂)₃NH₂, (CH₂)₃N(CH₂CH₃)₂, (CH₂)₃N(CH₃)₂, OCH₂CH₃, CH₂CH(OH)CH₂OH, OCH₃, CH₂CH₂OCH₃, CH₂CH₂NHC(O)CH₃, C(CH₃)₂CH₂OH, c-C₃H₅, c-C₆H₁₁, (CH₂)₃OCH₂CH₃, CH₂CH≡CH₂, C(CH₂CH₃)(CH₂OH)₂, CH₂C≡CH, CH₂CO₂CH₂CH₃, CH₂CH₂F, (CH₂)₃OCH₂)₁₁ CH₃, CH₂CH₂N(CH₃)₂, CH₂CH₂OCH₂CH₂NH₂, CH₂CF₃, NHCH₂CO₂CH₂CH₃, CH(CH₃)CO₂CH₃, C(CH₃)₂CH₂C(O)CH₃, CH(CO₂CH₂CH₃)₂, CH₂CH₃, CH(CH₂CH₂CH₃)CO₂CH₃, CH₂CH₂CH₂OCH₃, C(CH₃)₂C≡CH, (CH₂)₄CH₃, CH(CH₂CH₂CH₃)₂, (CH₂)₅CH₃,CH₂CH₂CO₂H, CH(CH(CH₃)₂)CO₂CH₃, OCH₂CO₂H, CH(CH(CH₃)₂)CH₂OH, CH(CH(CH₃)₂)CH₂OH, CH(CH₃)CH₂OH, CH(CH₃)CH₂OH, CH(CH₃)₂, C(CH₃)₃, (CH₂)CH(CH₃)₂, CH(CH₃)CH₂CH₃, CH₂CH(CH₃)OH, (CH₂)₃CH₃, (CH₂)₂OCH₂CH₃, 1-adamantyl, (CH₂)₈CH₃, CH(CH₃)CH(CH₃)₂, (CH₂)₃NHCH₃, (CH₂)₂N(CH₂CH₃)₂,

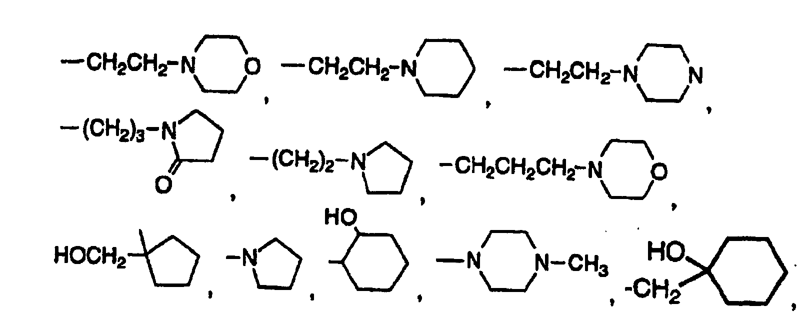

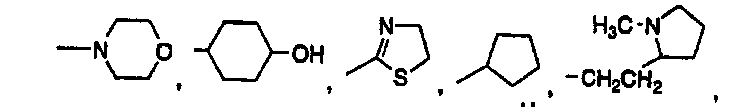

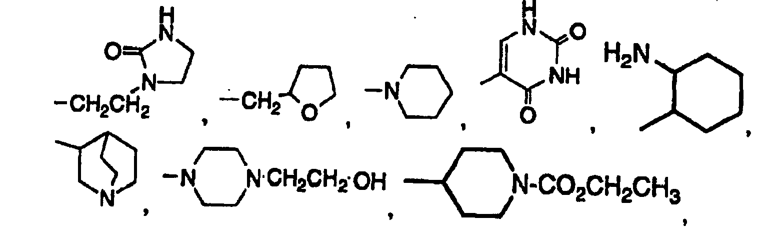

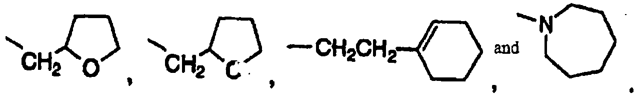

An especially preferred nodulisporamide compound for compositions for the inventive use is one where in R^{x} is with the t-butyl (or "nodulisporamide").
In another embodiment of the description the other parasiticide is an ectocidal parasiticide. In one embodiment of the ectocidal parasiticide is an arylpyrazole. Examples of suitable arylpyrazoles are those described in EP 295 117 (U.S. Patents 5232940, 5547974, 5608077, 5714191, 5916618 and 6372774).

In one embodiment of the arylpyrazole, the aryl group is attached to the pyrazole group at the 1-position of the pyrazole and the aryl group is a substituted phenyl group. In one embodiment of the substituted phenyl group, the substitution is at the 2-, 4-, and/or 6-position of the phenyl ring and the substituents are selected from the group consisting of C₁-C₄ alkyl, halogen, and C₁-C₄-haloalkyl. In one embodiment of the pyrazole group, the pyrazole is optionaly substituted at the 3-position with a moiety selected from the group consisting of cyano, nitro, halogen, acetyl or formyl; is optionally substituted at the 4-position with Z-S(O)_{q}-, wherein Z is C₁-C₄ alkyl or C₁-C₄-haloalkyl and q is 0, 1 or 2; and is optionally substituted at the 5-position with an amino group or an amino substituted with C₁-C₄ alkyl or C₁-C₄ alkanoyl groups.

In still another embodiment of the arylpyrazole compound, the compound is 5-amino-1-[2,6-dichloro-4-(trifluoromethyl)phenyl]-4-[(trifluoromethyl)sulfinyl]-1H-pyrazole-3-carbonitrile ("fipronil"), pyriprole, and pyrafluprole.

In one embodiment of the composition, the proportions by weight of parasiticide of formula I to the additional parasiticide is between about 5/1 and about 20,000/1.

In one embodiment of the composition, the parasiticide of formula I and the associated endectocidal parasiticide are to be contained in a controlled- and sustained-release preparation, such as, for example, microspheres, granules or implants. This can be obtained, for example, by mixing a controlled-release preparation of an ectoparasiticide, such as fipronil, and/or a controlled-release preparation of endectocide, such as ivermectin, in a suitable vehicle, such as water, oil or a medium-chain triglyceride.

In one embodiment of such a controlled-release preparation, the formulations are preferably drawn up so as to release between 5 and 100 mg/kg/day, for example 45 mg/kg/day, of compound of formula I, and from 0.01 to 15 mg/kg/day of ectoparasiticide, for example fipronil, or, for example, 0.5 mg/kg/day of endectocide, for example ivermectin.

In the case of such controlled-release preparations, a dose for a treatment of very long duration of an animal will preferably comprise a compound of formula I and between 1 and 20 mg/kg of fipronil or between 2 mg/kg and 3 mg/kg of endectocide, in particular of ivermectin.

Another subject of the invention is the use of the abovementioned compound corresponding to formula I for the preparation of the compositions capable of being employed in the methods according to the invention.

The compositions which can be used in the invention can comprise generally from about 0.001 to about 95% of the compound of formula I.

In an embodiment for the administration of the composition comprising the compounds of formula I, the compounds are applied in total amounts selected from ranges consisting of about 0.5 mg/kg to about 100 mg/kg per day and about 1 mg/kg to about 50 mg/kg per day.

Ready-to-use preparations contain the compounds acting against parasites such as ectoparasites, in concentrations selected from the ranges consisting of about 10 ppm to about 80 per cent by weight, from about 0.1 to about 65 per cent by weight, from about 1 to about 50 per cent by weight, and from about 5 to about 40 per cent by weight.

Preparations which are diluted before use contain the compounds acting against ectoparasites in concentrations selected from the ranges consisting of about 0.5 to about 90 per cent by weight and about 1 to about 50 per cent by weight.

Furthermore, the preparations comprise the compounds of formula I against endoparasites in concentrations in ranges selected from the group consisting of about 10 ppm to about 2 per cent by weight, about 0.005 to about 0.9 per cent by weight, and about 0.05 to about 0.25 per cent by weight.

In an embodiment for the administration of the composition comprising the compounds of formula I, the composition is applied dermally or topically.

In another embodiment of the administration of the composition comprising the compounds of formula I, the topical application is conducted in the form of compound-containing shaped articles such as collars, medallions, ear tags, bands for fixing at body parts, and adhesive strips and foils.

In another embodiment of the administration of the composition, the compositions are solid formulations which release compounds of formula I in total amounts selected from the ranges consisting of about 10 mg/kg to about 300 mg/kg, about 20 mg/kg to about 200 mg/kg, and about 25 mg/kg to about 160 mg/kg body weight of the treated animal in the course of three weeks.

For the preparation of the shaped articles, thermoplastic and flexible plastics as well as elastomers and thermoplastic elastomers are used. Suitable plastics and elastomers are polyvinyl resins, polyurethane, polyacrylate, epoxy resins, cellulose, cellulose derivatives, polyamides and polyester which are sufficiently compatible with the compounds of formula I. A detailed list of plastics and elastomers as well as preparation procedures for the shaped articles is given e.g. in WO 03/086075 (U.S. Patent Application Publication 2005-214336).

Additional guidance for making or administering the anti-parasiticidal compositions can be found in general texts known to those of skill in the art which include but are not limited to Plumb's Veterinary Drug Handbook, 5th Edition, ed. Donald C. Plumb, Blackwell Publishing, (2005) or The Merck Veterinary Manual, 9th Edition, (January 2005))

The invention is further described by the following non-limiting examples which further illustrate the invention, and are not intended, nor should they be interpreted to, limit the scope of the invention.

### EXAMPLES

### Compound examples

Examples of compounds of formula I are given in following table C:

**Table C:**

| Compound No. | Structure | Comment |
|---|---|---|
| C.I.1 | 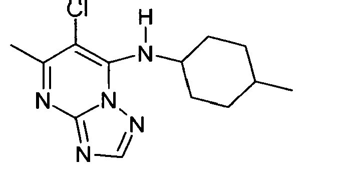 | Isomeric mixture |
| C.I.2 | 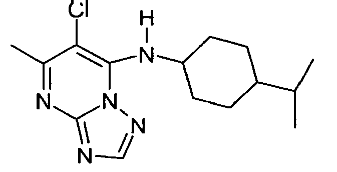 | Isomeric mixture |
| C.I.3 | 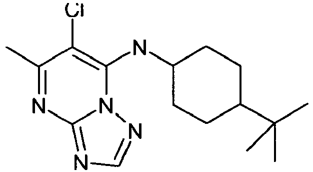 | Mixture of cis-trans-isomer |
| C.I.4 | 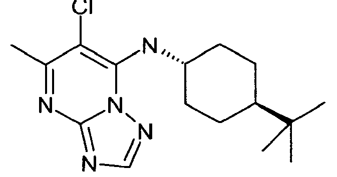 | Trans-isomer |
| C.I.5 | 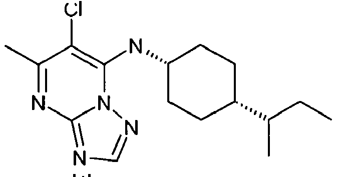 | 90% cis-isomer |
| C.I.6 | 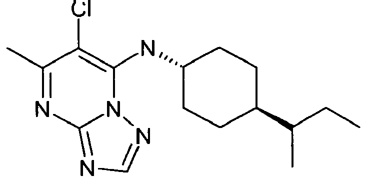 | 95% trans-isomer |
| C.I.7 | 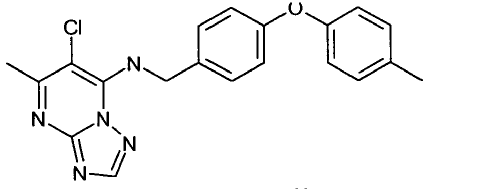 | |
| C.I.8 | 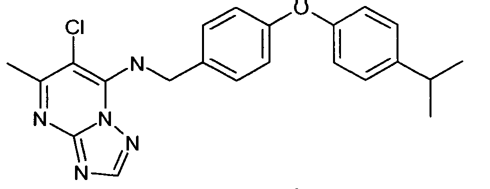 | |
| C.I.9 | 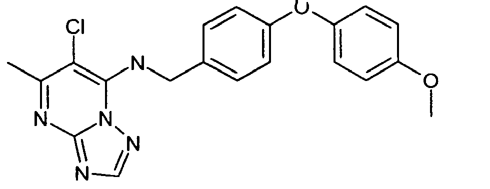 | |
| C.I.10 | 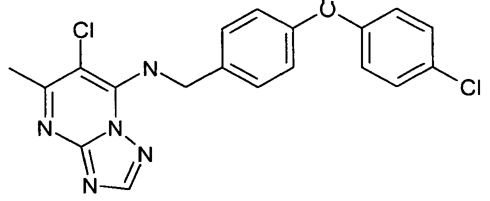 | |
| C.I.11 | 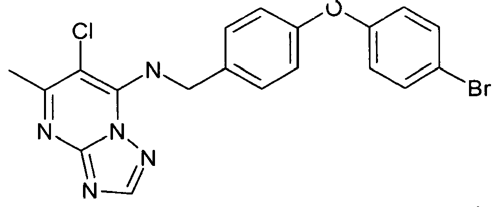 | |
| C.I.12 | 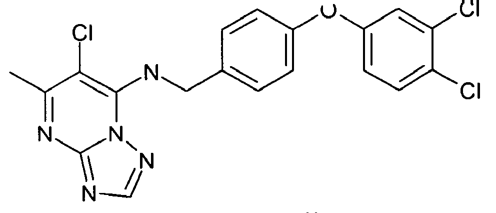 | |
| C.1.13 | 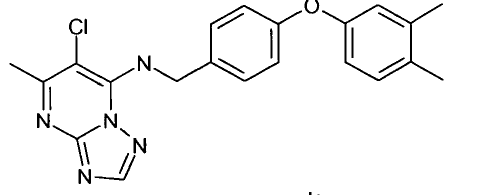 | |
| C.I.14 | 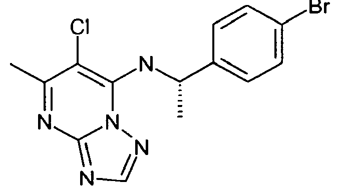 | S-enantiomer |
| C.I.15 | 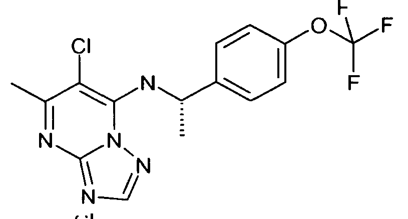 | S-enantiomer |
| C.I.16 | 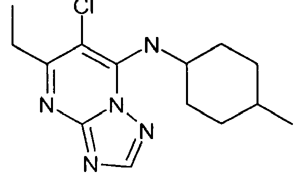 | Mixture of isomers |
| C.I.17 | 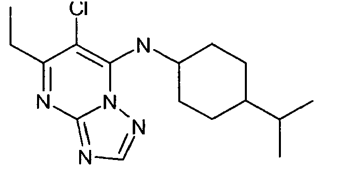 | Mixture of isomers |
| C.I.18 | 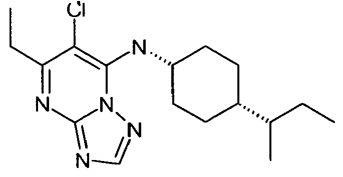 | 90% cis-isomer |
| C.I.19 | 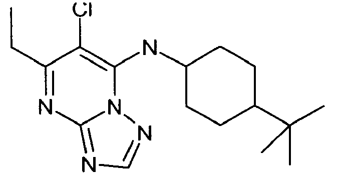 | Mixture of isomers |
| C.I.20 | 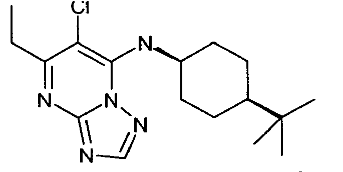 | Cis-isomer |
| C.I.21 | 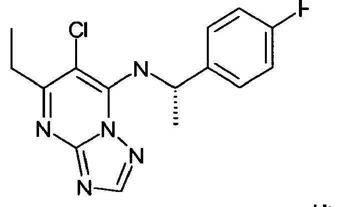 | S-enantiomer |
| C.I.22 | 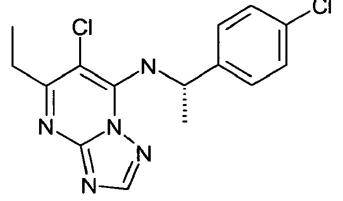 | S-enantiomer |
| C.I.23 | 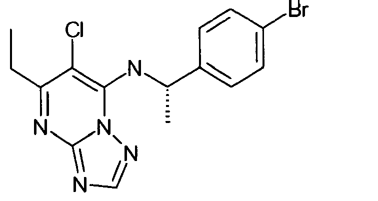 | S-enantiomer |
| C.I.24 | 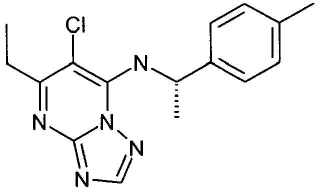 | S-enantiomer |
| C.I.25 | 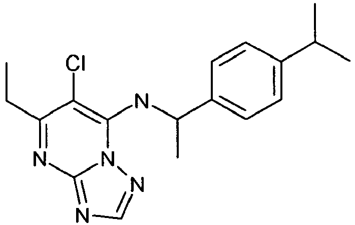 | Racemate |
| C.I.26 | 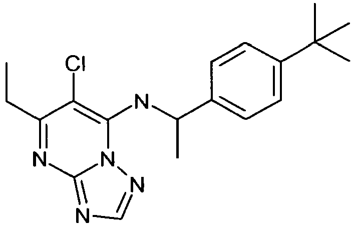 | Racemate |
| C.I.27 | 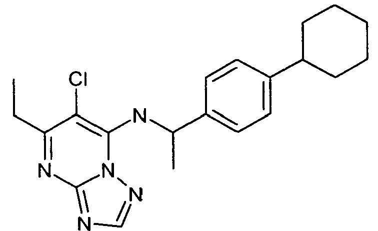 | Racemate |
| C.I.28 | 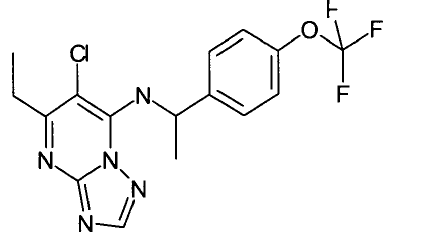 | Racemate |
| C.I.29 | 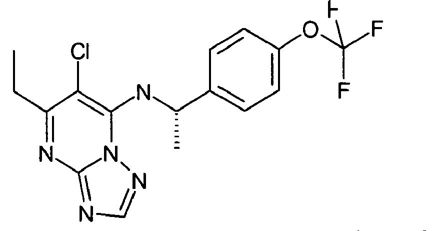 | S-enantiomer |
| C.1.30 | 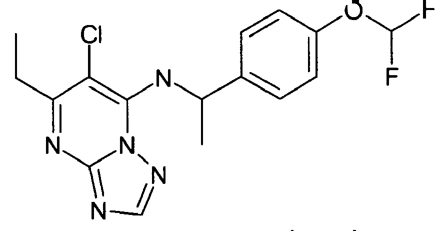 | Racemate |
| C.I.31 | 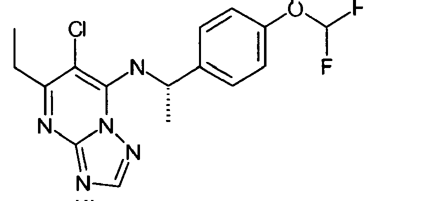 | S-enantiomer |
| C.I.32 | 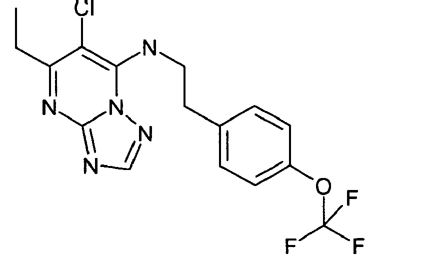 | |
| C.I.33 | 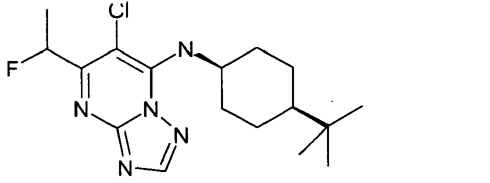 | Cis-isomer |
| C.I.34 | 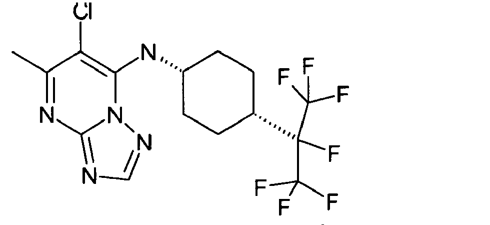 | 85% cis-isomer |
| C.I.35 | 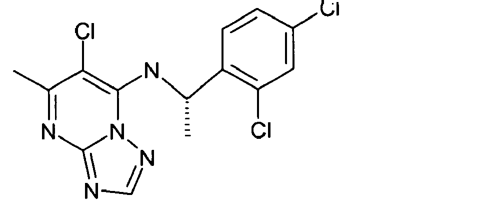 | S-enantiomer |
| C.I.36 | 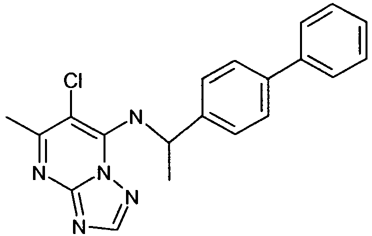 | Racemate |
| C.I.37 | 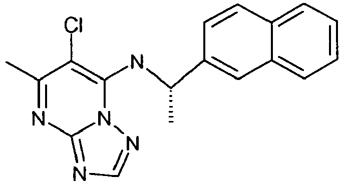 | S-enantiomer |
| C.I.38 | 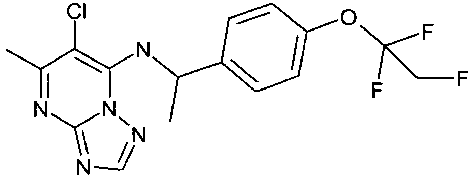 | Racemate |
| C.I.39 | 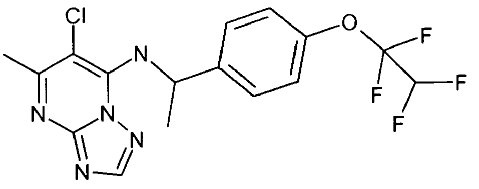 | Racemate |
| C.I.40 | 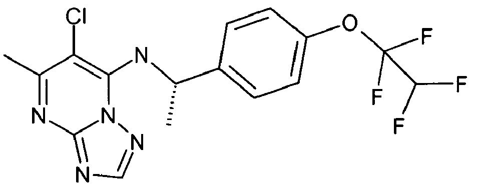 | S-enantiomer |
| C.I.41 | 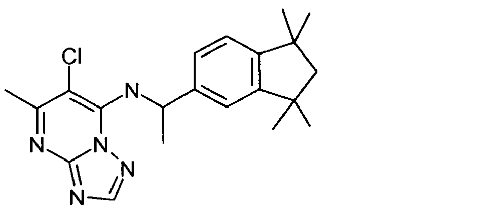 | Racemate |
| C.I.42 | 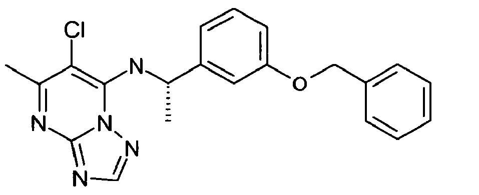 | S-enantiomer |
| C.I.43 | 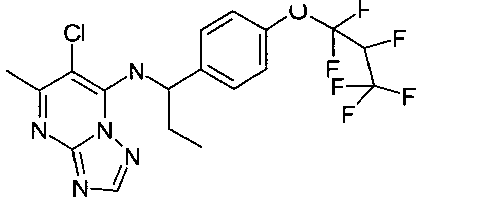 | Mixture of isomers |
| C.I.44 | 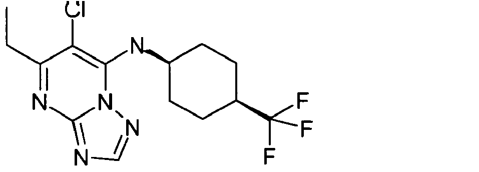 | Cis-isomer |
| C.I.45 | 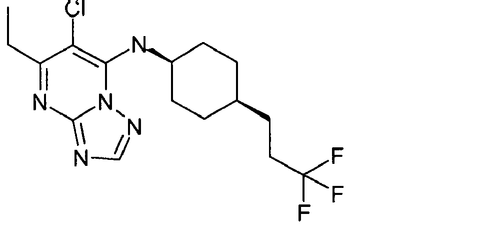 | Cis-isomer |
| C.I.46 | 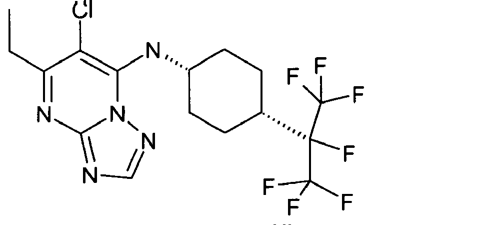 | 80% cis-isomer |
| C.I.47 | 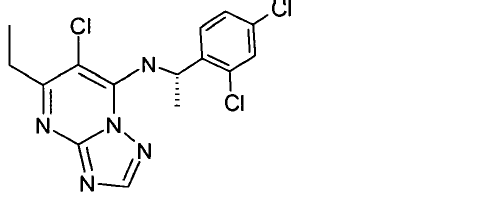 | S-enantiomer |
| C.I.48 | 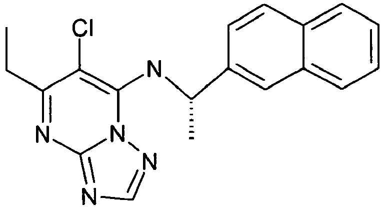 | S-enantiomer |
| C.I.49 | 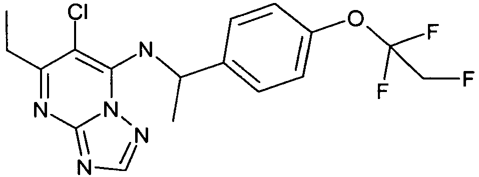 | Racemate |
| C.I.50 | 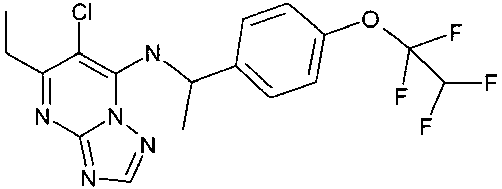 | Racemate |
| C.I.51 | 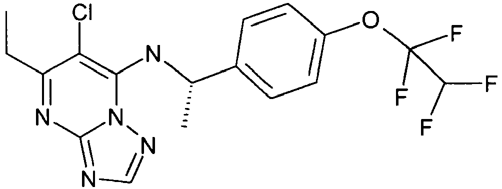 | S-enantiomer |
| C.I.52 | 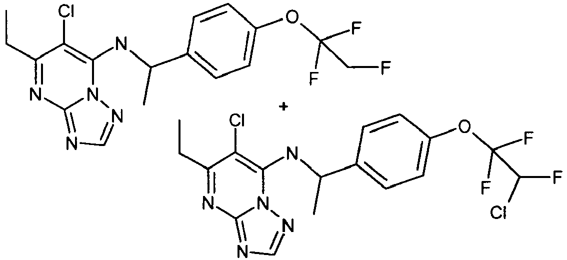 | Mixture |
| C.I.53 | 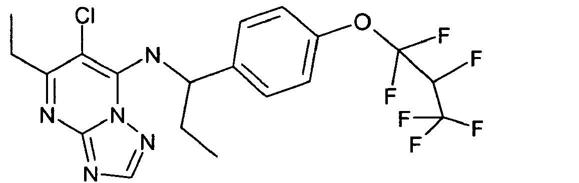 | Mixture of isomers |
| C.I.54 | Left blank intentionally | |
| C.I.55 | 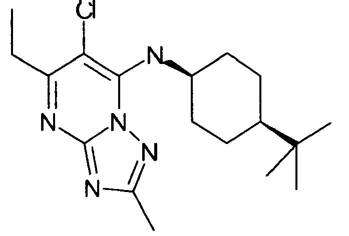 | Cis-isomer |
| C.I.56 | 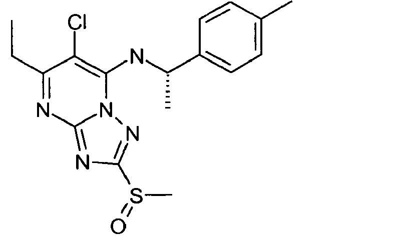 | S-enantiomer |
| C.I.57 | 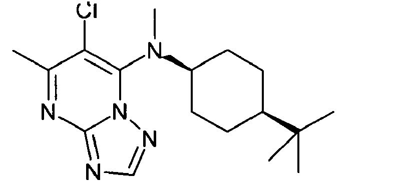 | Cis-isomer |
| C.1.58 | 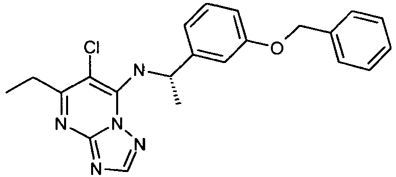 | S-enantiomer |
| C.I.59 | 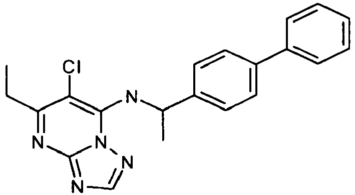 | Racemate |
| C.I.60 | 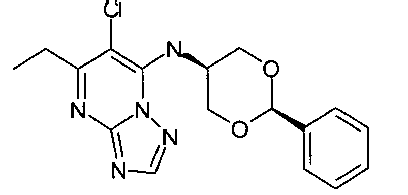 | Cis-isomer |

### Biological examples

### B.1 Activity against Mediterranean fruitfly (Ceratitis capitata)

The compounds were formulated in 1:3 DMSO:water. 50 to 80 eggs were placed into microtiterplates filled with 0.5% agar-agar and 14% diet in water. The eggs were sprayed with 5 µl of the test solution, the plates were sealed with pierced foils and kept at 27-29°C and 75-85% humidity under fluorescent light for 6 days. Mortality was assessed on the basis of the agility of the hatched larvae. Tests were replicated 2 times. In this test, compounds C.I from table C with Nos 22, 38, 43, 49, 53 and 55 at 2500 ppm showed over 75% mortality compared to 0% mortality of untreated controls.

### B.2 Activity against Caenorhabditis elegans

The compounds were formulated in 100% DMSO and were tested in microtiter plates containing 50µl nematode growth media, 1% E. coli and 20 L1 C. elegans. The efficacy of a compound was determined based on the motility of the larvae as compared to average motility of control wells containing DMSO only. Compounds with > 80% reduction in motility were tested in a dose response assay to determine EC50 values.
In this test, compounds C.I. from table C with Nos 1, 2, 10, 11, 12, 23, 28-30, 32, 34, 40, 43, 45, 46, 49-53, 56, 58 and 59 showed an EC50 values of <10 ppm.

### B.3 Activity against Barberpole worm (Haemonchus contortus)

The compounds were formulated in 100% DMSO and were tested in microtiter plates containing 50µl nematode media, 7% fecal slurry and 20 L1 H. contortus. The efficacy of a compound was determined based on the motility of the larvae as compared to average motility of control wells containing DMSO only. An MIC90 value was calculated by determining the lowest dose at which there was a 90% reduction in motility as compared to the control wells.
In this test, compounds C.I. from table C with Nos 10, 11, 12, 23, 28, 29, 32, 43, 45, 52, 56 and 59 showed a MIC 90 value of < 1 ppm.

### B.4. Activity against Yellowfever mosquito (Aedes aegypti)

The compounds were formulated in 100% DMSO and were tested in microtiter plates containing 180ul 1X Luria Broth media and 10 neonate A. aegypti larvae. The efficacy of a compound was determined based on the motility of the larvae as compared to average motility of control wells containing DMSO only. Compounds with > 80% reduction in motility were tested in a dose response assay to determine EC50 values.
In this test, compounds C.I. from table C with Nos 1-11, 13-22, 25-29, 31, 33-53 and 56-60 showed an EC50 values of <1 ppm.

### B.5 Activity against cat flea (Ctenocephalides felis)

### B.5.1 Ingestion Assay with Ctenocephalides felis

The compounds were formulated in 100% DMSO and were diluted with fresh cow blood to final testing concentrations of 50 ppm, 12.5 ppm, 3.125 ppm, 0.78 ppm and 0.195 ppm. Ten adult C. felis were loaded into testing cages and exposed to blood containing the test compound for up to 72 hours. Blood was changed at 24 hour intervals until the test was completed and kept at 37° C for duration of the test. The efficacy of a compound was determined based on mortality of C. felis at 72 hours post treatment. In this test, compounds C.I. from table C with no.5,7-9,10,11,17,18,20,31,34,37,38,42-52 and 58-60 showed an EC50 values of < 50 ppm.

The efficacy of a compound can also be determined by the following contact assays.

### B.5.2 Contact Assay with Ctenocephalides felis

A compound is formulated in 100% acetone to defined final test compound concentrations. A pipe cleaner is placed in the bottom of a glass scintillation vial and treated with compound and allowed to dry for 3-4 hours. Each vial is infested with 10 adult C. felis. The efficacy of a compound is determined based on mortality of C. felis up to 72 hours post treatment. A symptomatic effective concentration (SEC) is also calculated for test compounds. This value is generated based on the ability of a compound to affect flea movement; i.e. twitching, falling on side, or inability to stand up.

### B.6 Contact Assay with Rhipicephalus sanguineus

A compound is formulated in 100% DMSO and is diluted in 100% acetone to defined final test compound concentrations. Glass vials are treated with formulated compounds and allowed to dry. Filter papers are placed in the bottom and lid of the glass vials are treated with compound and allowed to try for 3-4 hours. Each vial is infested with 10 adult R. sanguineus. The efficacy of a compound is determined based on mortality of R. sanguineus at 24 hours post treatment and 48 hours post treatment.

### B.7 Study to evaluate efficacy of compounds against fleas and ticks in dogs

Dogs were infested with 100 cat fleas on days -1, 8, 15, 22, 29, and 36, and 50 brown dog ticks on days -1, 7 14, 21, 28, and 35. Infested dogs receive a treatment of a compound or placebo in the form of spot-on applied at the middle of the neck between the shoulder blades. Live fleas and ticks were removed and counted on days 2, 9, 16, 23, 30, and 37. Compound C.I. 20 from Table C showed >80% efficacy against fleas and ticks on days 9, 16 and 23 at 20 mg/kg compared to the placebo group.

## Claims

1. 6-halogeno-[1,2,4]-triazolo[1,5-a]-pyrimidine compound of the general formula I 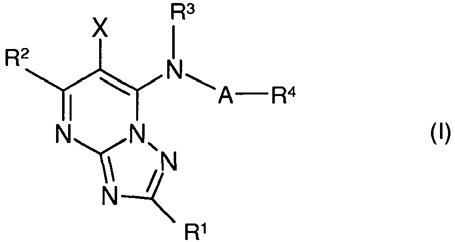 wherein X is halogen;
R¹ is hydrogen, halogen, OH, CN, C₁-C₁₀-alkyl, C₁-C₁₀-haloalkyl, C₁-C₁₀-alkoxy, C₁-C₆-alkoxy-C₁-C₆-alkyl, C₁-C₄-alkoxy-C₁-C₄-alkoxy, C₁-C₄-alkoxy-C₁-C₄-alkoxy-C₁-C₄-alkoxy, C₁-C₁₀-alkylthio, C₁-C₁₀-alkylsulfinyl, C₁-C₁₀-alkylsulfonyl, C₁-C₁₀-alkylamino, di(C₁-C₁₀-alkyl)amino, C₂-C₁₀-alkenyl, phenyl, phenoxy, benzyloxy, C₂-C₁₀-alkenyloxy, C₂-C₁₀-alkynyloxy or C₂-C₁₀-alkynyl, wherein C₁-C₁₀-alkylthio, C₁-C₁₀-alkylsulfinyl and C₁-C₁₀-alkylsulfonyl may be unsubstituted or partially or completely substituted with halogen, C₁-C₄-alkoxy, C₁-C₄-alkoxycarbonyl or COOH;
R² is C₁-C₁₀-alkyl, C₁-C₁₀-haloalkyl, C₁-C₆-alkoxy-C₁-C₆-alkyl, C₃-C₁₀-cycloalkyl, C₂-C₁₀-alkenyl or C₂-C₁₀-alkynyl;
R³ is hydrogen, C₁-C₁₀-alkyl, C₁-C₁₀-haloalkyl, C₁-C₆-alkoxy-C₁-C₆-alkyl, C₁-C₁₀-alkylcarbonyl, C₁-C₁₀-alkoxycarbonyl or arylcarbonyl;
A is a single bond or a C₁-C₆-alkylene chain, wherein one methylene group may be substituted by one heteroatom selected from oxygen or sulfur;
R⁴ is C₃-C₁₀-cycloalkyl, phenyl, naphthyl, or 3- to 7-membered heterocyclyl, being unsubstituted or substituted with 1, 2 or 3 radicals which are selected from the group consisting of halogen, cyano, nitro, hydroxy, C₁-C₁₀-alkyl, C₁-C₁₀-alkoxy, C₁-C₁₀-haloalkyl, C₁-C₁₀-haloalkoxy, amino, C₁-C₁₀-alkylamino, di(C₁-C₁₀-alkyl)amino, C₁-C₁₀-alkylthio, C₁-C₁₀-alkylsulfinyl, C₁-C₁₀-alkylsulfonyl, C₃-C₈-cycloalkyl, C₃-C₈-cycloalkyl-C₁-C₄-alkyl, phenyl, phenyl-C₁-C₄-alkoxy and phenyloxy, wherein the five last-mentioned radicals for their part may be unsubstituted or may carry one, two or three substituents which are selected from the group consisting of C₁-C₄-alkyl, C₁-C₄-alkoxy and halogen, and wherein the 3 to 7 membered heterocyclyl contains 1, 2 or 3 heteroatoms selected from the group consisting of oxygen, sulfur, nitrogen and a group NR^{a}, it being also possible for C₃-C₁₀-cycloalkyl, phenyl and 3- to 7-membered heterocyclyl to be fused to a 5- to 7-membered saturated, unsaturated or aromatic carbocyclic ring or to a 5- to 7-membered heterocyclic ring and said fused ring may be unsubstituted or may itself carry one, two, three, four, five or six substituents which are selected from the group consisting of halogen and C₁-C₄-alkyl; wherein R^{a} is hydrogen, C₁-C₆-alkyl, C₃-C₆-alkenyl or C₃-C₆-alkynyl; and/or an enantiomer, diastereomer or veterinarily acceptable salt thereof for use in treating, controlling, preventing or protecting against infestation or infection by parasites wherein the compound is for oral, topical or parenteral administration to an animal.

2. A compound for use according to claim 1, wherein in compounds of formula I, A is a single bond.

3. A compound for use according to claim 1, wherein in compounds of formula I, A is a C₁-C₃-alkylene chain.

4. A compound for use according to claim 1, wherein in compounds of formula I, A is CH₂, C₂H₄, CH(CH₃), or CH(C₂H₅).

5. A compound for use according to any of the preceding claims, wherein in compounds of formula I the radical
R⁴ is selected from phenyl, phenyl-C₁-C₄-alkyl or C₃-C₈-cycloalkyl, wherein each phenyl and C₃-C₈-cycloalkyl group may be unsubstituted or may carry one or two substituents as defined in claim 1.

6. A compound for use according to claim 2, wherein in compounds of formula I the radical
R⁴ is cis-cyclohexyl, which may be unsubstituted or may carry one or two substituents as defined in claim 1.

7. A compound for use according to claim 2 or 6, wherein in compounds of formula I the radical
R⁴ is cyclohexyl, which carries a substituent in the 4-position.

8. A compound for use according to claim 2 or 6, wherein in compounds of formula I the radical
R⁴ is cyclohexyl, which carries C₁-C₄-alkyl or C₁-C₄-haloalkyl in the 4-position.

9. A compound for use according to claims 3 or 4, wherein in compounds of formula I the radical
R⁴ is 1-phenylethyl, which is an S-enantiomer.

10. A compound for use according to claims 3, 4 or 9, wherein in compounds of formula I the radical
R⁴ is 1-phenylethyl, which carries a substituent in the 4-position.

11. A compound for use according to claims 3, 4 or 9, wherein in compounds of formula I the radical
R⁴ is 1-phenylethyl, which carries a substituent in the 4-position, and wherein the substituent is selected from the group consisting of halogen, C₁-C₄-alkyl, C₁-C₄-alkoxy, C₁-C₄-haloalkyl and C₁-C₄-haloalkoxy.

12. A compound for use according to any of the preceding claims, wherein in compounds of formula I the radical
R¹ is hydrogen, C₁-C₄-alkyl, C₁-C₄-haloalkyl, C₁-C₄-alkoxy, C₁-C₄-alkylthio, C₁-C₄-alkylsulfinyl or C₁-C₄-alkylsulfonyl.

13. A compound for use according to any of the preceding claims, wherein in compounds of formula I the radical R¹ is hydrogen.

14. A compound for use according to any of the preceding claims, wherein in compounds of formula I the radical
R² is C₁-C₄-alkyl, C₁-C₄-alkoxy or C₁-C₄-haloalkyl.

15. A compound for use according to any of the preceding claims, wherein in compounds of formula I the radical
R² is C₁-C₂-alkyl, C₁-C₂-alkoxy or C₁-C₂-fluoroalkyl.

16. A compound for use according to any of the preceding claims, wherein in compounds of formula I the radical
R³ is hydrogen or C₁-C₄-alkoxy-C₁-C₄-alkyl.

17. A compound for use according to any of the preceding claims, wherein in compounds of formula I the radical R³ is hydrogen.

18. A compound for use according to any of the preceding claims, wherein in compounds of formula I the radical X is chlorine.

19. A compound for use according to any preceding claim wherein the parasites are selected from the Diptera, Siphonaptera, and Ixodida orders.

20. A compound for use according to any one of claims 1 to 18 wherein the parasites are mosquitos.

21. A compound according for use according to any one of claims 1 to 18 wherein the parasites are flies.

22. A compound for use according to any one of claims 1 to 18 wherein the parasites are fleas.

23. A compound for use according to any one of claims 1 to 18 wherein the parasites are ticks.

24. A compound for use according to any one of claims 1 to 18 wherein the parasites are endoparasites.

25. A compound for use according to claim 24 wherein the endoparasites are round-worms, thorny headed worms and planarians.

26. A compound for use according to any of claims 1 to 18, wherein the animals are domestic animals.

27. A compound for use according to claim 26, wherein the animals are cats or dogs.

28. Use of a compound of formula I as defined in any one of claims 1 to 18 for the preparation of a veterinary medicament for treating, controlling, preventing or protecting animals against infestation or infection by parasites.

## Patentansprüche

1. 6-Halogen-[1,2,4]-triazol[1,5-a]-pyrimidinverbindung der allgemeinen Formel I 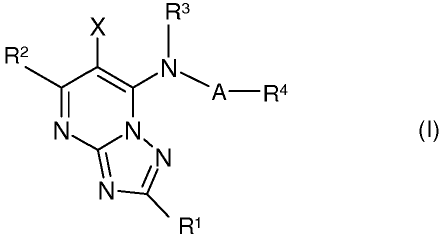 wobei
X Halogen ist;
R¹ Wasserstoff, Halogen, OH, CN, C₁-C₁₀-Alkyl, C₁-C₁₀-Haloalkyl, C₁-C₁₀-Alkoxy, C₁-C₆-Alkoxy-C₁-C₆-alkyl, C₁-C₄-Alkoxy-C₁-C₄-alkoxy, C₁-C₄-Alkoxy-C₁-C₄-alkoxy-C₁-C₄-alkoxy, C₁-C₁₀-Alkylthio, C₁-C₁₀-Alkylsulfinyl, C₁-C₁₀-Alkylsulfonyl, C₁-C₁₀-Alkylamino, Di(C₁-C₁₀-alkyl)amino, C₂-C₁₀-Alkenyl, Phenyl, Phenoxy, Benzyloxy, C₂-C₁₀-Alkenyloxy, C₂-C₁₀-Alkynyloxy oder C₂-C₁₀-Alkynyl ist, wobei C₁-C₁₀-Alkylthio, C₁-C₁₀-Alkylsulfinyl und C₁-C₁₀-Alkylsulfonyl unsubstituiert oder teilweise oder vollständig mit Halogen, C₁-C₄-Alkoxy, C₁-C₄-Alkoxycarbonyl oder COOH substituiert sein können;
R² C₁-C₁₀-Alkyl, C₁-C₁₀-Haloalkyl, C₁-C₆-Alkoxy-C₁-C₆-alkyl, C₃-C₁₀-Cycloalkyl, C₂-C₁₀-Alkenyl oder C₂-C₁₀-Alkinyl ist;
R³ Wasserstoff, C₁-C₁₀-Alkyl, C₁-C₁₀-Haloalkyl, C₁-C₆-Alkoxy-C₁-C₆-alkyl, C₁-C₁₀-Alkylcarbonyl, C₁-C₁₀-Alkoxycarbonyl oder Arylcarbonyl ist;
A eine Einfachbindung oder eine C₁-C₆-Alkylenkette ist, wobei eine Methylengruppe durch ein Heteroatom, ausgewählt aus Sauerstoff oder Schwefel, ersetzt sein kann;
R⁴ C₃-C₁₀-Cycloalkyl, Phenyl, Naphthyl, oder 3- bis 7-gliedriges Heterocyclyl ist, welche unsubstituiert oder mit 1, 2 oder 3 Resten, ausgewählt aus der Gruppe bestehend aus Halogen, Cyano, Nitro, Hydroxy, C₁-C₁₀-Alkyl, C₁-C₁₀-Alkoxy, C₁-C₁₀-Haloalkyl, C₁-C₁₀-Haloalkoxy, Amino, C₁-C₁₀-Alkylamino, Di(C₁-C₁₀-alkyl)amino, C₁-C₁₀-Alkylthio, C₁-C₁₀-Alkylsulfinyl, C₁-C₁₀-Alkylsulfonyl, C₃-C₈-Cycloalkyl, C₃-C₈-Cycloalkyl-C₁-C₄-alkyl, Phenyl, Phenyl-C₁-C₄-alkoxy und Phenyloxy substituiert sind, wobei die fünf letztgenannten Reste wiederum unsubstituiert sein können oder einen, zwei oder drei Substituenten, ausgewählt aus der Gruppe bestehend aus C₁-C₄-Alkyl, C₁-C₄-Alkoxy und Halogen, tragen können, und wobei das 3- bis 7-gliedrige Heterocyclyl 1, 2 oder 3 Heteroatome, ausgewählt aus der Gruppe bestehend aus Sauerstoff, Schwefel, Stickstoff und eine NR^{a}-Gruppe, enthält, wobei es auch möglich ist, dass C₃-C₁₀-Cycloalkyl, Phenyl und das 3- bis 7-gliedrige Heterocyclyl mit einem 5- bis 7-gliedrigen, gesättigten, ungesättigten oder aromatischen carbozyklischen Ring oder mit einem 5-bis 7-gliedrigen heterozyklischen Ring kondensiert sind und dieser kondensierte Ring unsubstituiert sein kann oder selbst einen, zwei, drei, vier, fünf oder sechs Substituenten tragen kann, ausgewählt aus der Gruppe bestehend aus Halogen und C₁-C₄-Alkyl; wobei R^{a} Wasserstoff, C₁-C₆-Alkyl, C₃-C₆-Alkenyl oder C₃-C₆-Alkinyl ist,
und/oder ein Enantiomer, Diastereomer oder veterinärmedizinisch annehmbares Salz davon
zur Verwendung in der Behandlung, Bekämpfung, Vorbeugung oder dem Schutz gegen Parasitenbefall oder -infektion, wobei die Verbindung zur oralen, topischen oder parenteralen Verabreichung an ein Tier bereitgestellt wird.

2. Eine Verbindung zur Verwendung gemäß Anspruch 1, wobei A in Verbindungen der Formel I eine Einfachbindung ist.

3. Eine Verbindung zur Verwendung gemäß Anspruch 1, wobei A in Verbindungen der Formel I eine C₁-C₃-Alkylenkette ist.

4. Eine Verbindung zur Verwendung gemäß Anspruch 1, wobei A in Verbindungen der Formel I CH₂, C₂H₄, CH(CH₃) oder CH(C₂H₅) ist.

5. Eine Verbindung zur Verwendung gemäß einem der vorangehenden Ansprüche, wobei in Verbindungen der Formel I der Rest
R⁴ ausgewählt ist aus Phenyl, Phenyl-C₁-C₄-alkyl oder C₃-C₈-Cycloalkyl, wobei jede Phenyl- und C₃-C₈-Cycloalkylgruppe unsubstituiert sein kann oder einen oder zwei wie in Anspruch 1 definierte Substituenten tragen kann.

6. Eine Verbindung zur Verwendung gemäß Anspruch 2, wobei in Verbindungen der Formel I der Rest
R⁴ cis-Cyclohexyl ist, das unsubstituiert sein kann oder einen oder zwei wie in Anspruch 1 definierte Substituenten tragen kann.

7. Eine Verbindung zur Verwendung gemäß Anspruch 2 oder 6, wobei in Verbindungen der Formel I der Rest
R⁴ Cyclohexyl ist, das einen Substituenten an der 4-Position trägt.

8. Eine Verbindung zur Verwendung gemäß Anspruch 2 oder 6, wobei in Verbindungen der Formel I der Rest
R⁴ Cyclohexyl ist, das C₁-C₄-Alkyl oder C₁-C₄-Haloalkyl an der 4-Position trägt.

9. Eine Verbindung zur Verwendung gemäß den Ansprüchen 3 oder 4, wobei in Verbindungen der Formel I der Rest
R⁴ 1-Phenylethyl ist, wobei es sich um ein S-Enantiomer handelt.

10. Eine Verbindung zur Verwendung gemäß den Ansprüchen 3, 4 oder 9, wobei in Verbindungen der Formel I der Rest R⁴ 1-Phenylethyl ist, das einen Substituenten an der 4-Position trägt.

11. Eine Verbindung zur Verwendung gemäß den Ansprüchen 3, 4 oder 9, wobei in Verbindungen der Formel I der Rest
R⁴ 1-Phenylethyl ist, das einen Substituenten an der 4-Position trägt, und wobei der Substituent ausgewählt ist aus der Gruppe bestehend aus Halogen, C₁-C₄-Alkyl, C₁-C₄-Alkoxy, C₁-C₄-Haloalkyl und C₁-C₄-Haloalkoxy.

12. Eine Verbindung zur Verwendung gemäß einem der vorangehenden Ansprüche, wobei in Verbindungen der Formel I der Rest R¹ Wasserstoff, C₁-C₄-Alkyl, C₁-C₄-Haloalkyl, C₁-C₄-Alkoxy, C₁-C₄-Alkylthio, C₁-C₄-Alkylsulfinyl oder C₁-C₄-Alkylsulfonyl ist.

13. Eine Verbindung zur Verwendung gemäß einem der vorangehenden Ansprüche, wobei in Verbindungen der Formel I der Rest R¹ Wasserstoff ist.

14. Eine Verbindung zur Verwendung gemäß einem der vorangehenden Ansprüche, wobei in Verbindungen der Formel I der Rest R² C₁-C₄-Alkyl, C₁-C₄-Alkoxy oder C₁-C₄-Haloalkyl ist.

15. Eine Verbindung zur Verwendung gemäß einem der vorangehenden Ansprüche, wobei in Verbindungen der Formel I der Rest R² C₁-C₂-Alkyl, C₁-C₂-Alkoxy oder C₁-C₂-Fluoralkyl ist.

16. Eine Verbindung zur Verwendung gemäß einem der vorangehenden Ansprüche, wobei in Verbindungen der Formel I der Rest R³ Wasserstoff oder C₁-C₄-Alkoxy-C₁-C₄-alkyl ist.

17. Eine Verbindung zur Verwendung gemäß einem der vorangehenden Ansprüche, wobei in Verbindungen der Formel I der Rest R³ Wasserstoff ist.

18. Eine Verbindung zur Verwendung gemäß einem der vorangehenden Ansprüche, wobei in Verbindungen der Formel I der Rest X Chlor ist.

19. Eine Verbindung zur Verwendung gemäß einem vorangehenden Anspruch, wobei die Parasiten aus der Ordnung Diptera, Siphonaptera und Ixodida ausgewählt sind.

20. Eine Verbindung zur Verwendung gemäß einem der Ansprüche 1 bis 18, wobei die Parasiten Stechmücken sind.

21. Eine Verbindung zur Verwendung gemäß einem der Ansprüche 1 bis 18, wobei die Parasiten Fliegen sind.

22. Eine Verbindung zur Verwendung gemäß einem der Ansprüche 1 bis 18, wobei die Parasiten Flöhe sind.

23. Eine Verbindung zur Verwendung gemäß einem der Ansprüche 1 bis 18, wobei die Parasiten Zecken sind.

24. Eine Verbindung zur Verwendung gemäß einem der Ansprüche 1 bis 18, wobei die Parasiten Endoparasiten sind.

25. Eine Verbindung zur Verwendung gemäß Anspruch 24, wobei die Endoparasiten Spulwürmer, Kratzwürmer und Strudelwürmer sind.

26. Eine Verbindung zur Verwendung gemäß einem der Ansprüche 1 bis 18, wobei die Tiere Haustiere sind.

27. Eine Verbindung zur Verwendung gemäß Anspruch 26, wobei die Tiere Katzen oder Hunde sind.

28. Eine Verwendung einer Verbindung der Formel I wie in einem der Ansprüche 1 bis 18 definiert zur Herstellung eines Tierarzneimittels zur Behandlung, Bekämpfung, Vorbeugung oder dem Schutz von Tieren gegen Parasitenbefall oder
-infektion.

## Revendications

1. Composé 6-halogéno-[1,2,4]-triazolo[1,5-a]-pyrimidine de formule générale I 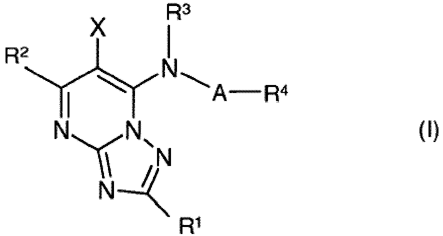 dans lequel
X est halogène ;
R¹ est hydrogène, halogène, OH, CN, alkyle en C₁ à C₁₀, halogénoalkyle en C₁ à C₁₀, alcoxy en C₁ à C₁₀, alcoxy en C₁ à C₆-alkyle en C₁ à C₆, alcoxy en C₁ à C₄-alcoxy en C₁ à C₄, alcoxy en C₁ à C₄-alcoxy en C₁ à C₄-alcoxy en C₁ à C₄, alkylthio en C₁ à C₁₀, alkylsulfinyle en C₁ à C₁₀, alkylsulfonyle en C₁ à C₁₀, alkylamino en C₁ à C₁₀, di(alkyle en C₁ à C₁₀)amino, alcényle en C₂ à C₁₀, phényle, phénoxy, benzyloxy, alcényloxy en C₂ à C₁₀, alcynyloxy en C₂ à C₁₀ ou alcynyle en C₂ à C₁₀, dans lequel alkylthio en C₁ à C₁₀, alkylsulfinyle en C₁ à C₁₀ et alkylsulfonyle en C₁ à C₁₀ peuvent être non substitués ou partiellement ou complètement substitués par halogène, alcoxy en C₁ à C₄, alcoxycarbonyle en C₁ à C₄ ou COOH ;
R² est alkyle en C₁ à C₁₀, halogénoalkyle en C₁ à C₁₀, alcoxy en C₁ à C₆-alkyle en C₁ à C₆, cycloalkyle en C₃ à C₁₀, alcényle en C₂ à C₁₀ ou alcynyle en C₂ à C₁₀ ; R³ est hydrogène, alkyle en C₁ à C₁₀, halogénoalkyle en C₁ à C₁₀, alcoxy en C₁ à C₆-alkyle en C₁ à C₆, alkylcarbonyle en C₁ à C₁₀, alcoxycarbonyle en C₁ à C₁₀ ou arylcarbonyle ;
A est une simple liaison ou une chaîne alkylène en C₁ à C₆, dans lequel un groupe méthylène peut être substitué par un hétéroatome choisi parmi l'oxygène ou le soufre ;
R⁴ est cycloalkyle en C₃ à C₁₀, phényle, naphtyle, ou hétérocyclyle à 3 ou 7 chaînons, étant non substitué ou substitué par 1, 2 ou 3 radicaux qui sont choisis dans le groupe consistant en halogène, cyano, nitro, hydroxy, alkyle en C₁ à C₁₀, alcoxy en C₁ à C₁₀, halogénoalkyle en C₁ à C₁₀, halogénoalcoxy en C₁ à C₁₀, amino, alkylamino en C₁ à C₁₀, di(alkyle en C₁ à C₁₀)amino, alkylthio en C₁ à C₁₀, alkylsulfinyle en C₁ à C₁₀, alkylsulfonyle en C₁ à C₁₀, cycloalkyle en C₃ à C₈, cycloalkyle en C₃ à C₈-alkyle en C₁ à C₄, phényle, phényl-alcoxy en C₁ à C₄ et phényloxy,
dans lequel les cinq derniers radicaux mentionnés pour leur partie peuvent être non substitués ou peuvent porter un, deux ou trois substituants qui sont choisis dans le groupe consistant en alkyle en C₁ à C₄, alcoxy en C₁ à C₄ et halogène,
et dans lequel l'hétérocyclyle à 3 à 7 chaînons contient 1, 2 ou 3 hétéroatomes choisis dans le groupe consistant en l'oxygène, le soufre, l'azote et un groupe NR^{a},
il est également possible pour cycloalkyle en C₃ à C₁₀, phényle et hétérocyclyle à 3 à 7 chaînons d'être fusionnés à un cycle carbocyclique aromatique saturé ou insaturé à 5 à 7 chaînons ou à un cycle hétérocyclique à 5 à 7 chaînons et ledit cycle fusionné peut être non substitué ou peut lui-même porter un, deux, trois, quatre, cinq ou six substituants qui sont choisis dans le groupe consistant en halogène et alkyle en C₁ à C₄ ; dans lequel
R^{a} est hydrogène, alkyle en C₁ à C₆, alcényle en C₃ à C₆ ou alcynyle en C₃ à C₆ ; et/ou un énantiomère, un diastéréoisomère ou un sel acceptable sur le plan vétérinaire de celui-ci pour une utilisation dans le traitement, le contrôle, la prévention ou la protection contre une infestation ou une infection par des parasites dans lequel le composé est destiné à une administration orale, topique ou parentérale à un animal.

2. Composé pour une utilisation selon la revendication 1, dans lequel dans les composés de formule I, A est une simple liaison.

3. Composé pour une utilisation selon la revendication 1, dans lequel dans les composés de formule I, A est une chaîne alkylène en C₁ à C₃.

4. Composé pour une utilisation selon la revendication 1, dans lequel dans les composés de formule I, A est CH₂, C₂H₄, CH(CH₃) ou CH(C₂H₅).

5. Composé pour une utilisation selon l'une quelconque des revendications précédentes, dans lequel dans les composés de formule I, le radical R⁴ est choisi parmi phényle, phényl-alkyle en C₁ à C₄ ou cycloalkyle en C₃ à C₈, dans lequel chaque groupe phényle et cycloalkyle en C₃ à C₈ peut être non substitué ou peut porter un ou deux substituants tels que définis en revendication 1.

6. Composé pour une utilisation selon la revendication 2, dans lequel dans les composés de formule I, le radical
R⁴ est cis-cyclohexyle, qui peut être non substitué ou peut porter un ou deux substituants tels que définis en revendication 1.

7. Composé pour une utilisation selon la revendication 2 ou 6, dans lequel dans les composés de formule I, le radical
R⁴ est cyclohexyle, qui porte un substituant en position 4.

8. Composé pour une utilisation selon la revendication 2 ou 6, dans lequel dans les composés de formule I, le radical
R⁴ est cyclohexyle, qui porte alkyle en C₁ à C₄ ou halogénoalkyle en C₁ à C₄ en position 4.

9. Composé pour une utilisation selon l'une quelconque des revendications 3 ou 4, dans lequel dans les composés de formule I, le radical
R⁴ est 1-phényléthyle, qui est un énantiomère S.

10. Composé pour une utilisation selon les revendications 3, 4 ou 9, dans lequel dans les composés de formule I, le radical
R⁴ est 1-phényléthyle, qui porte un substituant en position 4.

11. Composé pour une utilisation selon les revendications 3, 4 ou 9, dans lequel dans les composés de formule I, le radical
R⁴ est 1-phényléthyle, qui porte un substituant en position 4, et dans lequel le substituant est choisi dans le groupe consistant en halogène, alkyle en C₁ à C₄, alcoxy en C₁ à C₄, halogénoalkyle en C₁ à C₄ et halogénoalcoxy en C₁ à C₄.

12. Composé pour une utilisation selon l'une quelconque des revendications précédentes, dans lequel dans les composés de formule I, le radical
R¹ est hydrogène, alkyle en C₁ à C₄, halogénoalkyle en C₁ à C₄, alcoxy en C₁ à C₄, alkylthio en C₁ à C₄, alkylsulfinyle en C₁ à C₄ ou alkylsulfonyle en C₁ à C₄.

13. Composé pour une utilisation selon l'une quelconque des revendications précédentes, dans lequel dans les composés de formule I, le radical R¹ est hydrogène.

14. Composé pour une utilisation selon l'une quelconque des revendications précédentes, dans lequel dans les composés de formule I, le radical
R² est alkyle en C₁ à C₄, alcoxy en C₁ à C₄ ou halogénoalkyle en C₁ à C₄.

15. Composé pour une utilisation selon l'une quelconque des revendications précédentes, dans lequel dans les composés de formule I, le radical
R² est alkyle en C₁ à C₂, alcoxy en C₁ à C₂ ou fluoroalkyle en C₁ à C₂.

16. Composé pour une utilisation selon l'une quelconque des revendications précédentes, dans lequel dans les composés de formule I, le radical
R³ est hydrogène ou alcoxy en C₁ à C₄-alkyle en C₁ à C₄.

17. Composé pour une utilisation selon l'une quelconque des revendications précédentes, dans lequel dans les composés de formule I, le radical R³ est hydrogène.

18. Composé pour une utilisation selon l'une quelconque des revendications précédentes, dans lequel dans les composés de formule I, le radical X est le chlore.

19. Composé pour une utilisation selon l'une quelconque des revendications précédentes, dans lequel les parasites sont choisis parmi les ordres des diptères, siphonaptère et *Ixodida.*

20. Composé pour une utilisation selon l'une quelconque des revendications 1 à 18, dans lequel les parasites sont des moustiques.

21. Composé pour une utilisation selon l'une quelconque des revendications 1 à 18, dans lequel les parasites sont des mouches.

22. Composé pour une utilisation selon l'une quelconque des revendications 1 à 18, dans lequel les parasites sont des puces.

23. Composé pour une utilisation selon l'une quelconque des revendications 1 à 18, dans lequel les parasites sont des tiques.

24. Composé pour une utilisation selon l'une quelconque des revendications 1 à 18, dans lequel les parasites sont des endoparasites.

25. Composé pour une utilisation selon la revendication 24, dans lequel les endoparasites sont des vers ronds, des acanthocéphales et des planaires.

26. Composé pour une utilisation selon l'une quelconque des revendications 1 à 18, dans lequel les animaux sont des animaux domestiques.

27. Composé pour une utilisation selon la revendication 26, dans lequel les animaux sont des chats ou des chiens.

28. Utilisation d'un composé de formule I tel que défini dans l'une quelconque des revendications 1 à 18, pour la préparation d'un médicament vétérinaire destiné à traiter, contrôler, prévenir ou protéger contre une infestation ou une infection des animaux par des parasites.
